(19) Europäisches Patentamt / European Patent Office / Office européen des brevets

(11) **EP 2 949 747 B1**

(12) **EUROPEAN PATENT SPECIFICATION**

(45) Date of publication and mention
of the grant of the patent:
**09.08.2017 Bulletin 2017/32**

(51) Int Cl.:
***C12N 5/071*** (2010.01)     ***A61L 27/00*** (2006.01)
***C12N 5/0775*** (2010.01)

(21) Application number: **14743709.9**

(22) Date of filing: **24.01.2014**

(86) International application number:
**PCT/JP2014/000372**

(87) International publication number:
**WO 2014/115562 (31.07.2014 Gazette 2014/31)**

(54) **CREATION OF THREE-DIMENSIONAL SYNTHETIC TISSUE FROM PLURIPOTENT STEM CELL-DERIVED CELLS, AND OSTEOCHONDRAL REGENERATION TREATMENT USING SAID SNTHETIC TISSUE**

ERZEUGUNG VON DREIDIMENSIONALEM KÜNSTLICHEM GEWEBE AUS VON PLURIPOTENTEN STAMMZELLEN ABGELEITETEN ZELLEN UND BEHANDLUNG FÜR OSTEOCHONDRALE REGENERATION MITHILFE DES KÜNSTLICHEN GEWEBES

CRÉATION DE TISSU ARTIFICIEL TRIDIMENSIONNEL À PARTIR DE CELLULES DÉRIVÉES DE CELLULES SOUCHES PLURIPOTENTES ET TRAITEMENT DE RÉGÉNÉRATION OSTÉOCHONDRALE UTILISANT LEDIT TISSU ARTIFICIEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.01.2013 JP 2013012630**

(43) Date of publication of application:
**02.12.2015 Bulletin 2015/49**

(73) Proprietor: **Osaka University**
**Suita-shi, Osaka 565-0871 (JP)**

(72) Inventors:
• **YOSHIKAWA, Hideki**
**Suita-shi**
**Osaka 565-0871 (JP)**
• **NAKAMURA, Norimasa**
**Suita-shi**
**Osaka 565-0871 (JP)**
• **SHIMOMURA, Kazunori**
**Suita-shi**
**Osaka 565-0871 (JP)**
• **MORIGUCHI, Yu**
**Suita-shi**
**Osaka 565-0871 (JP)**
• **CHIJIMATSU, Ryota**
**Suita-shi**
**Osaka 565-0871 (JP)**

• **YASUI, Yukihiko**
**Suita-shi**
**Osaka 565-0871 (JP)**

(74) Representative: **Uexküll & Stolberg**
**Partnerschaft von**
**Patent- und Rechtsanwälten mbB**
**Beselerstraße 4**
**22607 Hamburg (DE)**

(56) References cited:
**EP-A1- 2 799 093     EP-B1- 1 651 756**
**JP-A- 2007 528 756**

• YU MORIGUCHI ET AL.: 'Usagi Haisei Kansaibo yori Yudo sareta Kan'yokei Kansaibo Yurai Scaffold Free Sanjigen Jinko Soshiki no Keisei to sono Nankotsu Bunka Yudo' THE JOURNAL OF THE JAPANESE ORTHOPAEDIC ASSOCIATION vol. 85, no. 8, 2011, pages S1267, 2 - 6-29, XP008180151
• BUCKLEY C.T. ET AL.: 'Expansion in the presence of FGF-2 enhances the functional development of cartilaginous tissues engineered using infrapatellar fat pad derived MSCs' JOURNAL OF THE MECHANICAL BEHAVIOR OF BIOMEDICAL MATERIALS vol. 11, 01 June 2012, pages 102 - 111, XP028926491 DOI: 10.1016/j.jmbbm.2011.09.004

**(Cont. next page)**

- **AZOUNA NESRINE BEN ET AL.: 'Phenotypical and functional characteristics of mesenchymal stem cells from bone marrow: comparison of culture using different media supplemented with human platelet lysate or fetal bovine serum' STEM CELL RESEARCH & THERAPY vol. 3, 01 January 2012, pages 1 - 14, XP021117611 DOI: 10.1186/scrt97**
- **TAKESHI TERAMURA ET AL.: 'Teisanso Baiyo ni yoru Usagi ES Saibo kara no Kan'yokei Kansaibo no Bunka Yudo' REGENERATIVE MEDICI NE vol. 11, 2012, pages 233, B-1 - 51, XP008181709**
- **LEE SANG-HWA ET AL.: 'Culture of Mesenchymal Stromal Cells from Dental Pulp: Culture Medium Study for Effective Expansion and Characterization' TISSUE ENGINEERING AND REGENERATIVE MEDICINE vol. 7, no. 2, 2010, pages 248 - 254, XP008181633**
- **CRAMER CHRISTOPHER ET AL.: 'Persistent High Glucose Concentrations Alter the Regenerative Potential of Mesenchymal Stem Cells' STEM CELLS AND DEVELOPMENT vol. 19, no. 12, 01 January 2010, pages 1875 - 1884, XP055266731 DOI: 10.1089/scd.2010.0009**
- **HWANG NATHANIEL S. ET AL.: 'In vivo commitment and functional tissue regeneration using human embryonic stem cell -derived mesenchymal cells' PROCEEDINGS OF THE NATIONAL ACADEMY OF SCIENCES vol. 105, no. 52, 30 December 2008, pages 20641 - 20646, XP055266736 DOI: 10.1073/pnas.0809680106**

SEQUENCE LISTING

<110> Osaka University

<120> Creation of three-dimensional synthetic tissue from pluripotent stem-derived cells and osteochondral regeneration treatment using said synthetic tissue

<130> OU004PCT

<150> JP 2013-12630
<151> 2013-01-25

<160> 28

<170> PatentIn version 3.5

<210> 1
<211> 19
<212> DNA
<213> Artificial sequence

58

<220>
<223> pig-GAPDH forward primer

<400> 1
ctgccccttc tgctgatgc          19

<210> 2
<211> 20
<212> DNA
<213> Artificial sequence

<220>
<223> pig-GAPDH reverse primer

<400> 2
catcacgcca cagtttccca          20

<210> 3
<211> 22
<212> DNA
<213> Artificial sequence

<220>
<223> pig-aggrecan forward primer

<400> 3
attgtaggac ccaaaggacc tc          22

<210> 4
<211> 20
<212> DNA
<213> Artificial sequence

<220>
<223> pig-aggrecan reverse primer

<400> 4
ggtcccaggt tctccatctc          20

<210> 5
<211> 22
<212> DNA
<213> Artificial sequence

<220>
<223> pig-collagen 1a2 forward primer

<400> 5
attgtaggac ccaaaggacc tc          22

<210> 6
<211> 20
<212> DNA
<213> Artificial sequence

<220>
<223> pig-collagen 1a2 reverse primer

<400> 6
ggtcccaggt tctccatctc        20


<210> 7
<211> 22
<212> DNA
<213> Artificial sequence


<220>
<223> pig-collagen 2a1 forward primer

<400> 7
attgtaggac ccaaaggacc tc        22


<210> 8
<211> 20
<212> DNA
<213> Artificial sequence


<220>
<223> pig-collagen 2a1 reverse primer

<400> 8
ggtcccaggt tctccatctc        20

<210> 9
<211> 20
<212> DNA
<213> Artificial sequence


<220>
<223> rabbit Pou5f1 forward primer

<400> 9
gatcactctg ggctacactc        20

<210> 10
<211> 20
<212> DNA
<213> Artificial sequence

<220>
<223> rabbit Pou5f1 reverse primer

<400> 10
agatggtggt ttggctgaac        20


<210> 11
<211> 21
<212> DNA
<213> Artificial sequence


<220>
<223> rabbit Nanog forward primer

<400> 11
tagtgaaaac tcccgactct g        21

<210> 12
<211> 20
<212> DNA
<213> Artificial sequence

<220>
<223> rabbit Nanog reverse primer

<400> 12
agctgggtct gggagaatac          20

<210> 13
<211> 18
<212> DNA
<213> Artificial sequence

<220>
<223> rabbit Sox2 forward primer

<400> 13
gagtggaaac ccttgtgg          18

<210> 14
<211> 22
<212> DNA
<213> Artificial sequence

<220>
<223> rabbit Sox2 reverse primer

<400> 14
atttataacc ctgtgctcct cc          22

<210> 15
<211> 19
<212> DNA
<213> Artificial sequence

<220>
<223> rabbit Klf4 forward primer

<400> 15
aggagcccaa gccaaagag          19

<210> 16
<211> 17
<212> DNA
<213> Artificial sequence

<220>
<223> rabbit Klf4 reverse primer

<400> 16
aatcgcaagt gtgggtg          17

<210> 17
<211> 20
<212> DNA

<213> Artificial sequence

<220>
<223> rabbit PDGFRalhpa forward primer

<400> 17
agtgagctgg cagtacccgg        20

<210> 18
<211> 19
<212> DNA
<213> Artificial sequence

<220>
<223> rabbit PDGFRalhpa reverse primer

<400> 18
agccagtgtt gttctcctc        19

<210> 19
<211> 19
<212> DNA
<213> Artificial sequence

<220>
<223> rabbit Vimentin forward primer

<400> 19
ttgacaatgc ttctttggc        19

<210> 20
<211> 19
<212> DNA
<213> Artificial sequence

<220>
<223> rabbit Vimentin reverse primer

<400> 20
ttcctcatcg tgcagtttc        19

<210> 21
<211> 19
<212> DNA
<213> Artificial sequence

<220>
<223> rabbit Aggrecan forward primer

<400> 21
ttgaggacag cgaggctac        19

<210> 22
<211> 18
<212> DNA
<213> Artificial sequence

<220>

<223> rabbit Aggrecan reverse primer

<400> 22
gcccgatagt ggaacaca        18

<210> 23
<211> 19
<212> DNA
<213> Artificial sequence

<220>
<223> rabbit Col2A1 forward primer

<400> 23
tcgtggccga gatggagag        19

<210> 24
<211> 20
<212> DNA
<213> Artificial sequence

<220>
<223> rabbit Col2A1 reverse primer

<400> 24
tcaaatcctc cagccatttg        20

<210> 25
<211> 21
<212> DNA
<213> Artificial sequence

<220>
<223> rabbit 28s rRNA forward primer

<400> 25
agcagaattc accaagcgtt g        21

<210> 26
<211> 20
<212> DNA
<213> Artificial sequence

<220>
<223> rabbit 28s rRNA reverse primer

<400> 26
taacctgtct cacgacggtc        20

<210> 27
<211> 19
<212> DNA
<213> Artificial sequence

<220>
<223> rabbit Gapdh forward primer

<400> 27

gtgaaggtcg gagtgaacg 19

<210> 28
<211> 19
<212> DNA
<213> Artificial sequence

<220>
<223> rabbit Gapdh reverse primer

<400> 28
taaaagcagc cctggtgac 19

**Description**

[Technical Field]

The present invention relates to the field of regenerative medicine. More particularly, the present invention relates to a three-dimensional synthetic tissue with an improved therapeutic effect and a method for using said synthetic tissue. The synthetic tissue of the present invention has biological integration capability and achieves a significant effect in the treatment of an osteochondral defect.

[Background Art]

Recently, regenerative therapy has attracted attention as a novel method of therapy for an osteochondral defect or the like, which utilizes genetic engineering, cell tissue engineering, regenerative medicine and the like. A large number of researchers throughout the world are vigorously working on this important and challenging subject of research in advanced medical practice.

The scale of the market associated with regenerative medicine (tissue engineering) is estimated to be about 48 trillion yen globally and about 5 trillion yen in Japan according to materials prepared by the New Energy and Industrial Technology Development Organization. Tissue engineering products alone account for about 10 trillion yen globally. Thus, regenerative medicine has world-wide expectation as the next-generation industry in the field.

The present inventors have made efforts to develop regenerative therapy in the field of musculoskeletal and cardiovascular tissues, and have reported a combination therapy of cell implantation and growth factor administration as well as a tissue implantation regenerative therapy based on tissue engineering. However, it is of urgent and utmost importance to secure a source of autologous cells for such regenerative medicine based on cell or tissue implantation. Many cells in musculoskeletal tissues have a high level of self-repairing ability. It has been reported that there are cells with a function as a stem cell among the cells of the musculoskeletal tissues.

Osteoarthritis (OA) is a common disease that causes arthralgia or deformation and dysfunction of joints, affecting several hundred millions of people worldwide [Non Patent Literature 1]. Clinical options for OA treatment include total joint arthroplasty, osteotomy, osteochondral implant and the like, depending on the extent of joint damage. Recently, initiatives utilizing tissue engineering and regenerative medicine have been considered.

As has been reported in Non Patent Literature 2 and the like, a cell sheet engineering technique, led by the group of Okano et al, utilizing a temperature sensitive culture dish is a typical cell implanting method. Such a cell sheet engineering technique is internationally acclaimed as a cell implant method with originality. However, a single sheet obtained by this technique is often fragile. Thus , it was necessary to stack multiple sheets in order to obtain strength that can withstand surgical manipulation, such as implantation.

Even when a Petri dish having a fixed temperature responsive polymer (e.g., trade name: UpCell and RepCell) is used to culture and detach cells, an extracellular matrix or the like is not appropriately distributed in three-dimension. Thus, a practical implantable synthetic tissue has yet to be developed [Non Patent Literatures 2-4].

Furthermore, it is reported in Patent Literature 1 and Patent Literature 2 that cells are cultured on a semipermeable membrane using alginate gel. However, the resultant tissue is poorly integrated with an extracellular matrix and is not free of a scaffold. In addition, the cells in the tissue are not self-organized. The tissue has no self-supporting ability. The cells no longer have a differentiation potential. The tissue loses morphological plasticity in terms of three-dimensional structure. Therefore, the tissue is not suitable for cell implantation.

In this regard, some of the inventions have developed, and filed for a patent on, a technique that does not use a scaffold, which was deemed important due to issues of side effects in implant medicine (Patent Literature 3).

Further, it is reported that mesenchymal stem cells are efficiently induced by using a technique of inducing differentiation of embryonic stem cells (ES cells) under low oxygen [Non Patent Literature 5].

[Citation List]

[Patent Literature]

[PTL 1] International Publication No. WO 00/51527
[PTL 2] International Publication No. WO 03/024463
[PTL 3] Japanese Patent No. 4522994

[Non Patent Literature]

[NPL 1] Harris ED, Jr., Arthritis Rheum 2001; 44:1969-1970
[NPL 2] Kushida A, Yamato M, Konno C, Kikuchi A, Sakurai Y, Okano T., J Biomed. Mater. Res. 45:355-362, 1999
[NPL 3] Okano T, Yamada N, Sakai H, Sakurai Y. , J Biomed Mater Res.1993; 27:1243-1251
[NPL 4] Shimizu T, Yamato M, Akutsu T et al., Circ Res.2002 Feb 22;90(3):e40
[NPL 5] Teramura, T., Takehara, T., Kawata, N., Fujinami, N., Mitani, T. , Takenoshita, M., Matsumoto, K. , Saeki, K. , Iritani, A., Sagawa, N., and Hosoi, Y. (2007). Primate embryonic stem cells proceed to early gametogenesis in vitro. Cloning Stem Cells 9, 144-156.

[Summary of Invention]

[Solution to Problem]

In the present invention, it was found that a therapeutic result is significantly improved by a synthetic tissue, which develops organization to impart a property of being readily detachable from a culture dish by culturing cells under a specific culture condition, such as culturing in a medium containing an extracellular matrix synthesis promoting agent, when material cells are improved and mesenchymal stem cells induced from pluripotent stemcells (inducedmesenchymalstemcells) are used. The present invention provides applications of such a complex in this area of the present invention. Further, preferred embodiments of a synthetic tissue (composite tissue) were found for osteochondral diseases, and the present invention provides a novel material based on such knowledge.

A synthetic tissue or a composite tissue provided by the present invention has properties, such as not requiring a scaffold, having self-supporting ability, readily formed into a three-dimensional structure, having morphological plasticity, having excellent ability to biologically adhere to the surrounding, and having a differentiation potential, so that the synthetic tissue or the composite tissue is effective for a replacement or resurfacing therapy at a defective site. The present invention also has excellent therapeutic results, exhibiting further improvement in union with a defective site in comparison to a conventional synthetic tissue, dramatic reduction in the period of treatment, decrease in ossification and near natural healing in terms of chondrogenesis or the like.

A synthetic tissue or composite tissue of the present invention can be constructed into various shapes and has sufficient strength. Therefore, surgical manipulation such as implantation is readily performed for the synthetic tissue or composite tissue of the present invention. According to the present invention, a large quantity (e.g., $10^6$ to $10^8$) of cells can be reliably supplied to a local site by means of tissue implantation. Further, cell adhesion molecules, such as collagen (e.g., type I, type III), fibronectin, and vitronectin, are present in large amounts in the matrix. Particularly, the cell adhesion molecules are integrated throughout the matrix.

Therefore, synthetic tissues or composite tissues of the present invention have an excellent ability to biologically adhere to surroundings of an implantation site. Thus, a synthetic tissue or a composite tissue biologically unites with a tissue of an implanted site in a very short period of time.

What the present invention has achieved includes a clinical application of the joint tissue regeneration using such a synthetic tissue or a composite tissue. The present invention makes it possible to develop therapies for bone regeneration at a conventionally intractable site, in which both periosteum and bone cortex are inflamed, partial thickness cartilage defect which does not reach the subchondral bone, and defect of a meniscus, a tendon, a ligament, an intervertebral disk, or cardiac muscle in an avascular area or a site with poor circulat ion. Furthermore, therapeutic results improve significantly, while compliance of a patient in clinical application is also improved.

The present inventors have previously developed a three-dimensional synthetic tissue that is not dependent on a scaffold which is derived from mesenchymal stem cells (MSC) from a synovial membrane for repairing a joint cartilage (Herein, also may be simply referred to as "somatic three-dimensional(ly organized) synthetic tissue" or "somatic synthetic tissue". Herein, a three-dimensional synthetic tissue may be denoted as tissue engineered construct=TEC. However, each term is used in the same meaning). When mesenchymal stem cells induced from pluripotent stem cells (e.g., embryonic stem cells (ES cells) or induced pluripotent stem cells (iPS cells))(herein, also referred to as "induced" mesenchymal stem cells (MSC) and those derived from ES cells are also referred to as ES-MSCs and those derived from iPS cells are also referred to as iPS-MSCs) are used to produce a three-dimensional(ly organized) synthetic tissue (Herein, also referred to as "induced" (MSC) (-) three-dimensional synthetic tissue (TEC) or the like. Further, those manufactured with MSCs derived from ES cells are also referred to as ES-TEC, and those manufactured with MSCs derived from iPS cells are also referred to as iPS-TEC) in the present invention, the present inventors have found that therapeutic results are unexpectedly improved in comparison to such a somatic three-dimensional synthetic tissue (TEC).

The present inventors have previously developed a three-dimensionally organized synthetic tissue (TEC) derived from somatic mesenchymal stem cells (MSC) (herein, also referred to as somatic (MSC) (-) three-dimensionally organized synthetic tissue (TEC) or the like) for applications in cartilage regenerative cell therapy. Further, such a three-dimensional synthetic tissue can be readily conjugated with an artificial bone that is widely used in bone regeneration therapy (β-TCP, Hydroxyapatite). Thus, a method of osteochondral regenerative therapy using such a three-dimensional synthetic tissue/artificial bone complex has been developed. These prior art techniques have achieved a certain level of success in osteochondral regeneration by using a somatic three-dimensional synthetic tissue in creating a three-dimensional synthetic tissue.

Conventional somatic MSC-three-dimensional synthetic tissues, since they involve invasion of /damage to a donor (tissue collection) site, have issues such as compliance and had limited cell growth (quantitative issue). Furthermore, since the ability to differentiate into cartilage associated with culturing decreased (decrease in function, qualitative issue), there was a limit in treatment with a somatic three-dimensional synthetic tissue. The induced MSC-three-dimensional synthetic tissue of the present invention has succeeded in overcoming these issues.

An induced MSC-three-dimensional synthetic tissue has been found to unexpectedly promote the ability to form (differentiate into) cartilage. In addition, it was discovered that an induced MSC-three-dimensional synthetic tissue could suppress ossification in osteochondral therapy. Further, improvement in therapeutic results at a level that is not possible with a somatic MSC-three-dimensional synthetic tissue has been observed in an induced MSC-three-dimensional synthetic tissue. Further, it is understood that an induced MSC-three-dimensional synthetic tissue has significantly increase expression of cell markers that are not observed in a somatic MSC-three-dimensional synthetic tissue, especially bone morphogenetic protein receptor 1A (BMPR1A), bone morphogenetic protein receptor 2 (BMPR2) and the like. Thus, it has been understood that an induced MSC-three-dimensional synthetic tissue has an enhanced ability to form cartilage in comparison to a somatic MSC-three-dimensional synthetic tissue via a BMP receptor. In addition, it is also understood that an induced MSC-three-dimensional synthetic tissue has the ability to differentiate into hyaline cartilage-like cartilage.

Specifically, as exemplified in the Examples, a three-dimensional synthetic tissue (ES-TEC), when created from mesenchymal stem cells (ES-MSC) that are efficiently induced with the technique of inducing differentiation of embryonic stem cells (ES cells) under low oxygen (Non Patent Literature 5), had about 6 times the glycosaminoglycan content after inducing differentiation into cartilage in *in vitro* trials and about 10 times the expression of collagen II in quantitative RT PCR in comparison to conventional somatic MSCs, thus exhibiting a very high level of ability to differentiate into cartilage.

Further, when conjugated with an artificial bone and implanted into an osteochondral defect, regeneration due to a hyaline cartilage-like tissue was observed at one month. Further, it was confirmed from the observation at two months that the tendency for ossification at an implanted site, which is an issue in the treatment with a somatic three-dimensional synthetic tissue (somatic MSC-TEC), is significantly suppressed. Such cartilage regeneration effect by an induced three-dimensional synthetic tissue (MSC-TEC) is far beyond the expectation from combined use of conventional techniques. Thus, induced MSC-TEC is a revolutionary technique as a method of osteochondral regenerative therapy using embryonic stem cells.

As in the case of conventional techniques with somatic MSCs, the induced three-dimensional synthetic tissue of the present invention (e.g., ES-MSC) can be three-dimensionally organized by making a cell sheet by a high density plate culture added with ascorbic acid and detaching the sheet from the bottom surface of a culture dish by utilizing shear stress. In actual therapy, since a three-dimensionally organized synthetic tissue (induced MSC-TEC), which was found to express many adhesion molecules such as fibronectin, readily forms a complex with an artificial bone, it can be applied in osteochondral regeneration therapy as an induced three-dimensional synthetic tissue/artificial bone complex.

In one embodiment, the present inventors made an osteochondral defect in an intercondylar section of a femur of a rabbit with a mature skeleton under anesthesia. When mesenchymal stem cells produced from pluripotent stem cells such as embryonic stem cells (ES cells) or induced pluripotent stem cells (iPS cells) are used, a healing effect could be achieved in a very short period of time (e.g. , one month) in comparison to a somatic three-dimensional synthetic tissue. In addition, it was demonstrated that ossification, which was an issue in somatic MSC-TEC as a side effect, can be suppressed. As in those produced from synovial MSCs, a complex (hybrid) of HA and a three-dimensional synthetic tissue derived from MSCs derived from pluripotent stem cells was formed without using an adhesive immediately prior to implantation, and the diphasic implant was implanted in the bone defect without suturing. The present inventors further prepared normal untreated knees as a control group for a biodynamic test. The defective section to which an implant was applied was morphologically evaluated at 1, 2, and 6 months after surgery. Furthermore, biodynamic analysis was carried out at six months after surgery. As for the therapeutic effect, ossification is unexpectedly suppressed, a defect is healed to a near natural state, and compliance of a patient is expected to improve.

The three-dimensional synthetic tissue immediately integrated with an HA block to yield a complex having strength that can sufficiently withstand a surgical implantation. An osteochondral defect treated with this composite tissue (hybrid material) exhibited excellent biological union with an adjacent cartilage and a response to repair a subchondral bone and cartilage at an earlier stage in comparison to HA alone. In addition, when the osteochondral tissues treated with this composite tissue (hybrid material) was repaired, rigidity equivalent to that of normal osteochondral tissues was restored.

The present inventors demonstrated that composite tissues of the present invention (hybrid implant) histologically and biodynamically improve osteochondral repair significantly. In particular, repair of a subchondral bone from an early stage and reliable and excellent biological union of a tissue to an adjacent host tissue can guarantee durability over an extended period of time. Since a three-dimensional synthetic tissue is not dependent on a scaffold, a substance derived from an animal or chemical substance is not contained. In addition, HA is extensively used in clinical settings. Thus, hybrid materials developed by the present inventors are suitable for efficient and safe repair of an osteochondral defect.

It is especially noteworthy that the three-dimensional synthetic tissue of the present invention can be developed without an exogenous scaffold, so that the risk of potential side effects induced by an artificial object or an exogenous biological substance contained in a scaffold is minimized in three-dimensional synthetic tissue implantation. Furthermore, an important biological feature of the three-dimensional synthetic tissue is its property of adhering to a tissue. The characteristic contributes to a fast and reliable adhesion of a three-dimensional synthetic tissue to an artificial bone. Thus, a hybrid implant consisting of a three-dimensional synthetic tissue and an artificial bone can be quickly and readily made and is potentially suitable for repair of a clinically-related osteochondral lesion.

The present invention used a rabbit osteochondral defect model in one embodiment to investigate the effectiveness of a hybrid implant of a TEC and an artificial bone to confirm the effect thereof.

It was discovered that the present invention also significantly enhances the ability to form cartilage by allowing a self-contraction reaction to take place in amore eutrophicated medium (DMEM, or αMEM added with basic fibroblast growth factors (bFGF) or the like) instead of a medium considered to be suitable for conventional MSCs (e.g., αMEM). Thus, the novel method of producing the synthetic tissue of the present invention is significant in terms of providing a three-dimensional synthetic tissue (TEC) more suited to osteochondral therapy compared to conventional methods.

A significant effect was exhibited when a TEC was differentiated into cartilage for three weeks in a medium of DMEM to which 1% ITS (Insulin-Transferrin-Selenite) + 0.2 mM ascorbic acid 2-phosphate and 200 ng/ml rhBMP-2 is added. Assessments were made by macroscopic observation with the naked eye. Significant effects were exhibited in assessments by each of GAG content, collagen content (hydroxyproline assay), tissue observation, immunohistochemistry (IHC), quantitative RT-PCR and the like.

Thus, the present invention provides the following.

<Induced TEC itself>

(1) An implantable synthetic tissue substantially made of a mesenchymal stem cell induced from a pluripotent stem cell, and an extracellular matrix derived from the cell.

(2) The synthetic tissue of item 1, wherein a cell in the synthetic tissue expresses at least one receptor selected from the group consisting of bone morphogenetic protein receptor 1A (BMPR1A) and bone morphogenetic protein receptor 2 (BMPR2) more than a somatic mesenchymal stem cell obtained from within the body.

(3) The synthetic tissue of item 1 or 2, wherein the synthetic tissue has a higher ability to differentiate into cartilage in comparison to a somatic mesenchymal stem cell obtained from within the body.

(4) The synthetic tissue according to any one of items 1-3, wherein the synthetic tissue has an ability to differentiate into hyaline cartilage-like cartilage.

(5) The synthetic tissue according to any one of items 1-4, wherein the extracellular matrix contains collagen I and/or collagen III and there is more of the collagen I and/or collagen III than collagen II.

(6) The synthetic tissue according to anyone of items 1-5 , wherein the extracellular matrix is diffusedly distributed in the tissue.

(7) The synthetic tissue according to anyone of items 1-6, wherein the mesenchymal stem cell is made in a low oxygen condition.

(7A) The synthetic tissue of item 7, wherein the mesenchymal stem cell or the equivalent cell thereof is obtained by culturing the pluripotent stem cell in suspension culture to form an embryoid body and culturing the embryoid body in a 1% oxygen condition.

<Novel three-dimensional synthetic tissue composite tissue>

**EP 2 949 747 B1**

(8) A composite tissue for treating or preventing a disease, disorder, or condition associated with an osteochondral defect, comprising the synthetic tissue according to any one of items 1-7 and 7A and an artificial bone

(9) The composite tissue according to item 8, wherein the artificial bone is made of a material selected from the group consisting of hydroxyapatite and β-tricalcium phosphate.

(10) The composite tissue according to any one of items 8-9,

wherein the disease, disorder, or condition is selected from the group consisting of osteoarthritis, osteochondral defect, osteochondral lesion, osteonecrosis, rheumatoid arthritis, bone tumor and diseases similar thereto.

<Novel production method of three-dimensional synthetic tissue>

(11) A method for producing the synthetic tissue according to any one of items 1-7 and 7A, the method comprising: A) providing mesenchymal stem cells induced from a pluripotent stem cell; B) positioning the cell in a container containing a cell culture solution including an agent selected from ascorbic acid, ascorbic acid 2-phosphate, or a derivative or salt thereof, wherein the container has a base with an area sufficient to accommodate a three-dimensional synthetic tissue having a desired size; C) culturing the cell in the container with the cell culture solution containing the agent for a period of time sufficient for forming the synthetic tissue having the desired size to form a synthetic tissue with the cell; D) detaching the synthetic tissue from the container to elicit self-contraction by the synthetic tissue, wherein the self-contraction is performed in a condition where (a) at least one component selected from the group consisting of sugar, vitamins and amino acids and/or (b) a basic fibroblast growth factor (bFGF) is enriched for αMEM; and E) adjusting a thickness of the synthetic tissue by a physical stimulus or a chemical stimulus to obtain a desired thickness.

(12) The method of item 11, wherein the self-contraction is performed in a condition where bFGFs are added to αMEM or in a condition of DMEM.

The present invention is understood as further encompassing use of any combination of the above-described features. Hereinafter, the present invention will be described by way of preferable examples. It will be understood by those skilled in the art that the embodiments of the present invention can be appropriately made or carried out based on the description of the present specification and commonly used techniques well known in the art. The function and effect of the present invention can be readily recognized by those skilled in the art.

[Advantageous Effects of Invention]

The ability to differentiate into cartilage by induced MSCs (e.g. , ES-MSCs) is reportedly the same or slightly better (1.2 fold in mRNA expression) in comparison to somatic MSCs (Non Patent Literature 5). However, in the present invention, these values increased far beyond expectations by forming a three-dimensional synthetic tissue with ES-MSCs, and regeneration by a hyaline cartilage-like tissue was observed at one month, at which point only a fibrous tissue is observed in conventional technique in animal experiments. Further, a hyaline cartilage-like repaired tissue suppresses unnecessary ossification tendency in the surrounding at two months. In view of the above, it is considered to be a method of osteochondral regeneration therapy with an excellent effect and speed that cannot be realized by conventional techniques. The above-described effect provides great contribution in early recovery, shorter hospitalization period, and rehabilitation back to society for patients in clinical applications.

Injuries sustained as a cartilage defect are often accompanied by a coexisting damage or a subsequent secondary degeneration in a subchondral bone. Further, the number of cases where the site of disease reaches from cartilage to bones is significant among the estimated 20 million osteoarthritis patients in Japan. With an ES-TEC/artificial bone complex, it is possible to expect a stable and excellent therapeutic effect and speed that cannot be realized with conventional techniques as a cell implantation therapy for such osteochondral lesions.

[Brief Description of Drawings]

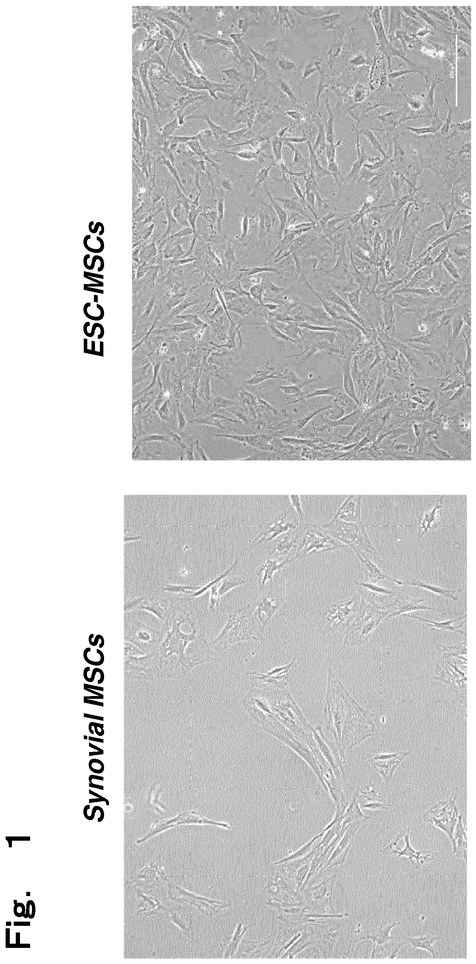

[Figure 1] Figure **1** shows a comparison of MSCs obtained from the synovial membrane of a rabbit (left) and MSCs induced from ES cells (ESC-MSC; right). ESC-MSCs have a self-replication capability, and cell surface antigens had MSC characteristics (e.g. , was PDFGRα+, CD105+ and CD271+) as well as osteogenicity, chondrogenicity, and adipogenicity.

7

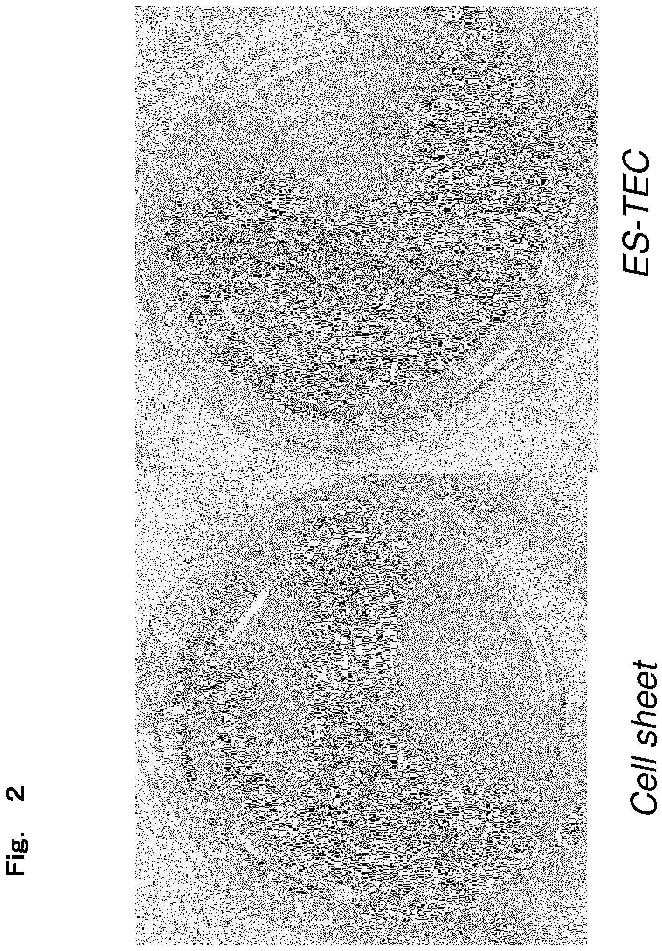

[Figure 2] Figure **2** shows a comparison of a cell sheet (left) and TEC induced from ES cells, which is an example of the present invention.

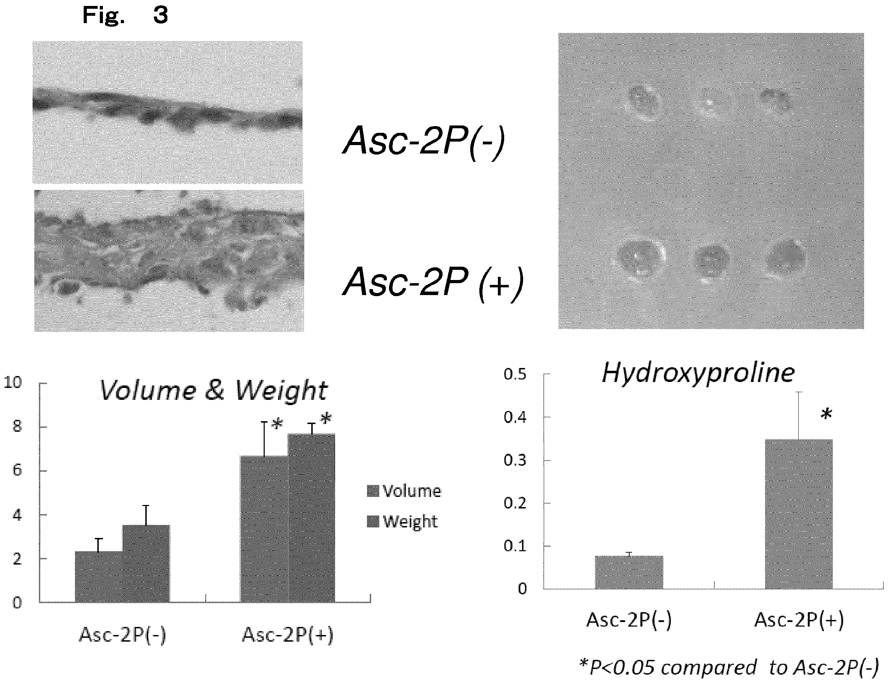

[Figure 3] Figure **3** shows increase in the production of extracellular matrix (collagen) by an addition of ascorbic acid 2-phosphate. The top left section shows the difference of a tissue fragment with or without ascorbic acid 2 -phosphate. The top right section shows the difference in the general view of the tissue with or without ascorbic acid 2-phosphate (top row: without addition of ascorbic acid 2-phosphate, bottom row: with addition of ascorbic acid 2-phosphate). The bottom left section shows the difference in the volume (left column) and weight (right column) with or without ascorbic acid 2-phosphate. Two bars on the left side of the graph are without ascorbic acid 2 -phosphate, and the two bars on the right side are with addition of ascorbic acid 2-phosphate. The y-axis indicates volume μl/weight mg. The bottom right section is a comparison of the amount of hydroxyproline with or without ascorbic acid 2-phosphate. The y-axis indicates hydroxyproline concentration mg/l. Each * indicates statistical significance ($p < 0.05$).

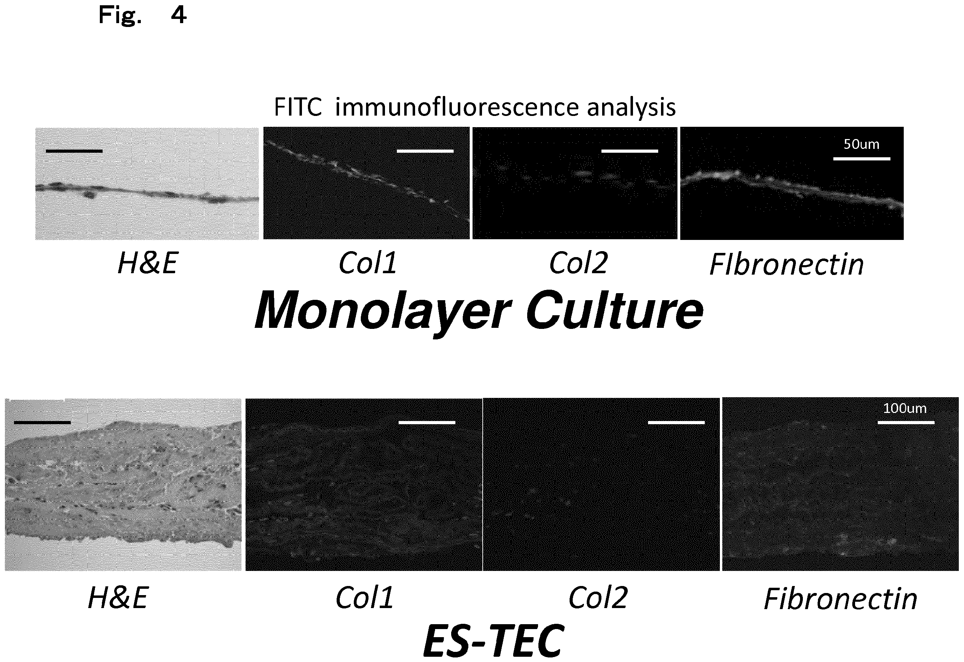

[Figure 4] Figure **4** shows a comparison of a monolayer culture produced with ES cells to a three-dimensional synthetic tissue (TEC), which is a representative example of the present invention (prior to differentiation into cartilage). The top row shows the result for the monolayer culture and the bottom row shows the result for TEC. The results of FITC immunofluorescence analysis are shown for H&E stain, collagen 1, collagen 2, and fibronectin in this order from the left.

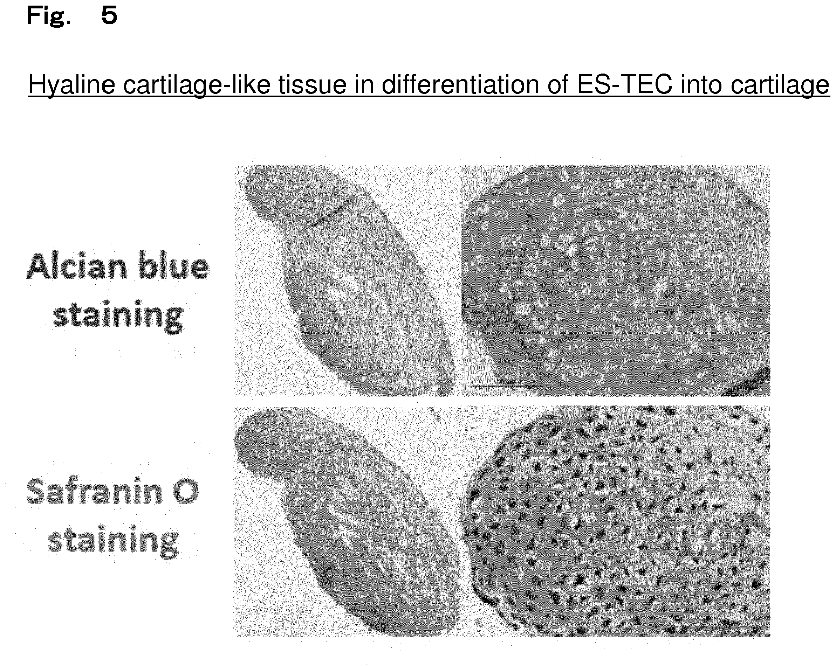

[Figure 5] Figure **5** shows that differentiation of a three-dimensional synthetic tissue (TEC) produced with MSCs induced from ES cells, which is a representative example of the present invention, into cartilage results in hyaline cartilage-like tissue. The top row shows Alcian blue staining and the bottom row shows Safranin O staining.

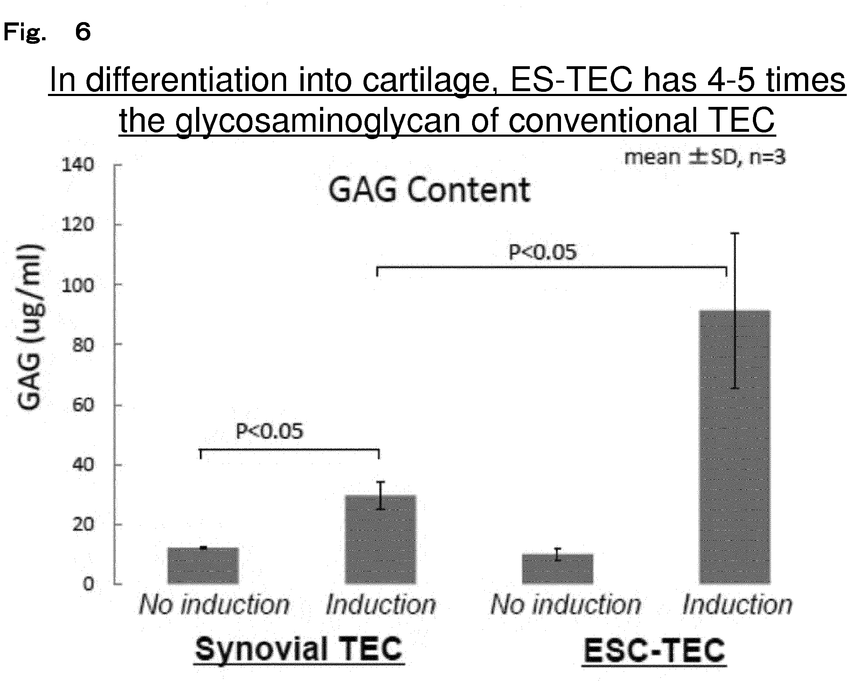

[Figure 6] Figure **6** shows that a three-dimensional synthetic tissue (TEC) produced with MSCs induced from ES cells, which is a representative example of the present invention, produces significantly more glycosaminoglycan (GAG) than conventional somatic three-dimensional synthetic tissues upon differentiation into cartilage. The left side shows a three-dimensional synthetic tissue derived from a synovial membrane, and the right side shows the three-dimensional synthetic tissue (TEC) produced with MSCs induced from ES cells, which is a representative example of the present invention. For each side, the left side is without induction of differentiation into cartilage and the right side is with induction of differentiation into cartilage. $p < 0.05$ indicates statistical significance. The standard deviation is indicated by the bar (n=3). The y-axis indicates the amount of expression of GAG (μg/ml).

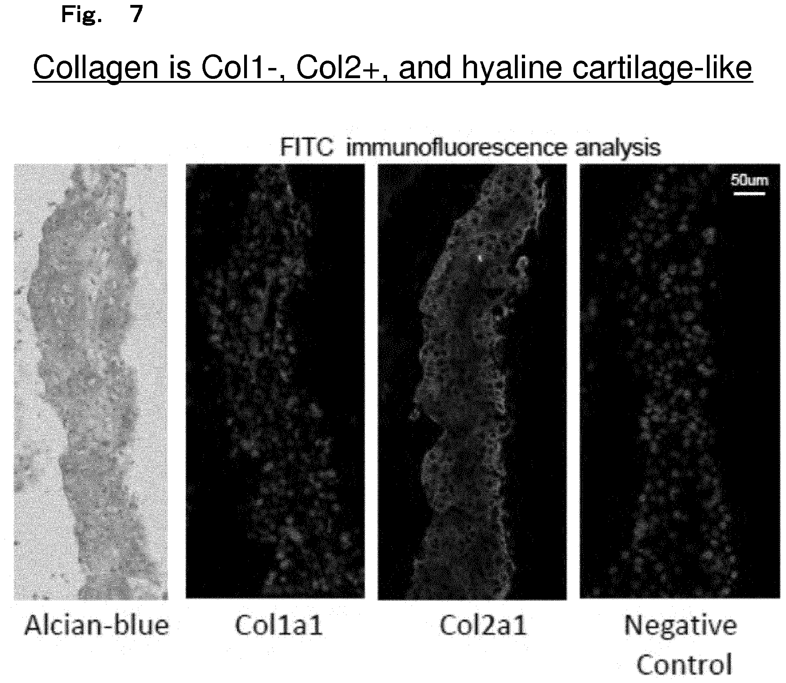

[Figure 7] Figure **7** shows FITC immunofluorescence analysis demonstrating that a three-dimensional synthetic tissue (TEC) produced with MSCs induced from ES cells, which is a representative example of the present invention, has a hyaline cartilage-like phenotype when differentiated into cartilage. Alcian-blue stain is shown in the left, collagen 1a1 is shown second from the left, collagen 2a1 is shown second from the right, and a negative control is shown at the rightmost side.

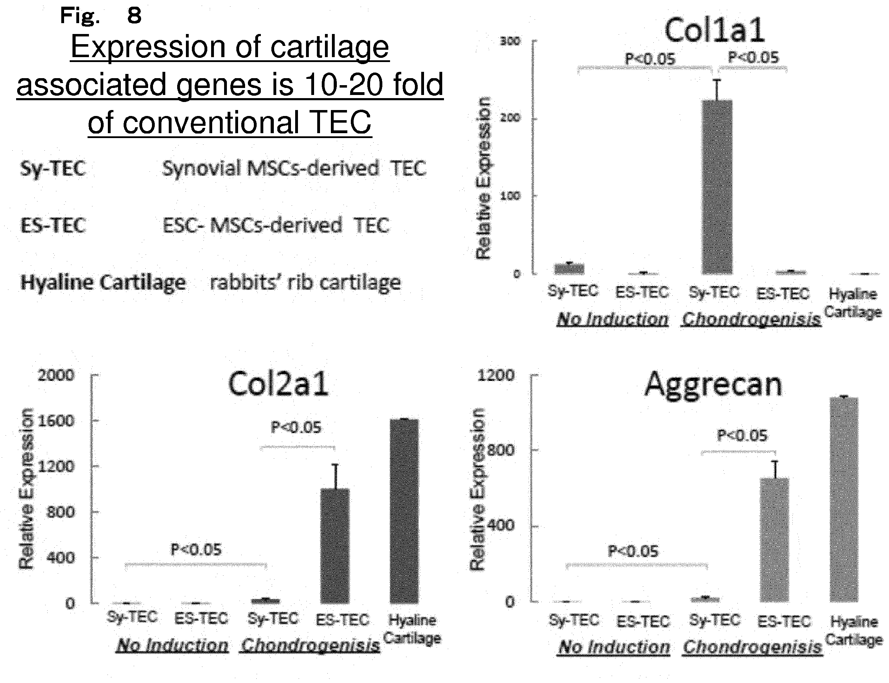

[Figure 8] Figure **8** shows patterns of gene expression before and after inducing differentiation of three-dimensional synthetic tissue (TEC) produced with MSCs induced from ES cells, which is a representative example of the present invention, into cartilage, and shows that expression of a cartilage associated gene which is 10-20 fold of somatic TEC is observed. Sy-TEC indicates TEC derived from synovial MSCs. ES-TEC indicates TEC derived from ES cell derived-MSCs. Hyaline Cartilage indicates hyaline cartilage-like tissue. No Introduction indicates no induction. Chondrogenesis indicates the state after stimulus for differentiation into cartilage. $p<0.05$ indicates statistical significance. The y-axis indicates the relative expression intensity.

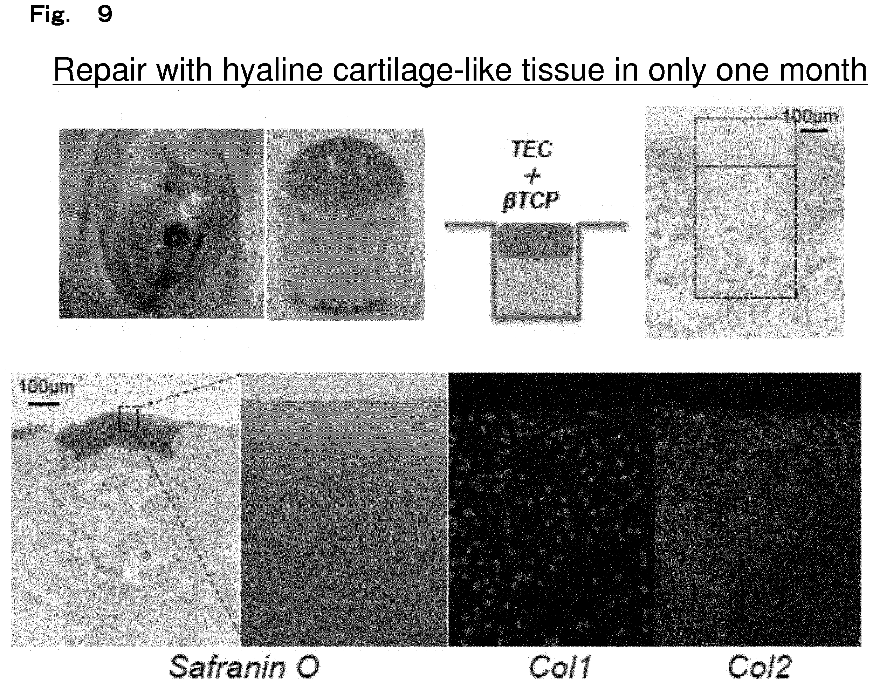

[Figure 9] Figure **9** shows a result of treating a bone defect using a composite tissue made from conjugating a three-dimensional synthetic tissue (TEC) produced with MSCs induced from ES cells, which is a representative example of the present invention, to βTCP. As shown, repair with a hyaline cartilage-like tissue is observed only after one month. The left most section on the top row shows a bone defect site. The second section from the left in the top row shows an example of a composite tissue made by conjugating TEC produced with MSCs induced from ES cells, which is a representative example of the present invention, to βTCP. The second section from the right in the top row shows a schematic diagram of a composite tissue of TEC + βTCP. The rightmost section in the top row is an actual picture of a composite tissue upon implantation into an actual osteochondral defect, which is shown in correspondence to the schematic diagram. The bottom row shows Safranin O staining after treatment of a defective site for onemonth on the leftmost section. The bar is 100 μm. The second section from the left is an expanded diagram thereof. The second section from the right shows the staining of collagen type 1 and the rightmost section shows the staining of collagen type 2.

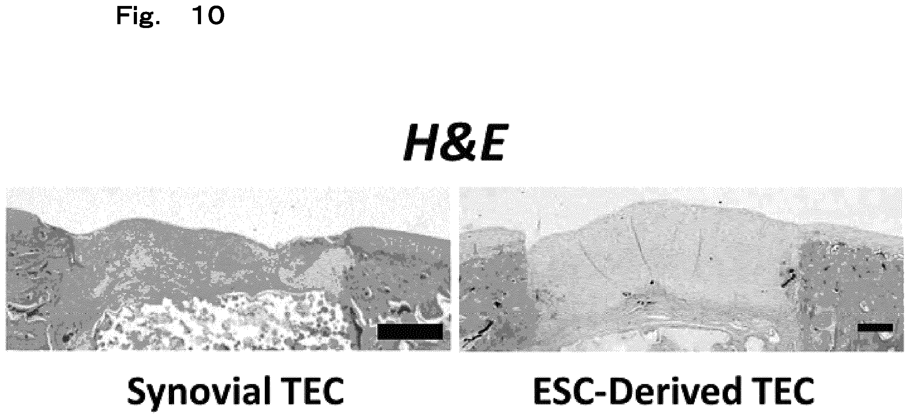

[Figure 10] Figure **10** shows the result of hematoxylin-eosin staining comparing the result of treating a bone defect by using a composite tissue made by conjugating a three-dimensional synthetic tissue (TEC) produced with MSCs induced from ES cells, which is a representative example of the present invention, with βTCP to that using a somatic (synovial membrane-derived) TEC. The left side shows the result using the synovial membrane-derived TEC composite tissue, and the right side shows the result using the composite tissue made by conjugating the TEC produced with MSCs induced from ES cells with βTCP. The portion that is not stained pink is the cartilage.

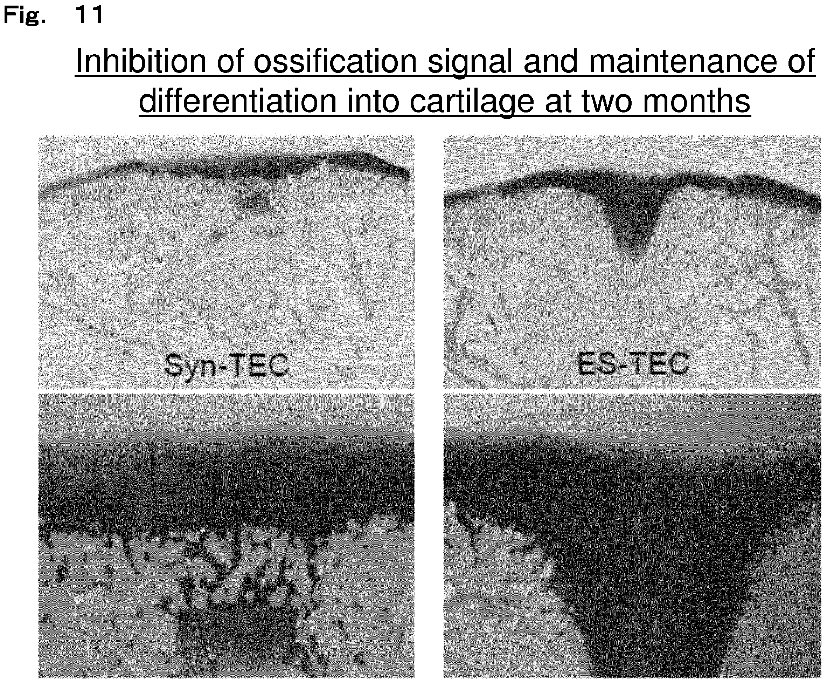

[Figure 11] Figure **11** is a result (toluidine blue staining) comparing the result of treating a bone defect by us ing a composite tissue made by conjugating a three-dimensional synthetic tissue (TEC) produced with MSCs induced from ES cells, which is a representative example of the present invention, with βTCP to that using a somatic (synovial membrane-derived) TEC in terms of suppressing ossification signals and maintenance of differentiation into cartilage after two months. The left side shows the result using the synovial membrane-derived TEC composite tissue, and the right side shows the result using the composite tissue made by conjugating TEC produced with MSCs induced from ES cells with βTCP. The top row is the picture of the whole, and the bottom row is an expanded view.

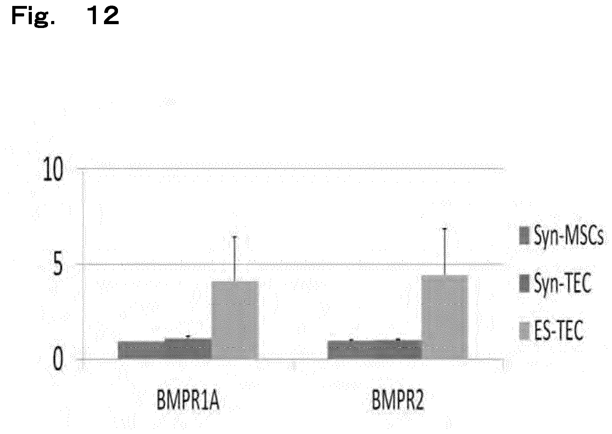

[Figure 12] Figure **12** shows that the expression of BMP receptors is significantly higher in a three-dimensional synthetic tissue (TEC) produced with MSCs induced from ES cells, which is a representative example of the present invention, than in a somatic (synovial membrane-derived) TEC or synovial MSCs. Syn-MSCs indicate synovial membrane-derived MSCs themselves. Syn-TEC indicates synovial membrane-derived TEC. ES-MSC indicates a TEC produced with MSCs induced from ES cells, which is a representative example of the present invention. The left side shows BMPR1A, which is one of the BMP receptors, and the right side shows BMPR2, which is another BMP receptor. The y-axis indicates the ratio of increase in the expression of each marker relative to the value before the induction of differentiation.

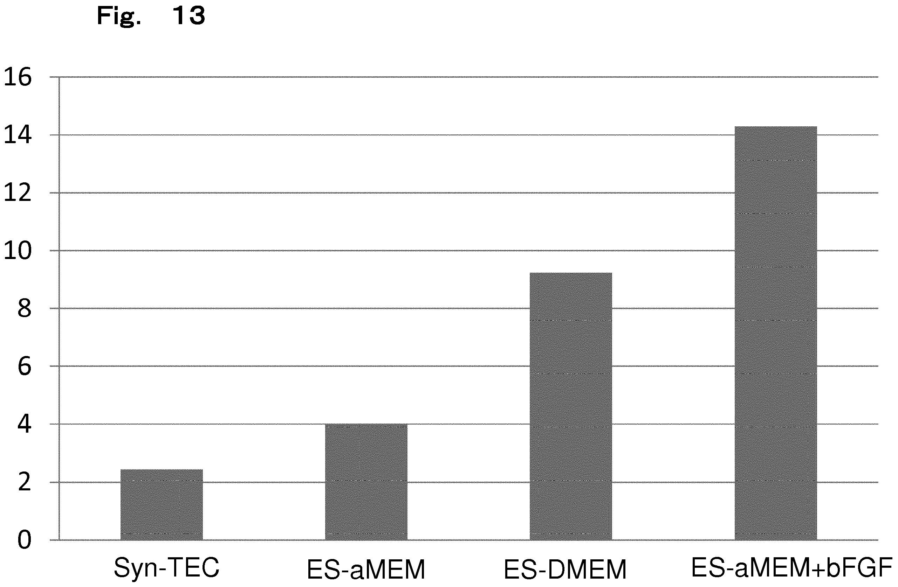

[Figure 13] Figure 13 shows the ratio of increase in GAG (glycosaminoglycan) after inducing differentiation in *vitro*. The Figure shows, from the left side, a synovial membrane - derived three-dimensional synthetic tissue (Syn-TEC), MSCs prepared fromES cells organized into a three-dimensional synthetic tissue with αMEM (ES-aMEM), MSCs prepared from ES cells organized into a three-dimensional synthetic tissue with DMEM (ES-DMEM), and MSCs prepared from ES cells organized into a three-dimensional synthetic tissue with αMEM + bFGF (ES-aMEM+bFGF). The y-axis shows the ratio of increase in GAG relative to the value before induction of differentiation.

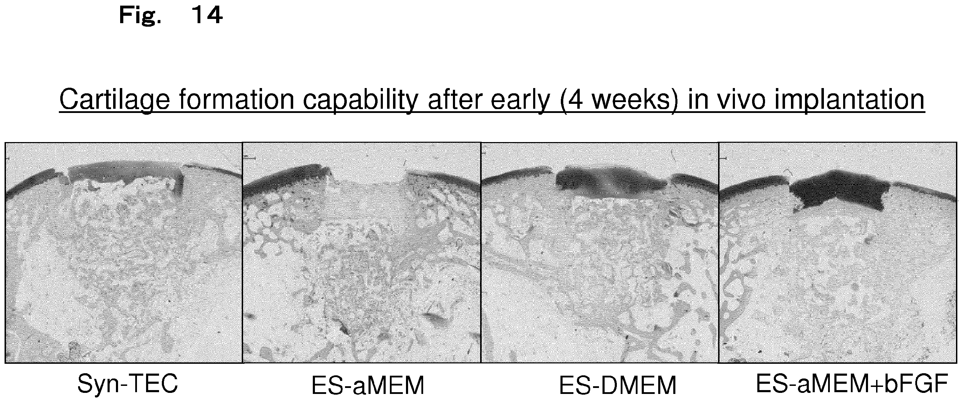

[Figure 14] Figure **14** shows the ability to form cartilage *in vivo* after 4 weeks from implantation. The Figure shows, from the left side, a synovial membrane-derived three-dimensional synthetic tissue (Syn-TEC), MSCs prepared from ES cells organized into a three-dimensional synthetic tissue with αMEM (ES-aMEM), MSCs prepared from ES cells organized into a three-dimensional synthetic tissue with DMEM (ES-DMEM), and MSCs prepared from ES cells organized into a three-dimensional synthetic tissue with αMEM + bFGF (ES-aMEM+bFGF).

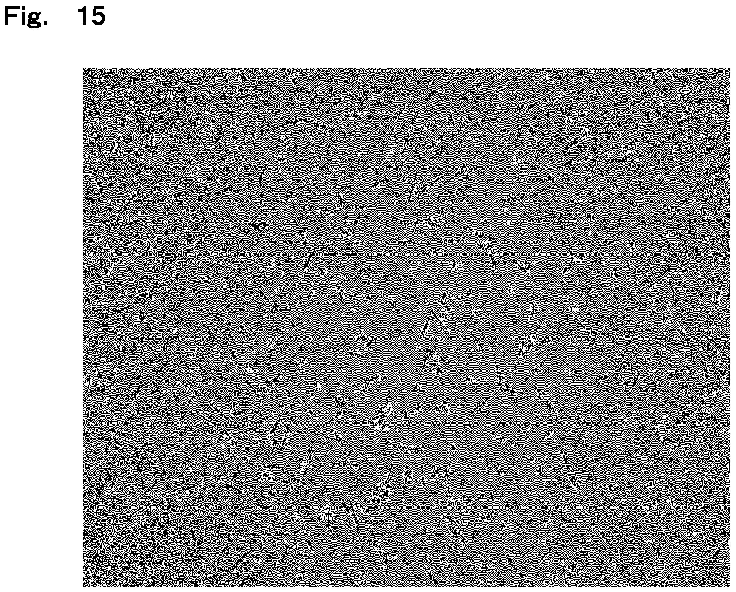

[Figure 15 Figure 15 shows MSCs (iPS-MSC) induced from human iPS cells (253G1) obtained from RIKEN. When the iPS cells (253G1) obtained from RIKEN are cultured at oxygen partial pressure of 1%, spindle shaped cells are observed around the colony. When such cells are sorted for CD44/CD73/CD105 positive cells with a flow cytometer, they would be iPS-MSCs differentiating into cartilage, bone, and fat.

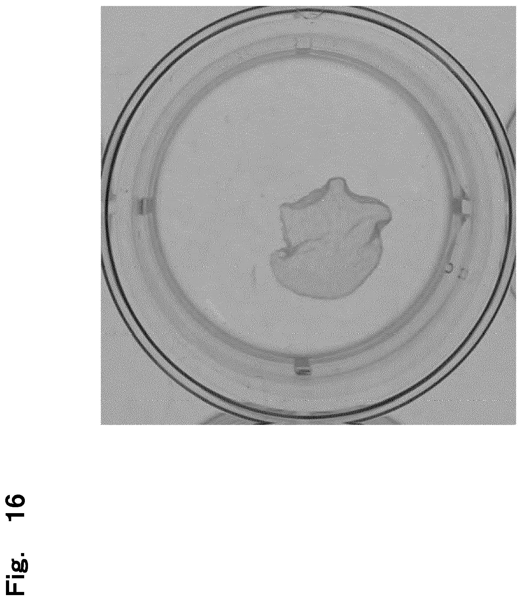

[Figure 16] Figure 16 shows a three-dimensional tissue (TEC) made by using MSCs induced from iPS cells (also referred to as iPS-TEC).

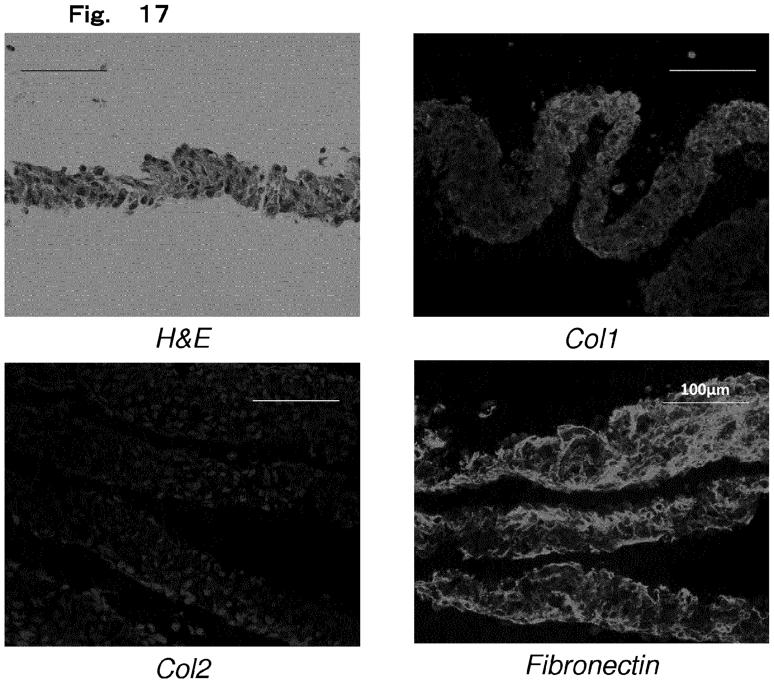

[Figure 17] Figure 17 shows the result of making a monolayer culture produced with iPS cells and three-dimensional synthetic tissue (TEC), which is a representative example of the present invention (before differentiation into cartilage). The result of FITC immunofluorescence analysis is shown for H&E staining at the top left, collagen 1 at the top right, collagen 2 at the bottom left, and fibronectin at the bottom right. The figure shows the state of iPS-TEC when differentiation is not induced. It is understood that it is mainly fibrous collagen such as type 1 collagen while type 2 collagen of hyaline cartilage is not expressed, as in the conventional technique. Further, adhesive proteins such as fibronectin are also expressed, which are related to implantation without suture being possible. Furthermore, it can be seen that a tissue, when organized from a monolayer into three-dimension experiences a significant increase in thickness and undergoes self-contraction frommerely by being detached from the bottom surface, is organized in three-dimension.

[Description of Embodiments]

The present invention is described below. Throughout the entire specification, a singular expression should be understood as encompassing the concept thereof in a plural form unless specifically noted otherwise. Thus, singular modifiers such as articles (e.g., "a", "an", "the" and the like in case of English) should be understood as encompassing the concept thereof in a plural form unless specifically noted otherwise. Further, the terms used herein should be understood as being used in the meaning that is commonly used in the art, unless specifically noted otherwise. Thus, unless defined otherwise, all terminologies and scientific technical terms that are used herein have the same meaning as the terms commonly understood by those skilled in the art to which the present invention belongs. In case of a contradiction, the present specification (including the definitions) takes precedence.

(Definition of terms)

The definitions of specific terms used herein are described below.

(Regenerative medicine)

As used herein, the term "regeneration" refers to a phenomenon in which when an individual organism loses a portion of tissue, the remaining tissue grows and recovers. The extent and manner of regeneration vary depending on animal species or tissues in the same individual. Most human tissues have limited regeneration capability, and complete regeneration is not expected if a large portion of tissue is lost. In the case of severe damage, a tissue with strong proliferation capability different from that of a lost tissue may grow, resulting in incomplete regeneration where the damaged tissue is incompletely regenerated and the function of the tissue cannot be recovered. In this case, regenerative medicine is administered, wherein a structure made of a bioabsorbable material is used to prevent a tissue with strong proliferation capability from infiltrating the defect portion of the tissue so as to secure a space for proliferation of the damaged tissue, and a cell growth factor is supplemented to enhance the regeneration capability of the damaged tissue. Such a regeneration therapy is applied to cartilages, bones, hearts, and peripheral nerves, for example. It was believed until now that cartilages, nerve cells, and cardiac muscles have no or poor regeneration capability. There are reports of the presence of tissue stem cells (somatic stem cells), which have both the capability of differentiating into these tissues and self-proliferation capability. Some are about to be used in practice. Expectations are running high for regenerative medicine using tissue stem cells. Embryonic stem cells (ES cells) have the capability of differentiating into all tissues. Induced pluripotent stem (iPS) cells are stem cells that have the ability to differentiate into all tissues. IPS cells can be produced without the use of an embryo or a fetus.

As used herein, the term "cell" is defined in its broadest sense in the art, referring to a structural unit of a tissue of a multicellular organism or a life form, which is surrounded by a membrane structure for separating the living body from the external environment, has self-regeneration capability inside, and has genetic information and a mechanism for expressing the information. In the method of the present invention, any cell can be used as a subject. The number of "cells" used in the present invention can be counted through an optical microscope. When counting with an optical microscope, counting is performed by counting the number of nuclei. For example, the tissues are sliced into tissue sections, which are then stained with hematoxylin-eosin (HE) to variegate nuclei derived from extracellular matrices (e.g., elastin or collagen) and cells with dye. These tissue sections can be observed under an optical microscope to count the number of cells by estimating the number of nuclei in a particular area (e.g., 200 $\mu$m x 200 $\mu$m) to be the number of cells. Cells used herein may be either naturally-occurring cells, pluripotent stem cells (e.g., ES cells, iPS cells, etc.) or artificially modified cells (e.g., fusion cells, genetically modified cells, etc.). Examples of a cell source include, but are not limited to, a single-cell culture; the embryo, blood, or a body tissue of a normally-grown transgenic animal; and a cell mixture such as cells derived from normally-grown cell lines. Primary culture cells may be used as the cells. Alternatively, subculture cells may also be used. As used herein, cell density may be represented by the number of cells per unit area (e.g., cm$^2$).

As used herein, the term "stem cell" refers to a cell that has self-replication capability and ability to differentiate into multiple directions = pluripotency. Typically, stem cells can regenerate a tissue when the tissue is injured. Stem cells used herein are mesenchymal stem cells (also referred to as tissular stem cell, tissue-specific stem cell, or somatic stem cell). A stem cell may be an artificially produced cell as long as it has the above-described capabilities. ES cells are pluripotent stem cells derived from early embryos. An embryonic stem cell was first established in 1981, and has been applied to production of knockout mice since 1989. In 1998, a human ES cell was established, which is currently becoming available for regenerative medicine. Recently, iPS cells have drawn attention. IPS cells also can be made by induction (initialization) using the so-called Yamanaka factor or the like from skin cells or the like (Takahashi K, Yamanaka S. (2006). "Induction of pluripotent stem cells from mouse embryonic and adult fibroblast cultures by defined factors". Cell 126: 663-676. Okita K, Ichisaka T, Yamanaka S. (2007). "Generation of germline-competent induced pluripotent stem cells". Nature 448: 313-317, TakahashiK, TanabeK, OhnukiM, NaritaM, Ichisaka T, Tomoda K, Yamanaka S. (2007). "Induction of Pluripotent Stem Cells from Adult Human Fibroblasts by Defined Factors". Cell 131: 861-872). Tissue stem cells have a relatively limited level of differentiation unlike ES cells. Tissue stem cells are present in specific location of tissues and have an undifferentiated intracellular structure. Thus, the level of pluripotency of tissue stem cells is low. Tissue stem cells have a higher nucleus/cytoplasm ratio and have few intracellular organelles. Most tissue stem cells have pluripotency, a long cell cycle, and proliferative ability maintained beyond the life of an individual. Induction from pluripotent stem cells such as ES cells or iPS cells into a mesenchymal stem cell, which is also called mesenchyme-like stem cell, can be carried out by using a known technique in the art. For example, induction from iPS cells into a mesenchymal stem cell, which is also called mesenchyme-like stem cell, can be carried out by referring to Jung et al, STEM CELLS, 2011; doi:10.1002/stem.727. Further, induction from ES cells into a mesenchymal stem cell, which is also called mesenchyme-like stem cell, can be carried out by referring to, for example, de Peppo et al., TISSUE ENGINEERING:Part A, 2010; 16; 3413-3426; Toh et al. , Stem Cell Rev. and Rep., 2011; 7:544-559; Varga et al., Biochem.Biophys.Res.Commun.,2011; doi:10.1016/j.bbrc.2011.09.089; Barbet et al., Stem Cells International, 2011, doi:10.4061/2011/368192; Sanchez et al., STEM CELLS, 2011; 29:251-262; Simpson et al., Biotechnol. Bioeng., 2011; doi:10.1002/bit.23301. Alternatively, the low oxygen method or the like reported in Teramura et al., Cell transplant 2012 may be used as an improved method thereof.

Historically, tissue stem cells are separated into categories of sites from which the cells are derived, such as the dermal system, the digestive system, the bone marrow system, and the nervous system. Tissue stem cells in the dermal system include epidermal stem cells, hair follicle stem cells, and the like. Tissue stem cells in the digestive system include pancreatic (common) stem cells, hepatic stem cells, and the like. Tissue stem cells in the bone marrow system include hematopoietic stem cells, mesenchymal stem cells (e.g. , derived from fat or bone marrow), and the like. Tissue stem cells in the nervous system include neural stem cells, retinal stem cells, and the like. It is now possible to produce these tissue stem cells by differentiation from ES cells, iPS cells or the like. Thus, such classification by origin has recently been redefined in terms of differentiation capability of the stem cell as an index. Herein, stem cells having the same differentiation capability as a specific tissue stem cell (e.g., mesenchymal stem cell) are understood to be capable of achieving the objective of the present invention to be achieved by the specific tissue stem cell, regardless of whether the original is an ES cell, iPS cell or the like with differentiation capability of each stem cell as the index. However, in this light, the present invention has been found to achieve a more significant effect when using a mesenchymal stem cell induced from pluripotent cells such as ES cells or iPS cells.

As used herein, the term "somatic cell" refers to any cell other than a germ cell, such as an egg or a sperm, which does not directly transfer its DNA to the next generation. Typically, somatic cells have limited or no pluripotency. Somatic cells used herein may be naturally-occurring or genetically modified. Anything derived from a somatic cell is called "somatic" herein.

Cells can be classified by the origin thereof into stem cells derived by the ectoderm, endoderm, or mesoderm. Cells of ectodermal origin, including neural stem cells, are mostly present in the brain. Cells of endodermal origin, including blood vessel stem cells, hematopoietic stem cells, and mesenchymal stem cells, are mostly present in bone marrow. Cells of mesoderm origin, including hepatic stem cells and pancreatic stem cells, are mostly present in organs. When used herein, the term "mesenchymal stem cell; MSC" refers to a somatic stem cell with the ability to differentiate into a mesenchymal cell or a stem cell induced in this manner. The differentiation capability includes differentiation into mesenchymal tissue such as bone, cartilage, blood vessel, and myocardium. Mesenchymal stem cells may be applied to regenerative medicine such as reconstruction of such tissues. Representative examples thereof include somatic stem cells derived from mesenchyme (e.g., marrow mesenchymal stem cells present in marrow stromal cells, mesenchymal stem cells present in synovial cells) and stem cells induced in this manner.

Mesenchymal stem cells (MSC) are found in mesenchymes. Here, mesenchyme refers to a population of free cells which have an asteroid-shaped or irregular projections and bridge gaps between epithelial tissues and which are recognized in each stage of development of multicellular animals. Mesenchyme also refers to a tissue formed with intercellular substance associated with the cells. Mesenchymal stem cells have proliferation capability and the capability to differentiate into osteocytes, chondrocytes, muscle cells, stroma cells, tendon cells, and adipocytes. Mesenchymal stem cells are employed in order to culture or grow bone marrow cells or the like collected from patients or to allow differentiation into chondrocytes or osteoblasts. Mesenchymal stem cells are also employed as reconstruction materials for alveolar bones; bones, cartilages or joints for arthropathy or the like; and the like. There is a large demand for mesenchymal stem cells. Thus, the composite tissue comprising mesenchymal stem cells or differentiated mesenchymal stem cells of the present invention is particularly useful when a structure is required in these applications.

Whether a cell is a mesenchymal stem cell (MSC) can verified by using one or more of self-replication capability, differentiation capability (osteogenic, chondrogenic, adipogenic), and cell markers (e.g., PDGF receptor $\alpha$+, vimentin+, CD105+, and CD271+) as an indicator. Whether a cell is a mesenchymal stem cell (MSC) can verified by using a method exemplified in the Examples provided herein.

As used herein, the term "self-replication capability" is defined as an ability to produce stem cells similar to itself (replicate). Self-replication capability can be verified by a method exemplified in the Examples provided herein.

As usedherein, the term "osteogenic" is defined as an ability to newly create a bone tissue with osteoprogenitor cells involving calcification. Such formation capability can be verified by subjecting a test subject to osteogenic conditions as disclosed herein. Further, osteogenicity can be verified by a method exemplified in the Examples provided herein.

As used herein, the term "chondrogenic" is defined as an ability to newly create a cartilage-specific extracellular matrix (including type II collagen and aggrecan) with chondroprogenitor cells. Such formation capability can be verified by subjecting a test subject to chondrogenic conditions as disclosed herein. Further, chondrogenicity can be verified by a method exemplified in the Examples provided herein.

As used herein, the term "adipogenic" is defined as an ability to newly create an adipose tissue with adipocyte progenitor cells. Such formation capability can be verified by subjecting a test subject to adipogenic conditions as disclosed herein. Further, adipogenicity can be verified by a method exemplified in the Examples provided herein.

As used herein, the term "isolated" means that substances that are naturally accompanied in a normal circumstance are at least reduced, or preferably substantially eliminated. Therefore, an isolated cell, tissue or the like refers to a cell that is substantially free of other accompanying substances (e.g. , other cells, proteins, etc.) in normal circumstances.Fortissues, isolated tissue refers to a tissue substantially free of substances other than that tissue (e.g. , in the case of synthetic tissues or complexes, substances, scaffolds, sheets, coating, or the like that are used when the synthetic tissue is produced). As used herein, the term "isolated" preferably refers to a scaffold-free state. Therefore, it is understood that the synthetic tissue of the present invention or medical device comprising the synthetic tissue (e.g. , complex) in an isolated state may contain components such as a medium used in the production thereof.

As used herein, the term "scaffold-free" indicates that a synthetic tissue is substantially free of a material (scaffold) which is conventionally used for production of a synthetic tissue. Examples of such a scaffold material include, but are not limited to, chemical polymeric compounds, ceramics, or biological formulations such as polysaccharides, collagens, gelatins, and hyaluronic acids. A scaffold is a material which is substantially solid and has strength which allows it to support cells or tissues.

As used herein, the term "established" in relation to cells refers to a state of a cell in which a particular property (e.g. , pluripotency) is maintained and the cell undergoes stable proliferation under culture conditions. Therefore, established stem cells maintain pluripotency. For example, the MSCs used in the present invention may be established induced MSCs or MSCs induced from established ES cells or iPS cells.

As used herein, the term "non-embryonic" refers to not being directly derived from early embryos. Therefore, the term "non-embryonic" refers to cells derived from parts of the body other than early embryos. Modified embryonic stem cells (e.g. , genetically modified or fusion embryonic stem cells) are encompassed by non-embryonic cells.

As used herein, the term "differentiated cell" refers to a cell having a specialized function and form (e.g., muscle cells and neurons). Unlike stem cells, differentiated cells have no or little pluripotency. Examples of differentiated cells include epidermic cells, pancreatic parenchymal cells, pancreatic duct cells, hepatic cells, blood cells , cardiac muscle cells, skeletal muscle cells, osteoblasts, skeletal myoblasts, neurons, vascular endothelial cells , pigment cells , smooth muscle cells, adipocytes, osteocytes, chondrocytes, and the like.

As used herein, the term "tissue" refers to a group of cells having the same function and form in cellular organisms. In multicellular organisms, constituent cells usually differentiate so that the cells have specialized functions, resulting in division of labor. Therefore, multicellular organisms are not simple cell aggregations, but instead constitute organic or social cell groups having a certain function and structure. Examples of tissues include, but are not limited to, integument tissue, connective tissue, muscular tissue, nervous tissue, and the like. Tissues targeted by the present invention may be derived from any organ or part of an organism. In a preferable embodiment of the present invention, tissues targeted by the present invention include, but are not limited to, a bone, a cartilage, a tendon, a ligament, a meniscus, an intervertebral disk, a periosteum, a dura mater, and the like.

As used herein, the term "cell sheet" refers to a structure made of a monolayer of cells. Such a cell sheet has at least two-dimensional biological integration. A sheet having biological integration is characterized in that after the sheet is manufactured, the connection between cells is not substantially destroyed even when the sheet is handled individually. Such biological integration includes intracellular integration via an extracellular matrix. Such a cell sheet may partially include a two- or three-layer structure.

As used herein, the term "synthetic tissue" refers to a tissue in a state that is different from natural states. Typically, a synthetic tissue is herein prepared by cell culture. A tissue which is directly removed in an existing form from an organism is not referred to as a synthetic tissue herein. Therefore, a synthetic tissue may include materials derived from organisms and materials not derived from organisms. The synthetic tissue of the present invention typically is made of a cell and/or a biological material, and may comprise other materials. More preferably, the synthetic tissue of the present invention is substantially made of only a cell and/or a biological material. Such a biological material is preferably a substance derived from cells constituting the tissue (e.g. , extracellularmatrix).

As used herein, the term "implantable synthetic tissue" refers to a synthetic tissue, which can be used for actual clinical implantation and can function as a tissue at an implantation site for at least a certain period of time after implantation. Implantable synthetic tissues typically have sufficient biocompatibility, sufficient affinity, and the like.

The sufficient strength of an implantable synthetic tissue varies depending on a part targeted by implantation. However, the strength can be appropriately determined by those skilled in the art. The strength is sufficient to provide self-supporting ability, and can be determined depending on the environment of implantation. Such strength can be measured by measuring stress or distortion characteristics or by conducting a creep characteristics indentation test. The strength may also be evaluated by observing the maximum load.

The sufficient size of an implantable synthetic tissue varies depending on a part targeted by implantation. However, the size can be appropriately determined by those skilled in the art. The size can be determined depending on the environment of implantation. However, an implantable synthetic tissue preferably has at least a certain size. Such a size, in terms of area, may be at least 1 $cm^2$, preferably at least 2 $cm^2$, more preferably at least 3 $cm^2$, even more preferably at least 4 $cm^2$, at least 5 $cm^2$, at least 6 $cm^2$, at least 7 $cm^2$, at least 8 $cm^2$, at least 9 $cm^2$, at least 10 $cm^2$, at least 15 $cm^2$, or at least 20 $cm^2$ , but the size is not limited thereto. The area can be 1 $cm^2$ or less or 20 $cm^2$ or greater depending on the application. The essence of the present invention is understood such that a synthetic tissue of any size (area, volume) can be produced, i.e., the size is not particularly limited.

When the size is represented by volume, the size may be, but is not limited to, at least 2 $mm^3$ or at least 40

EP 2 949 747 B1

$mm^3$. It is understood that the size may be 2 $mm^3$ or less or 40 $mm^3$ or greater.

The sufficient thickness of an implantable synthetic tissue varies depending on a part targeted by implantation. However, the thickness can be appropriately determined by those skilled in the art. The thickness can be determined depending on the environment of implantation. The thickness may exceed 5 mm. For example, when an implantable synthetic tissue is applied to a bone, a cartilage, a ligament, a tendon, or the like, the tissue generally has a thickness of at least about 1 mm, e.g. , at least about 2 mm, more preferably at least about 3 mm, at least about 4 mm, and even more preferably about 5 mm, or about 5 mm or greater or about 1 mm or less. The essence of the present invention is understood such that a tissue or complex of any thickness can be produced, i.e., the size is not particularly limited.

The sufficient biocompatibility of an implantable synthetic tissue varies depending on a part targeted by implantation. However, the degree of biocompatibility can be appropriately determined by those skilled in the art. Typically, a desired level of biocompatibility is, for example, such that biological integration to surrounding tissues is achieved without any inflammation or any immune reaction, but the present invention is not limited thereto. Examples of parameters indicating biocompatibility include, but are not limited to, the presence or absence of an extracellular matrix, the presence or absence of an immune reaction, and the degree of inflammation. Such biocompatibility can be determined by examining the compatibility of a synthetic tissue at an implantation site after implantation (e.g., confirming that an implanted synthetic tissue is not destroyed) (See "Hito Ishoku Zoki Kyozetsu Hanno no Byori Soshiki Shindan Kijyun Kanbetsu Shindan to Seiken Hyohon no Toriatsukai (Zufu) Jinzo Ishoku, Kanzo Ishoku, Suizo Ishoku, Shinzo Ishoku Oyobi Hai Ishoku [Pathological Tissue Diagnosis Criterion for Human Imsplanted Organ Rejection Reaction Handling of Differential Diagnosis and Biopsy Specimen (Illustrated Book) Kidney Implantation, Liver Implantation, Pancreas Implantation, Heart Implantation and Lung Implantation]", The Japan Society for Transplantation and The Japanese Society for Pathology editors, Kanehara Shuppan Kabushiki Kaisha (1998)).

Sufficient affinity of an implantable synthetic tissue varies depending on a part targeted by implantation. However, the degree of affinity can be appropriately determined by those skilled in the art. Examples of parameters for affinity include, but are not limited to, biological integration capability between an implanted synthetic tissue and its implantation site. Such affinity can be determined based on the presence of biological integration at an implantation site after implantation. Preferable affinity herein includes an implanted synthetic tissue disposed to have the same function as that of a site in which the tissue is implanted.

As used herein, the term "self-supporting ability" refers to a property of a tissue (e.g., a synthetic tissue), by which the synthetic tissue is not substantially destroyed when it is restrained on at least one point thereof. Self-supporting ability is herein observed if a tissue (e.g., a synthetic tissue) is picked up by using forceps with a tip having a thickness of 0.5 to 3.0 mm (preferably, tissue is picked up by using forceps with a tip having a thickness of 1 to 2 mm or 1 mm; the forceps preferably have a bent tip) and the tissue is not substantially destroyed. Such forceps are commercially available (e.g., from Natsume Seisakusho). A force exerted for picking up a tissue as used herein is comparable to a force typically exerted by a medical practitioner handing a tissue. Therefore, the self-supporting ability can also be represented by a property, by which the tissue is not destroyed when it is picked up by hand. Examples of such forceps include, but are not limited to, a pair of curved fine forceps (e.g., No. A-11 (tip: 1.0 mm in thickness) and No. A-12-2 (tip: 0.5 mm in thickness) commercially available from Natsume Seisakusho) . A bent tip is more suitable for picking up a synthetic tissue. However, the forceps are not limited to a bent tip type.

For example, when a joint is treated, replacement is mainly performed. The strength of a synthetic tissue of the present invention required in such a case is sufficient at the minimum self-supporting ability described above. Cells contained in the synthetic tissue are subsequently replaced with cells in an affected portion. The replacing cells produce a matrix to enhance the mechanical strength, so that healing progresses. In one embodiment, it is understood that the present invention may be used in conjunction with an artificial joint.

In the present invention, self-supporting ability plays an important role in evaluating the supporting ability of a synthetic tissue when actually produced. When a synthetic tissue of the present invention is produced, a cell sheet is formed in a container. When the sheet is detached from the container, the synthetic tissue of the present invention is understood as applicable to substantially any situation because such a synthetic tissue already has sufficient strength, i.e., has self-supporting ability, to endure being separated from a container in a form of a monolayer sheet prior to without being destroyed. It is understood that the monolayer may partially include a two or three-layer structure. In addition, typically, after a synthetic tissue is produced and detached, the strength and self-supporting ability of the synthetic tissue increases, as is observed in the present invention. Therefore, in the present invention, it is understood that the self-supporting ability evaluated upon production may be an important aspect. Naturally, the strength upon implantation is also important in the present invention. Thus, it may also be important to evaluate the self-supporting ability of a synthetic tissue when a predetermined time has passed after the production of the tissue. Therefore, it is understood that those skilled in the art can determine the timing and strength at the time of transport by back-calculating the time the tissue is to be used based on the above-described relationship.

As used herein, the term "organ" refers to a structure, which is a specific part of an individual organism, where a certain function of the individual organism is locally performed and is morphologically independent. Generally, in multicellular organisms (e.g., animals and plants), organs are made of several tissues in specific spatial arrangement and tissue consists of a number of cells. Examples of such organs include, but are not limited to, skin, blood vessel, cornea, kidney, heart, liver, umbilical cord, intestine, nerve, lung, placenta, pancreas, brain, joint, bone, cartilage, peripheral limbs, and retina. Examples of such organs also include, but are not limited to, organs of the skin system, the parenchyma pancreas system, the pancreatic duct system, the hepatic system, the blood system, the myocardial system, the skeletal muscle system, the osteoblast system, the skeletal myoblast system, the nervous system, the blood vessel endothelial system, the pigment system, the smooth muscle system, the fat system, the bone system, and the cartilage system.

In one embodiment, the present invention targets organs including, but not limited to, an intervertebral disk, a cartilage, a joint, a bone, a meniscus, a synovial membrane, a ligament, and a tendon. In another preferable embodiment, the present invention targets organs including, but is not limited to, bones and cartilages.

As used herein, the term "wrap" in relation to wrapping a composite tissue or the like around a certain part (e.g., an injured site) means that the synthetic tissue or the like of the present invention is arranged so as to cover the part (i.e. , conceal an injury or the like). The terms "wrap" and "arrange so as to cover" the part are used interchangeably. By observing the spatial arrangement between the part and the synthetic tissue, composite tissue comprising the synthetic tissue or the like, it can be determined whether the part is arranged to be covered by the synthetic tissue, composite tissue comprising the synthetic tissue or the like. In a preferable embodiment, in a wrapping step, a synthetic tissue or the like can be wrapped one turn around a certain site.

As used herein, the term "replace" means that a lesion (a site of an organism) is replaced, or cells which have originally been in a lesion are replaced with cells supplied by a synthetic tissue, a complex according to the present invention or the like. Examples of a disease for which replacement is suitable include, but not limited to, a ruptured site. The term "fill" may be used in place of the term "replace" in the present specification.

As used herein, "sufficient time required for biologically integration" between a "synthetic tissue" and a certain "part" varies depending on a combination of the part and the synthetic tissue, but can be appropriately determined by those skilled in the art based on the combination. Examples of such a time include, but are not limited to, 1 week, 2 weeks, 1 month, 2 months, 3 months, 6 months, and 1 year after an operation. In the present invention, a synthetic tissue preferably comprises substantially only cells and materials derived from the cells. Hence, there is no particular material which needs to be extracted after an operation. Therefore, the lower limit of the sufficient time is not particularly important. In addition, it was found in the present invention that such biological integration is significantly shortened in comparison to a case of using somatic MSCs.

As used herein, the term "immune reaction" refers to a reaction due to the dysfunction of immunological tolerance between an implant and a host. Examples of immune reactions include a hyperacute rejection reaction (within several minutes after implantation) (immune reaction caused by antibodies, such as β-Gal), an acute rejection reaction (reaction caused by cellular immunity about 7 to 21 days after implantation), and a chronic rejection reaction (rejection reaction caused by cellular immunity 3 or more months after operation). As used herein, the elicitation of an immune reaction can be confirmed by pathological and histological examination of the type, number, or the like of infiltration of (immunological) cells into an implanted tissue by using staining such as HE staining, immunological staining, or microscopic inspection of tissue sections.

As used herein, the term "calcification" refers to precipitation of calcareous substances in organisms. "Calcification" in *vivo* can be determined herein by Alizarin Red staining and measuring calcium concentration. Quantification is possible by taking out an implanted tissue and dissolving a tissue section by acid treatment or the like to measure the atomic absorption of the solution or the like by a trace element quantifying device.

As used herein, the term "(with) in organism(s)" or "*in vivo*" refers to the inner part of organism(s). In a specific context, "within organism(s)" refers to a position of interest where a subject tissue or organ is to be placed.

As used herein, "*in vitro*" indicates that apart of an organism is extracted or released "outside the organism" (e.g., in a test tube) for various purposes of research. The term in *vitro* is in contrast to the term in *vivo.*

As used herein, the term "*ex vivo*" refers to a series of operations where target cells into which a gene will be introduced are extracted from a subject; a therapeutic gene is introduced *in vitro* into the cells; and the cells are returned into the same subject.

As used herein, the term "material derived from cell(s)" refers to any material originating from the cell(s), including, but not being limited to, materials constituting the cell(s), materials secreted by the cell(s), and materials metabolized by the cell(s). Representative examples of materials derived from cells include, but are not limited to, extracellular matrices, hormones,and cytokines.Materials derived from cells typically have no adverse effect on the cells and their hosts. Therefore, when the material is contained in a three-dimensional synthetic tissue or the like, the material typically has no adverse effect.

As used herein, the term "extracellular matrix" (ECM) refers to a substance existing between somatic cells regardless of whether the cells are epithelial cells or non-epithelial cells. Extracellular matrices are typically produced by cells, and are therefore biological materials. Extracellular matrices are involved in not only supporting a tissue, but also in structuring an internal environment essential for survival of all somatic cells. Extracellular matrices are generally produced from connective tissue cells, but some extracellular matrices are secreted from cells with a basal membrane, such as epithelial cells or endothelial cells. Extracellular matrices are roughly divided into fibrous components and matrices filled there between. Fibrous components include collagen fibers and elastic fibers. A basic component of matrices is a glycosaminoglycan (acidic mucopolysaccharide), most of which is bound to a non-collagenous protein to form a polymer of a proteoglycan (acidic mucopolysaccharide-protein complex). In addition, matrices include glycoproteins, such as laminin of a basal membrane, microfibrils around elastic fibers, fibers, and fibronectins on cell surfaces. Particularly differentiated tissues have the same basic structure. For example, in hyaline cartilage, chondroblasts characteristically produce a large amount of cartilage matrices including proteoglycans. In bones, osteoblasts produce bone matrices which cause calcification. Herein, examples of a typical extracellular matrix include, but not limited to, collagen I, collagen III, collagen V, elast*in vitro*nectin, fibronectin, laminin, thrombospondin, and proteoglycans (for example, decolin, byglican, fibromodulin, lumican, hyaluronic acid, and aggrecan). Various types of extracellular matrix may be utilized in the present invention as long as cell adhesion is achieved.

In one embodiment of the present invention, an extracellular matrix (e.g., elastin, collagen (e.g., Type I, Type III, or Type IV), or laminin) included in a three-dimensional synthetic tissue or the like comprised in the composite tissue of the present invention may be advantageously similar to the composition of an extracellular matrix of a site of an organ for which implantation is intended. In the present invention, extracellular matrices include cell adhesion molecules. As used herein, the terms "cell adhesion molecule" and "adhesion molecule" are used interchangeably to refer to a molecule for mediating the joining of two or more cells (cell adhesion) or adhesion between a substrate and a cell. In general, cell adhesion molecules are divided into two groups: molecules involved in cell-cell adhesion (intercellular adhesion) (cell-cell adhesion molecules) and molecules involved in cell-extracellular matrix adhesion (cell-substrate adhesion) (cell-substrate adhesion molecules). A three-dimensional synthetic tissue of the present invention typically comprises suchacelladhesionmolecule. Therefore, cell adhesion molecules herein include a protein of a substrate and a protein of a cell (e.g., integrin) in cell-substrate adhesion. A molecule other than proteins falls within the concept of cell adhesion molecules herein as long as it can mediate cell adhesion.

A feature of the present invention is in the fact that the synthetic tissue included in the composite tissue of the present invention comprises cells and an (autologous) extracellular matrix produced by the cells themselves. Therefore, the present invention is characterized in having a complex composition with a mixture of collagen I, collagen III, collagen V, elastin vitronectin, fibronectin, laminin, thrombospondin, proteoglycans (for example, decolin, byglican, fibromodulin, lumican, hyaluronic acid, and aggrecan) or the like. Conventionally, a synthetic tissue containing such cell-derived ingredients has not been provided. Practically, it is nearly impossible to obtain a synthetic tissue having such a composition when an artificial material is used. Thus, a composition containing such ingredients (particularly, collagen I and collagen III) is recognized to be a native composition.

More preferably, an extracellular matrix includes each of collagen (Types I, Type III, etc.), vitronectin, and fibronectin. A synthetic tissue containing vitronectin and/or fibronectin in particular has never been provided before. Therefore, the synthetic tissue and the complex according to the present invention are understood to be novel in this regard.

In the present invention, it is understood that somatic MSCs and induced MSCs may have different cell markers, and such different cell markers (also referred to as identification marker herein) are used to distinguish conventional MSCs from MSCs used in the present invention. Examples of such identification markers include, but not limited to, BMP receptors (e.g., BMPR1 (BMPR1A, BMPR1B), and BMPR2).

As used herein, the term "provided" or "distributed" in relation to an extracellular matrix, when discussed in relation to the synthetic tissue of the present invention, indicates that the extracellular matrix is present in the synthetic tissue. It is understood that such provision can be visualized and observed with stain by immunologically staining an extracellular matrix of interest or the like.

As used herein, the term "in a diffused manner" or "diffusedly" in relation to the "distribution" of an extracellular matrix indicates that the extracellular matrix is not localized. Such diffusion of an extracellular matrix refers to diffusion with a ratio of the distribution densities of two arbitrary 1 cm$^2$ sections within a range of typically about 1:10 to about 10:1, and representatively about 1: 3 to about 3:1, and preferably about 1: 2 to about 2:1. More preferably, the ratio is substantially evenly distributed in any section the synthetic tissue. However, the ratio is not limited thereto. When an extracellular matrix is not localized, but is diffused over a surface of the extracellular matrix, the synthetic tissue of the present invention has biological integration capability evenly with respect to the surrounding. Therefore, the synthetic tissue of the present invention achieves an excellent effect of recovery after implantation.

For cell-cell adhesion, cadherin, a number of molecules belonging to an immunoglobulin superfamily (NCAM, L1, ICAM, fasciclin II, III, etc.), selectin, and the like are known, each of which is known to join cell membranes via a specific molecular reaction. Therefore, in one embodiment, the three-dimensional synthetic tissue or the like of the present invention preferably has substantially the same composition of cadherin, immunoglobulin superfamily molecules, or the like as that of a site for which implantation is intended.

In this manner, various molecules having different functions are involved in cell adhesion. Thus, those skilled in the art can appropriately select, depending on the objective, a molecule to be contained in a three-dimensional synthetic tissue used in the present invention. Techniques for cell adhesion other than those described above are also well known, as described in, for example, "Saibogaimatorikkusu -Rinsho heno Oyo-" [Extracellular matrix -Clinical Applications-], Medical Review.

It is possible to determine whether a certain molecule is a cell adhesion molecule by determining that a positive reaction is exhibited in an assay, such as biochemical quantification (an SDS-PAGE method, a labeled-collagen method), immunological quantification (an enzyme antibody method, a fluorescent antibody method, an immunohistological study), a PCR method, or a hybridization method. Examples of such a cell adhesion molecule include, but are not limited to, collagen, integrin, fibronectin, laminin *vitro*nectin, fibrinogen, an immunoglobulin superfamily member (e.g., CD2, CD4, CD8, ICM1, ICAM2, VCAM1), selectin, and cadherin. Most of these cell adhesion molecules transmit into a cell an auxiliary signal for cell activation due to intercellular interaction as well as cell adhesion. Therefore, an adhesion molecule for use in an implant of the present invention preferably transmits such an auxiliary signal for cell activation into a cell. This is because cell activation can promote growth of cells originally present or aggregating in a tissue or organ at an injured site after application of an implant thereto. It can be determined whether such an auxiliary signal can be transmitted into a cell by determining that a positive reaction is exhibited in an assay, such as biochemical quantification (an SDS-PAGE method, a labeled-collagen method), immunological quantification (an enzyme antibody method, a fluorescent antibody method, an immunohistological study), a PCR method, or a hybridization method.

An example of a cell adhesion molecule includes cadherin, which is widely known in cell systems capable of being fixed to a tissue. Cadherin can be used in a preferable embodiment of the present invention.

Non-fixed cells need to adhere to a specific tissue in order to act on the tissue. In this case, it is believed that cell-cell adhesion is gradually enhanced via a first adhesion by a selectin molecule or the like which is constantly expressed and a second adhesion by a subsequently activated integrin molecule. Therefore, as cell adhesion molecules used in the present invention, it is possible to use an agent for mediating the first adhesion and another agent for mediating the second adhesion, or both.

As used herein, the term "actin regulatory agent" refers to a substance with a function of interacting directly or indirectly with actin in cells to change the form or state of the actin. It is understood that actin regulatory agents are categorized into two classes, actin depolymerizing agents and actin polymerizing agents, depending on the action on actin. Examples of actin depolymerizing agents include, but are not limited to, Slingshot, cofilin, CAP (cyclase associated protein), AIP1 (actin-interacting-protein 1), ADF (actin depolymerizing factor), destrin, depactin, actophorin, cytochalasin, and NGF (nerve growth factor). Examples of actin polymerizing agents include, but are not limited to, RhoA, mDi, profilin, Rac1, IRSp 53, WAVE2, ROCK, LIM kinase, cofilin, cdc42, N-WASP, Arp2/3, Drf3, Mena, LPA (lysophosphatidic acid), insulin, PDGFa, PDGFb, chemokine, and TGF-$\beta$. The above-described actin regulatory agents include substances which can be identified by the following assay. Interaction of an actin regulatory agent with respect to actin is assayed herein as follows. Actin is made visible with an actin staining reagent (Molecular Probes, Texas Red-X phalloidin) or the like. By observing actin aggregation or cell outgrowth under a microscope, the presence of the interaction is determined by confirming the aggregation and reconstruction of actin and/or an increase in the cell outgrowth rate. The determination may be performed quantitatively or qualitatively. The above-described actin regulatory agents are used in the present invention so as to promote the detachment or a multilayer structure of the synthetic tissue. An actin regulatory agent used in the present invention may be derived from any organism, including mammalian species such as a human, mouse, or bovine. The above-described agents involved in actin polymerization control actin polymerization in relation to Rho and examples of the agents include the following (see, for example, "Saibokokkaku/Undo ga wakaru (Understanding of cytoskeleton/movement)", (Ed./Hiroaki Miki), Yodo-sha).

Actin polymerization (see Takenaka T et al. J.Cell Sci., 114: 1801-1809, 2001) RhoA → mDi → profilin ⇒ actin polymerization RhoA → ROCK/Rho → LIM kinase → phosphorylation of cofilin (suppression) ⇒ actin polymerization Rac1 → IRSp53 → WAVE2 → profilin, Arp2/3 ⇒ actin polymerization cdc42 → N-WASP → profilin, Arp2/3 ⇒ actin polymerization cdc42 → Drf3 → IRSp53 → Mena ⇒ actin polymerization (In the above description, → indicates a signal transduction pathway of phosphorylation or the like.

In the present invention, any agent involved in such a pathway can be utilized.

Actin depolymerization Slingshot → dephosphorization of cofilin (activation) ⇒ actin depolymerization Actin depolymerization is controlled by the balance between phosphorylation by LIMkinase activity of actin depolymerization cofilin and dephosphorization by Slingshot. As another agent for activating cofilin, CAP (cyclase-associated protein) and AIPI (actin-interacting-protein 1) are identified. It is understood that any suitable agent can be used.

LPA (lysophosphatidic acid) of any chain length can be used.

Any chemokine can be used. However, examples of preferable chemokine include interleukin 8, MIP-1, and SDF-1.

Any TGF-$\beta$ can be used. However, examples of preferable TGF-$\beta$ include TGF-$\beta$1 and TGF-$\beta$3. TGF-$\beta$1 and TGF-$\beta$3 have an extracellular matrix generation promoting activity. Thus, it is noted that TGF-$\beta$1 and TGF-$\beta$3 can be

used in the present invention.

As used herein, the term "tissue strength" refers to a parameter which indicates a function of a tissue or organ and a physical strength of the tissue or organ. Tissue strength can be generally determined by measuring tensile strength (e.g., break strength, modulus of rigidity, and Young's modulus). Such a general tensile test is well known. By analyzing data obtained from a general tensile test, various data, such as break strength, modulus of rigidity, and Young's modulus, can be obtained. These values can be used herein as indicators of tissue strength. Typically, tissue strength which allows clinical applications is herein required.

Herein, the tensile strength of a three-dimensional synthetic tissue or the like that is used in the present invention can be determined by measuring the stress and distortion characteristics thereof. Briefly, a load is applied to a sample; the resultant distortion and the load are input into respective A/D converters (e.g., ELK-5000) (e.g., 1 ch: distortion, 2 ch: load); and the stress and distortion characteristics are measured to determine the tensile strength. Tensile strength can also be determined by testing creep characteristics. A creep characteristics indentation test is a test to investigate how a sample extends over time while a constant load is applied to the sample. For small materials, thin materials, and the like, an indentation test is conducted using, for example, a tetrahedronal indenter with a tip having a radius of about 0.1 $\mu$m to about 1 $\mu$m. Initially, the indenter is pushed into a test piece to apply a load. When the indenter reaches several tens of nanometers to several micrometers in depth into the test piece, the indenter is withdrawn to remove the load. Rigidity, Young's modulus, or the like can be obtained based on the behavior of the load and the push depth derived from the curve. The tensile strength of the synthetic tissue of the present invention may be low. The tensile strength increases when the extracellular matrix in the cell to extracellular matrix ratio is increased, and decreases when the cell to extracellular matrix ratio is increased. The present invention is also characterized in that the strength can be freely adjusted as necessary. The present invention is characterized in that the strength can be high or low relative to that of a tissue to be implanted. Therefore, it is recognized that the strength can be set to comply with any desired site.

As used herein, the term "physiologically active substance" refers to a substance capable of acting on a cell or tissue. Physiologically active substances include cytokines and growth factors. A physiologically active substance may be naturally-occurring or synthesized. Preferably, a physiologically active substance is one that is produced by a cell or one that has a function similar thereto. As used herein, a physiologically active substance may be in the form of a protein or a nucleic acid or in other forms. In actual practice, cytokines typically refer to proteins. In the present invention, a physiologically active substance may be used to promote the affinity of an implanted synthetic tissue of the present invention.

As used herein, the term "cytokine" is defined in the broadest sense in the art and refers to a physiologically active substance which is produced from a cell and acts on the same or different cell. Cytokines are generally proteins or polypeptides having a function of controlling an immune response, regulating the endocrine system, regulating the nervous system, acting against a tumor, acting against a virus, regulating cell growth, regulating cell differentiation, or the like. Cytokines are in the form of a protein or a nucleic acid or in other forms herein. In actual practice, cytokines typically refer to proteins.

The terms "growth factor" or "cell growth factor" are used herein interchangeably and each refers to a substance which promotes or controls cell growth. Growth factors are also called proliferation factors or development factors. Growth factors may be added to cell or tissue culture medium to replace the action of serum macromolecules. It has been revealed that a number of growth factors have a function of controlling differentiation in addition to a function of promoting cell growth.

Examples of representative cytokines include interleukins, chemokines, hematopoietic factors such as colony stimulating factors, a tumor necrosis factor, and interferons, and a platelet-derived growth factor (PDGFa, PDGFb), an epidermal growth factor (EGF), a fibroblast growth factor (FGF), a hepatocyte growth factor (HGF), and a vascular endothelial cell growth factor (VEGF) as growth factors having proliferative activity.

Physiologically active substances, such as cytokines and growth factors, typically have redundancy in function. Accordingly, cytokines or growth factors that are known by another name or function can be used in the present invention as long as they have the activity of a physiologically active substance for use in the present invention. Cytokines or growth factors can be used in a therapeutic method or pharmaceutical agent according to a preferred embodiment of the present invention as long as they have preferable activity as described herein.

Therefore, in one embodiment of the present invention, it was revealed that when such a cytokine or growth factor (e.g., BMP-2) is provided to an implantation site (e.g., an injured site of a cartilage) concomitantly with a synthetic tissue or three-dimensional structure of the present invention, the affinity of the synthetic tissue or three-dimensional structure and an improvement in the function of the implantation site are observed. Thus, the present invention provides such a combined therapy.

As used herein, the term "differentiation" refers to a developmental process of the state of parts of organisms, such as cells, tissues, or organs and a process in which a characteristic tissue or organ is formed. The term "differentiation" is mainly used in embryology, developmental biology, and the like. In organisms, various tissues and organs are formed from divisions of a fertilized ovum (a single cell) to be an adult. At early developmental stages (i.e., before cell division or after insufficient cell division), each cell or cell group has no morphological or functional feature and is thus indistinguishable. Such a state is referred to as "undifferentiated". "Differentiation" may occur at the level of organs. A cell constituting an organ develops into various cells or cell groups having different characteristics. This phenomenon is also referred to as differentiation within an organ in the formation of the organ. Therefore, a three-dimensional synthetic tissue that is used in the present invention may use a tissue including differentiated cells. In one embodiment, when differentiation is required to produce a three-dimensional synthetic tissue or a composite tissue of the present invention, the differentiation may be allowed to occur either before or after the organization of the cells.

As used herein, the terms "differentiation agent" and "differentiation promoting agent" are used interchangeably and refer to any agent which is known to promote differentiation into differentiated cells (e.g., chemical substances or temperature). Examples of such an agent include, but are not limited to, various environmental factors, such as temperature, humidity, pH, salt concentration, nutrients, metals, gas, organic solvent, pressure, chemical substances (e.g., steroids and antibiotics), and any combinations thereof. Representative examples of differentiation agents include, but are not limited to, cellular physiologically active substances. Representative examples of such cellular physiologically active substances include, but are not limited to, DNA demethylating agents (e.g., 5-azacytidine), histone deacetylating agents (e.g., trichostatin), intranuclear receptor ligands (e.g., retinoic acid (ATRA), vitamin $D_3$, and T3), cell growth factors (e.g., activin, IGF-1, FGF, PDGFa, PDGFb, TGF-$\beta$, and BMP2/4), cytokines (e.g., LIF, IL-2, and IL-6), hexamethylenebisacetoamides, dimethylacetoamides, dibutyl cAMPs, dimethylsulfoxides, iododeoxyuridines, hydroxyl ureas, cytosine arabinosides, mitomycin C, sodium lactate, aphydicolin, fluorodeoxyuridine, polybren and selenium.

Specific examples of differentiation agents are described below. These differentiation agents may be used alone or in combination: 1) Synovial cell: FGF, TGF-$\beta$ (particularly, TGF-$\beta$1, TGF-$\beta$3); 2) Osteoblast: BMP (particularly, BMP-2, BMP-4, BMP-7), FGF; 3) Chondroblast: FGF, TGF-$\beta$ (particularly, TGF-$\beta$1, TGF-$\beta$3), BMP (particularly, Bump-2, BMP-4, BMP-7), TNF-$\alpha$, IGF; 4) Adipocyte: insulin, IGF, LIF; and 5) Muscle cell: LIF, TNF-$\alpha$, FGF.

Such agents can be used upon examining differentiation capability.

As used herein, the term "osteogenesis" refers to making differentiate into an osteocyte. It is known that osteogenesis is promoted in the presence of dexamethasone, $\beta$-glycerophosphate, and ascorbic acid 2-phosphate. A bone morphogenetic factor (BMP, (particularly, BMP-2, BMP-4, BMP-7)) may be added to promote osteogenesis.

As used herein, the term "chondrogenesis" refers to making any cell differentiate into a chondrocyte. It is known that chondrogenesis is promoted in the presence of pyrubic acid, dexamethasone, ascorbic acid 2-phosphate, insulin, transferrin, and selenite. A bone morphogenetic factor (BMP, (particularly, BMP-2, BMP-4, BMP-7)), TGF-$\beta$ (particularly, TGF-$\beta$1 and TGF-$\beta$3), FGF, TNF-$\alpha$ or the like may be added to promote chondrogenesis.

As used herein, the term "adipogenesis" refers to making any cell differentiate into an adipocyte. It is known that adipogenesis is promoted in the presence of insulin, IGF, LIF, or ascorbic acid 2-phosphate.

As used herein, the terms "implant", "transplant", "graft", and "tissue graft" are used interchangeably, referring to a homologous or heterologous tissue or a cell group which is inserted into a particular site of a body and thereafter forms a part of the body after insertion. Therefore, a three-dimensional synthetic tissue that is used in the present invention can be used as an implant. Examples of grafts include, but are not limited to, organs or portions of organs, dura mater, joint capsule, bone, cartilage, cornea, and tooth. Therefore, grafts encompass anything that is inserted into a defective part so as to compensate for the lost portion. Grafts include, but arenotlimitedto, autografts, allografts, andxenografts, which depend on the type of their donor.

As used herein, the term "autograft" (a tissue, a cell, an organ, etc.) refers to a graft (a tissue, a cell, an organ, etc.) which is implanted into the same individual from which the graft is derived. As used herein, the term "autograft" (a tissue, a cell, an organ, etc.) may encompass a graft (a tissue, a cell, an organ, etc.) from another genetically identical individual (e.g. an identical twin) in a broad sense. As used herein, the expressions "autologous" and "derived from a subject" are used interchangeably. Therefore, the expression "not derived from a subject" is synonymous to the graft not being autologous (i.e., heterologous).

As used herein, the term "allograft (a tissue, a cell, an organ, etc.)" refers to a graft (a tissue, a cell, an organ, etc.) which is implanted from a donor genetically different from, though of the same species as , the recipient. Since an allograft is genetically different from the recipient, the allograft (a tissue, a cell, an organ, etc.) may elicit an immune reaction in the recipient of implantation. Examples of such grafts (a tissue, a cell, an organ, etc.) include, but are not limited to, grafts derived from parents (a tissue, a cell, an organ, etc.). The synthetic tissue of the present invention can be used as an allograft, which is noteworthy in terms of being demonstrated to have satisfactory therapeutic results.

As used herein, the term "xenograft" (a tissue, a cell, an organ, etc.) refers to a graft (a tissue, a cell, an organ, etc.) which is implanted from a different species. Therefore, for example, when a human is a recipient, a porcine-derived graft (a tissue, a cell, an organ, etc.) is called a xenograft (a tissue, a cell, an organ, etc.).

As used herein, the term "recipient" (acceptor) refers to an individual who receives a graft (a tissue, a cell, an organ, etc.) or implanted matter (a tissue, a cell, an organ, etc.) and is also called "host". In contrast, an individual providing a graft (a tissue, a cell, an organ, etc.) or implanted matter (a tissue, a cell, an organ, etc.) is called "donor" (provider).

When making a composite tissue of the present invention, a synthetic tissue derived from any cell can be used. This is because a synthetic tissue (e.g. , membranous tissues or organs) that is used in a composite tissue formed by the method of the present invention can exhibit a desired function while the tissue injury rate is maintained at a level which does not interfere with the therapy of interest (i.e., a low level). Conventionally, tissues or organs could only be directly used as grafts. Under such a circumstance, the present invention was able to form a tissue that is three-dimensionally integrated from cells to enable use of such a synthetic three-dimensional tissue, leading to a significant improvement in therapeutic results over prior art techniques can be considered one of the significant effects of the present invention which could not be achieved by conventional techniques.

As used herein, the term "subject" refers to an organism to which treatment of the present invention is applied and is also referred to as "patient". A patient or subject may be preferably a human.

Cells contained in a composite tissue of the present invention may be cells of a syngeneic origin (self origin), an allogenic origin (non-self origin), or a heterologous origin. Considering the possibility of rejection reactions, syngeneic cells are preferable. If rejection reactions do not raise problems, allogenic cells may be used. Cells which elicit rejection reactions can also be employed by optionally treating the cells in a manner that overcomes rejection reactions. Procedures for avoiding rejection reactions are known in the art, as described in, for example, "Shin Gekagaku Taikei, Dai 12 Kan, Zoki Ishoku (Shinzo Ishoku · Hai Ishoku Gijutsuteki, Rinriteki Seibi kara Jisshi ni Mukete [New Whole Surgery, Vol. 12, Organ Implantation (Heart Implantation · Lung Implantation From Technical and Ethical Improvements to Practice)]" (Revised 3rd ed.), Nakayama Shoten. Examples of such methods include a method using immunosuppressants or steroidal drugs. For example, there are currently the following immunosuppressants for preventing rejection reactions: "cyclosporine" (SANDIMMUNE/NEORAL); "tacrolimus" (PROGRAF); "azathioprine" (IMURAN); "steroid hormone" (prednine, methylprednine); and "T-cell antibodies" (OKT3, ATG, etc.). A method which is used worldwide as a preventive immunosuppression therapy in many facilities is the concurrent use of three drugs "cyclosporine, azathioprine, and steroid hormone". An immunosuppressant is desirably administered concurrently with a pharmaceutical agent of the present invention, but the present invention is not limited to this. An immunosuppressant may be administered before or after a regenerative/therapeutic method of the present invention as long as an immunosuppression effect can be achieved.

Examples of a condition of a target subject of the present invention include, but are not limited to, dura mater implant at the time of brain surgery, a joint injury or denaturation; a chondral injury or denaturation; osteonecrosis; meniscus injury or denaturation; intervertebral disk denaturation; ligament injury or denaturation; a fracture; and implantation of a joint, cartilage, or bone to a patient having a bone defect.

Tissues targeted by the present invention may be any mesenchymal organ of an organism, especially organs comprising an adipose tissue, bone tissue and/or cartilage tissue. Tissues and organs targeted by the present invention may be derived from any animal. Examples of organisms targeted by the present invention include vertebrates. Preferably, organisms targeted by the present invention are mammals (e.g., monotremata, marsupialia, edentate, dermoptera, chiroptera, carnivore, insectivore, proboscidea, perissodactyla, artiodactyla, tubulidentata, pholidota, sirenia, cetacean, primates, rodentia, lagomorpha, or the like). Illustrative examples of a subject include, but are not limited to, animals, such as cattle, pigs, horses, chickens, cats, dogs, and the like. More preferably, organisms targeted by the present invention are primates. Most preferably, organisms targeted by the present invention are humans. This is because there is limitation to implantation therapies and therapy is desired. Further, this is because it is understood by those skilled in the art that the therapy is applicable to humans from the results demonstrated in the Examples herein.

As used herein, the term "flexibility" in relation to a synthetic tissue refers to an ability to resist physical stimuli from external environments (e.g., pressure) or the like. A synthetic tissue having flexibility is preferable when the implantation site moves or deforms autonomously or due to external effects.

As used herein, the term "extendibility and contractibility" in relation to a synthetic tissue refers to a property of having an ability to resist extending or contracting stimuli from external environments (e.g., pulsation). A synthetic tissue having extendibility and contractibility is preferable when the implantation site is subjected to extending or contracting stimuli. Examples of implantation sites, which are subjected to extending or contracting stimuli, include, but are not limited to, muscle, joint, cartilage, tendon, and the like.

As used herein, the term "part" or "portion" refers to any part or portion, tissue, cell, or organ in the body. Examples of such parts, tissues, cells, and organs include, but are not limited to, a portion which can be treated with skeletal myoblasts, fibroblasts, synovial cells, or stem cells. A marker specific to a portion may be any parameter, such as a nucleic acid molecule (expression of mRNA), a protein, an extracellular matrix, a specific phenotype, or a shape of a cell. Therefore, any marker which is not specified herein may be used to identify a synthetic tissue of the present invention as long as the marker can indicate that cells are considered equivalent to being derived from said portion. Representative examples of such portions include, but are not limited to, portions containing somatic or induced mesenchymal stem cells or cells derived there from, other tissues, organs, myoblasts (e.g., skeletal myoblasts), fibroblasts, and synovial cells.

The following markers can be used as an index to observe a cartilage tissue or the like.

Sox9 (human: Accession No. NM_000346) is a marker specific to chondrocytes. The marker can be confirmed mainly by observing the presence of mRNA (Kulyk WM, Franklin JL, Hoffman LM. Sox9 expression during chondrogenesis in micromass cultures of embryonic limb mesenchyme. Exp Cell Res. 2000 Mar 15, 255(2):327-32.).

Col 1A1 (human Accession No. NM_000017) is a marker specific to osteocytes, which decreases in cartilage. The marker can be confirmed mainly by observing the presence of mRNA (Swartz MF, et al. J Am Coll Cardiol, 2012 Oct 30. PMID23040566.).

Col 2A1 (human: Accession No. NM_001844) isamarkerspecific to chondrocytes. The marker can be confirmed mainly by observing the presence of mRNA (Kulyk WM, Franklin JL, Hoffman LM. Sox9 expression during chondrogenesis in micromass cultures of embryonic limb mesenchyme. Exp Cell Res. 2000 Mar 15; 255(2):327-32.).

Aggrecan (human: Accession No. NM_001135) is a marker specific to chondrocytes. The marker can be confirmed mainly by observing the presence of mRNA (Kulyk WM, Franklin JL, Hoffman LM. Sox9 expression during chondrogenesis in micromass cultures of embryonic limb mesenchyme. Exp Cell Res. 2000 Mar 15; 255(2):327-32.).

Bone sialoprotein (human: Accession No. NM_004967) is a marker specific to osteoblasts. The marker can be confirmed mainly by observing the presence of mRNA (Haase HR, Ivanovski S, Waters MJ, Bartold PM. Growth hormone regulates osteogenic marker mRNA expression in human periodontal fibroblasts and alveolar bone-derived cells. J Periodontal Res. 2003 Aug; 38(4):366-74.).

Osteocalcin (human: Accession No. NM_199173) is a marker specific to osteoblasts. The marker can be confirmed mainly by observing the presence of mRNA (Haase HR, Ivanovski S, Waters MJ, Bartold PM. Growth hormone regulates osteogenic marker mRNA expression in human periodontal fibroblasts and alveolar bone-derived cells. J Periodontal Res. 2003 Aug; 38(4): 366-74.).

GDF5 (human: Accession No. NM_000557) is a marker specific to ligament cells. The marker can be confirmedmainly by observing the presence of mRNA (Wolfman NM, Hattersley G, Cox K, Celeste AJ, Nelson R, Yamaji N, Dube JL, DiBlasio-Smith E, Nove J, Song JJ, Wozney JM, Rosen V. Ectopic induction of tendon and ligament in rats by growth and differentiation factors 5, 6, and 7, members of the TGF-beta gene family. J Clin Invest. 1997 Jul 15; 100(2):321-30.).

Six1 (human: Accession No. NM_005982) is a marker specific to ligament cells (Dreyer SD, Naruse T, Morello R, Zabel B, Winterpacht A, Johnson RL, Lee B, Oberg KC. Lmx1b expression during joint and tendon formation: localization and evaluation of potential downstream targets. Gene Expr Patterns. 2004 Jul; 4(4): 397-405.). The marker can be confirmed mainly by observing the presence of mRNA.

Scleraxis (human: Accession No. BK000280) is a marker specific to ligament cells (Brent AE, Schweitzer R, Tabin CJ. A somitic compartment of tendon progenitors. Cell. 2003 Apr 18; 113(2): 235-48.). The marker can be confirmed mainly by observing the presence of mRNA.

CD56 (human: Accession No. U63041) is a marker specific to myoblasts. The marker can be confirmed mainly by observing the presence of mRNA.

MyoD (human: Accession No. X56677) is a marker specific to myoblasts. The marker can be confirmed mainly by observing the presence of mRNA.

Myf5 (human: Accession No. NM_005593) is a marker specific to myoblasts. The marker can be confirmed mainly by observing the presence of mRNA.

Myogenin (human: Accession No. BT007233) is a marker specific to myoblasts. The marker can be confirmed mainly by observing the presence of mRNA.

In other embodiments, other markers specific to other tissues can be utilized. Examples of such markers include: Oct-3/4, SSEA-1, Rex-1, and Otx2 for embryonic stem cells; VE-cadherin, Flk-1, Tie-1, PECAM1, vWF, c-kit, CD34, Thy1, and Sca-1 for endothelial cells; skeletal muscle α actin in addition to the above-described markers for skeletal muscles; Nestin, Glu receptor, NMDA receptor, GFAP, and neuregulin-1 for nerve cells; and c-kit, CD34, Thy1, Sca-1, GATA-1, GATA-2, and FOG for hematopoietic cell lines.

Examples of characteristic markers of an induced three-dimensional synthetic tissue (MSC-TEC) of the present invention include BMP receptor markers (e.g., BMPR1A and BMPR2).

BMPR1A is a type 1A bone morphogenetic protein receptor. BMPR1A is a marker represented by human: OMIM: 601299, Accession No. BC028383. The marker can be confirmed mainly by observing the presence of mRNA.

BMPR2 is a type 2 bone morphogenetic protein receptor. BMPR2 is a marker represented by human: OMIM: 600799, Accession No. Z48923. The marker is known as a receptor of a protein inducing extracellular osteogenesis. Type II receptors bind to ligands in the absence of a type I receptor. The marker can be confirmed mainly by observing the presence of mRNA.

As used herein, the term "derived" in relation to cells means that the cells are separated, isolated, or extracted from a cell mass, tissue, or organ in which the cells have been originally present, or that the cells are induced from stem cells.

As used herein, the term "three-dimensional(ly organized) synthetic tissue" refers to a synthetic tissue comprised in a composite tissue of the present invention, which is substantially made of a cell and an extracellular matrix from the cell. Such three-dimensional synthetic tissues typically constitute a three-dimensional structure. As used herein, the term "three-dimensional(ly organized) structure" refers to an object extending three-dimensionally, wherein the object comprises cells having intracellular integration or alignment and wherein matrices are oriented three-dimensionally and cells are arranged three-dimensionally. The extracellular matrix contains fibronectin, collagen I, collagen III, and vitronectin, and the extracellular matrix is diffusedly distributed in the tissue. The extracellular matrix are integrated (biologically integrated or biologically united) with the cell to form a three-dimensional structure together, have an ability to integrate (biologically integrate or biologically united) with the surroundings when implanted, and have sufficient strength to provide a self-supporting ability.

As used herein, the term "artificial bone" refers to a medical device made of artificial material for filling a defective portion of a bone. Artificial bones are typically made of a material with high affinity to a human body. Preferably, an artificial bone uses components of actual bone. Such a material is typically made of a material selected from the group consisting of materials with high affinity to a human body such as ceramics such as hydroxyapatite (HA) and $\alpha$-tricalcium phosphate or $\beta$-tricalcium phosphate, bioglass (silicon), bioceramics such ascarbon/alumina/zirconia,metalssuch as titanium or tungsten, and coral materials.

As used herein, the term "composite tissue" refers to a tissue obtained by combining a three-dimensional synthetic tissue with another synthetic tissue such as an artificial bone. As used herein, the term "composite tissue" maybe called "hybrid graft" , which is used to have the same meaning as "composite tissue". Thus, such a composite tissue can be used in the treatment of a plurality of tissues (bone/cartilage or the like) . For example, such a composite tissue can be used in treating both a cartilage and a bone. In the composite tissue of the present invention, an implantable (three-dimensional) synthetic tissue biologically integrates with another synthetic tissue. Such integration can be achieved by allowing contact between, and optionally culturing, two tissues. Such biological integration is mediated by an extracellular matrix. A three-dimensional synthetic tissue refers to an object extending three-dimensionally, wherein the object comprises cells having intracellular integration and alignment and wherein matrices are oriented three-dimensionally and cells are arranged three-dimensionally.

As used herein, the term "biological union" or "biological integration" in relation to the relationship between biological entities means that there is certain biological interaction between the two biological entities (it is understood that integration and union can be interchangeably used). For body tissues such as bones and cartilages, biological union or biological integration is referred to as "integration", which is used herein in the same meaning. Examples of such interaction include, but are not limited to, interaction via biological molecules (e.g., extracellular matrix), interaction via signal transduction, and electrical interaction (electrical integration, such as synchronization of electrical signals). Biological integration includes biological integration in a synthetic tissue and biological integration of a synthetic tissue with its surroundings (e.g., surrounding tissues and cells after implantation). In order to confirm interactions, an assay appropriate for a characteristic of the interaction is employed. In order to confirm physical interactions via biological molecules, the strength of a three-dimensional synthetic tissue or the like is measured (e.g., a tensile test). In order to confirm interaction via signal transduction, gene expression or the like is investigated for the presence of signal transduction. In order to confirm electrical interactions, the electric potential of a three-dimensional synthetic tissue or the like is measured to determine whether the electric potential is propagated with constant waves. In the present invention, biological integration is typically provided in all three dimensional directions. Preferably, it is advantageous to have substantially uniform biological integration in all directions in a three-dimensional space. However, in another embodiment, it is possible to use a three-dimensional synthetic tissue or the like, which has substantially uniform two-dimensional biological integration with slightly weaker biological integration in three-dimensional directions. Alternatively, biological integration via an extracellular matrix can be confirmed based on the degree of staining by staining the extracellular matrix. An integration experiment using a cartilage is a method for observing biological integration *in vivo*. In this experiment, a surface of the cartilage is removed and digested with chondroitinase ABC (Hunziker E.B. et al., J. Bone Joint Surg. Am., 1996 May; 78(5): 721-33). Thereafter, a tissue of interest is implanted onto the cut surface, followed by culturing the tissue for about 7 days. The subsequent integration is histologically observed. It is understood that a capability to adhere to surrounding cells and/or extracellular matrix can be determined by such an experiment using a cartilage.

A three-dimensional synthetic tissue, a composite tissue comprising the same or the like of the present invention may be provided using known preparation methods as a pharmaceutical product or as medical instrument, an animal drug, a quasi-drug, a marine drug, a cosmetic product or the like.

The present invention, when used as a pharmaceutical agent, may further comprise a pharmaceutically acceptable carrier or the like. Examples of a pharmaceutically acceptable carrier contained in a pharmaceutical agent of the present invention include any material known in the art.

Examples of such a suitable formulation material or pharmaceutically acceptable carrier include, but are not limited to, antioxidants, preservatives, colorants, flavoring agents, diluents, emulsifiers, suspending agents, solvents, fillers, bulking agents, buffers, delivery vehicles, diluents, excipients and/or pharmaceutical adjuvants.

The amount of a pharmaceutical agent (e.g., a synthetic tissue, a composite tissue, a pharmaceutical compound used in conjunction therewith, etc.) used in the treatment method of the present invention can be readily determined by those skilled in the art while considering the purpose of use, target disease (type, severity, and the like), patient's age, weight, sex, case history, form or type of the tissue, or the like. The frequency of the treatment method of the present invention applied to a subject (or patient) can also be readily determined by those skilled in the art while considering the purpose of use, target disease (type, severity, and the like), patient's age, weight, sex, case history, progression of the therapy, or the like. The frequency of treatment may be once, as many cases are healed after one treatment. Needless to say, treatment of twice or more is also contemplated while considering the results.

As used herein, the term "administer", in relation to a composite tissue or the like of the present invention or a pharmaceutical agent comprising the same, means that it is administered alone or in combination with another therapeutic agent. A synthetic tissue, composite tissue or the like of the present invention may be introduced into therapy sites (e.g., osteochondral defect) by the following methods , in the following forms, and in the following amounts. Specifically, administration methods of a synthetic tissue, composite tissue or the like of the present invention include direct insertion into an impaired site of osteoarthritis, or the like. Combinations may be administered, for example, either concomitantly as an admixture, separately but simultaneously or concurrently; or sequentially. This includes presentations in which the combined agents are administered together as a therapeutic mixture, and also procedures in which the combined agents are administered separately but simultaneously (e.g., a synthetic tissue, composite tissue or the like is directly provided by operation, while other pharmaceutical agents are provided by intravenous injection). "Combination" administration further includes separate administration of one of the compounds or agents given first, followed by the second.

As used herein, the term "reinforcement" means that the function of a targeted part of an organism is improved.

As used herein, the term "instructions" refers to an article describing how to handle a synthetic tissue, composite tissue, reagents and the like, usage, a preparation method, a method of creating a synthetic tissue, a contraction method, a method of administering a pharmaceutical agent of the present invention or the like, a method for diagnosis, or the like for persons conducting the administration such as a physician or a patient or persons who providing the diagnosis (e.g., may be the patients) . The instructions have descriptions for instructing the procedure for administering a diagnostic agent, pharmaceutical agent or the like of the present invention. The instructions are prepared in accordance with a format defined by an authority of the country in which the present invention is practiced (e.g. , Health, Labor and Welfare Ministry in Japan or Food and Drug Administration (FDA) in the U.S.), with an explicit description that the instructions are approved by the authority. The instructions are so-called package insert and are typically provided in paper media, but are not limited thereto. The instructions may also be provided in a form such as electronic media (e.g., web sites or emails provided on the Internet).

As used herein, the term "extracellular matrix synthesis promoting agent" refers to any agent which promotes the production of an extracellular matrix of a cell. In the present invention, when an extracellular matrix synthesis promoting agent is added to a cell sheet, an environment which promotes detachment of the cell sheet from a culture container is provided and such a sheet biologically integrates in three-dimensional directions, where biological integration includes integration of extracellular matrix and cells in a tissue and integration of extracellular matrix with each other. Here, three-dimensional organization is further promoted by self-contraction. Representative examples of such an agent include agents that promote the secretion of an extracellular matrix (e.g. , TGF-$\beta$1 and TGF-$\beta$3). Representative examples of an extracellular matrix synthesis promoting agent include TGF-$\beta$1, TGF-$\beta$3, ascorbic acid, ascorbic acid 2-phosphate, and a derivative and salt thereof. Preferably, such an extracellular matrix synthesis promoting agent may be a component of an extracellular matrix of a part targeted by application and/or a component(s) capable of promoting the secretion of an extracellular matrix in an amount similar thereto. When such an extracellular matrix synthesis promoting agent comprises a plurality of components, such components may be components of an extracellular matrix of a part targeted by application and/or components in an amount similar thereto.

As used herein, the term "ascorbic acid or a derivative thereof" includes ascorbic acid and analogs thereof (e.g., ascorbic acid 2-phosphate), and salts thereof (e.g., sodium salt, magnesium salt , and potassium salt). Ascorbic acid is preferably, but is not limited to, an L-isomer.

(Description of the Preferred Embodiments)

Hereinafter, preferred embodiments of the present invention will be described. It is understood that the following embodiments are provided for a better understanding of the present invention and the scope of the present invention should not be limited to the following description. It will be clearly appreciated by those skilled in the art that variations and modifications can be appropriately made without departing from the scope of the present invention with reference to the descriptions of the specification.

(Induced three-dimensional synthetic tissue)

In one aspect, the present invention provides an induced three-dimensional synthetic tissue. Such an induced three-dimensional synthetic tissue may be used for treating or preventing a disease, disorder, or condition associated with an osteochondral defect. The induced three-dimensional synthetic tissue of the present invention is expressed as an implantable synthetic tissue substantially made of a mesenchymal stem cell induced from a pluripotent stem cell and an extracellular matrix derived from said cell.

In one Embodiment, cells in the synthetic tissue of the present invention express at least one receptor selected from the group consisting of bone morphogenetic protein receptor 1A (BMPR1A) and bone morphogenetic protein receptor 2 (BMPR2) more than a somatic mesenchymal stem cell obtained from within the body. The induced three-dimensional synthetic tissue of the present invention has been found to unexpectedly express many such markers. Though not wishing to be bound by any theory, the induced three-dimensional synthetic tissue of the present invention is characterized in that the cartilage differentiation capability is enhanced, as is typified by elevated expression of these markers. Such a characteristic of induced three-dimensional synthetic tissues was not obtained with a conventional three-dimensional synthetic tissue produced from a somatic cell.

In a preferred embodiment, the synthetic tissue of the present invention has the capability to differentiating into hyaline cartilage-like cartilage. Hyaline cartilage is the most commonly-seen cartilage encompassing articular cartilage covering the facies articularis, tracheal cartilage surrounding the trachea so that the trachea does not collapse, thyroid cartilage and the like. Hyaline cartilage is homogenous, amorphous and semi-transparent structure. Further, hyaline cartilage forms a rough shape of a bone which is substituted with a bone in cartilaginous ossification. Thus, this is considered to serve an important role in healing in an osteochondral defect. Thus, although not wishing to be bound by any theory, the synthetic tissue of the present invention is considered to be capable of exerting an improved therapeutic effect by exhibiting an ability to differentiate into hyaline-cartilage-like cartilage.

In one embodiment, the three-dimensional synthetic tissue used in the present invention generally is made substantially made of induced mesenchymal stem cell and an extracellular matrix from said cell. A three-dimensional synthetic tissue used in the present invention is substantially made of an induced mesenchymal stem cell or a substance derived from the cell. The synthetic tissue is substantially made of only an induced mesenchymal stem cell and a cell-derived material (e.g., extracellular matrix), so that the synthetic tissue can have an increased level of biocompatibility and affinity. As used herein, the term "substantially made of ..." should be understood to be defined such that cells and materials derived from the cells are included, as well as any other material as long as it does not have any harmful effect (herein, mainly, adverse effect on implantation). Such materials which do not have any harmful effect are known to those skilled in the art or can be verified by conducting a simple test. Typically, such materials are, but not limited to, any additives and ingredients involved in cell culture approved by the Ministry of Health, Labor and Welfare (or PMDA), FDA, or the like. The cell-derived material representatively includes extracellular matrices. In particular, the three-dimensional synthetic tissue used in the present invention preferably comprises a cell and an extracellular matrix at a suitable ratio. Examples of such a suitable ratio of cell to extracellular matrices include 1:3 to 20:1. The strength of a tissue is adjusted by the ratio of cell to extracellular matrices. Thus, the ratio of cell to extracellular matrices can be adjusted for use in accordance with application of cell implantation and physical environment at the implantation site. The preferable ratio varies depending on the intended treatment. Such a variation is apparent to those skilled in the art and can be estimated by investigating the ratio of a cell in an organ of interest to an extracellular matrix.

In a preferred embodiment, the extracellular matrix comprised in a three-dimensional synthetic tissue used in the present invention contains fibronectin, collagen I, collagen III, and vitronectin. Preferably, various such extracellular matrices contain all of those listed above. It is advantageous that they are integrated and mixed. In another preferred embodiment, the extracellular matrix is diffusedly distributed in the tissue. Alternatively, extracellular matrices are preferably scattered across the entire tissue. Such a distribution achieves a significant effect of improving compatibility and affinity with an environment when implanted. In the preferred embodiment, for extracellular matrices that are diffusedly distributed in the three-dimensional synthetic tissue used in the present invention, distribution densities in any two section of 1 cm$^2$, when compared, preferably have a ratio within the range of about 1:3 to 3:1. Any known method in the art can be used for measuring distribution density, including, for example, immunostaining. In the preferred embodiment, for extracellular matrices used in the three-dimensional synthetic tissue used in the present invention, distribution densities in any two section of 1 cm$^2$, when compared, preferably have a ratio within the range of about 1:2 to 2:1 and more preferably about 1.5:1-1.5:1. The diffusion of distribution of extracellular matrices is advantageously uniform. Preferably, extracellular matrix is substantially uniformly dispersed, but not limited thereto. The three-dimensional synthetic tissue used in present invention is known to be characterized in that adhesion to intercellular matrix, which promotes cell adhesion to a matrix, cell extension, and cell chemotaxis, is promoted especially by including collagen (Types I, III), as well as vitronectin, fibronectin, and the like. In one embodiment, an extracellular matrix distributed in a three-dimensional synthetic tissue used in the present invention may include collagen I, collagen III, vitronectin, fibronectin or the like. However, a synthetic tissue which integrally includes collagen (Types I, III), vitronectin, fibronectin, and the like has not been provided. Though not wishing to be bound by any theory, collagen (Types I, III), vitronectin, fibronectin, and the like are considered to play a role in exhibiting the biological integration capability with the surrounding. Therefore, in a preferable embodiment, it is advantageous that vitronectin are diffusedly distributed on a surface of the three-dimensional synthetic tissue used in the present invention. This is due to the belief that adhesion, affinity, and stability after implantation would be significantly different. Thus, in one embodiment of the present invention, the extracellular matrix encompassed by the present invention contains collagen I and/or collagen III and there is more of the collagen I and/or collagen III than collagen II.

In a preferable embodiment, the three-dimensional synthetic tissue used in the present invention has a capability to biologically integrate with the surroundings. As used herein, the term surrounding typically refers to an implanted environment, and examples thereof include tissues and cells. The biological integration capability of surrounding tissues, cells, and the like can be confirmed by photomicrograph, physical test, staining of a biological marker, or the like. Conventional synthetic tissues have a low affinity to tissues or the like in an implanted environment. It was not even assumed that conventional synthetic tissues can exhibit biological integration capability. Conventional synthetic tissues depend on a regeneration capability of an organism, serving as a temporary solution until autologous cells or the like gather and regenerate. Thus, these conventional synthetic tissues are not intended for permanent use. Therefore, the composite tissue of the present invention should be deemed capable of being a practical part in an implantation treatment. Thus, the biological integration capability mentioned in the present invention preferably includes the capability to adhere to surrounding cells and/or extracellular matrices. Such an adhesion capability can be measured by an in vitro culturing assay with a tissue section (e.g., a cartilage section). In addition, it is demonstrated that sufficient biological integration capability is exerted and a therapeutic effect with excellent integration condition was achieved at a level that could not be achieved with conventional art by using a composite tissue of the present invention. In a preferred embodiment, the synthetic tissue or complex of the present invention biologically integrates in all three dimensional directions. Synthetic tissues prepared by conventional methods have biological integration in two dimensional directions to some degree. However, no tissue having biological integration in all three dimensional directions is prepared by conventional methods. Therefore, since the three-dimensional synthetic tissue used in the present invention biologically integrates to all three dimensional directions in this manner, the three-dimensional synthetic tissue has a property of being substantially implantable in any application. Examples of indicative biological integration in the present invention include, but are not limited to, interconnection of extracellular matrices, electrical integration, and the presence of intracellular signal transduction. The interaction of extracellular matrices can be observed with a microscope by staining intracellular adhesion as appropriate. Electrical integration can be observed by measuring electric potential.

It is preferable that fibronectin is also distributed in the three-dimensional synthetic tissue used in the present invention. It is known that fibronectin plays a role in cell adhesion, in cell shape regulation, and in adjustment in cell migration. However, a synthetic tissue in which fibronectin is expressed has not been provided. Though not wishing to be bound by any theory, fibronectin is also considered to play a role in exerting a capability to biologically integrate with the surrounding. Therefore, in a preferable embodiment, it is advantageous that fibronectin is also diffusedly distributed on a surface of three-dimensional synthetic tissue used in the present invention. This is because it is considered that adhesion, affinity, and stability after implantation would be significantly different.

Since the three-dimensional synthetic tissue used in the present invention includes an abundance of adhesion molecules such as extracellular matrix including collagen (types I, III, etc.), vironectin, and fibronectin, the tissue is accepted by the surrounding tissue. Thus, implanted cells can be stably accepted by the implantation site. In conventional cell implantation, it was difficult for cells to be stably accepted by the implantation site not only in cells implantation without a scaffold, but also in cell implantation using an additional stabilizing treatment (sewing of a patch, use of scaffold, etc.). However, use of the present invention facilitates stabilization. When only cells are used, reinforcement by another tissue, fixing scaffold, or the like is necessary. However, if a three-dimensional synthetic tissue of the present invention or a composite tissue comprising the same is used, cells which may have pluripotency included in the three-dimensional synthetic tissue can be stably accepted, without requiring such means, by the implantation site without an additional fixation means.

In another preferred embodiment, the extracellular matrix and the induced mesenchymal stem cell integrate to form a three-dimensional structure together. In another preferred embodiment, the extracellular matrix and the cell have an ability to integrate with the surroundings when implanted and have sufficient strength to provide a self-supporting ability. Preferably, the three-dimensional synthetic tissue that may be used is substantially made of the induced mesenchymal stem cell and the extracellular matrix derived from the cell. The extracellular matrix contains collagen I and/or collagen III, and there are more of the collagen I and/or collagen III than collagen II. The extracellular matrix is diffusedly distributed in the tissue. Such a three-dimensional synthetic tissue is implantable and has tissue strength capable of being used in clinical applications. Such a three-dimensional synthetic tissue is also characterized in being scaffold-free. Mesenchymal stem cells are used in the present invention, they are not somatic cells obtained from an actual tissue, but induced mesenchymal stem cells. Such induced cells may be any less differentiated stem cells such as those differentiated from ES cells or iPS cells. Any method of induction can be used therefor. However, in a preferred embodiment, induction in low oxygen condition may be preferred.

In an alternative embodiment, the three-dimensional synthetic tissue of the present invention may employ heterologous cells, allogenic cells, isogenic cells or autologous cells. In the present invention, it was found that even when allogenic cells, and particularly mesenchymal cells are used, no adverse reaction such as immune rejection occurred. Thus, the present invention leads to the development of the treatment of *ex vivo,* as well as a therapeutic method which produces a synthetic tissue using induced mesenchymal stem cells of others and utilizes the tissue without using an immune rejection suppressor or the like.

Cells in the three-dimensional synthetic tissue used in the present invention may be one type of induced mesenchymal stem cells or multiple types of induced mesenchymal stem cells. Cells in the three-dimensional synthetic tissue used in the present invention are induced mesenchymal cells (e.g. , cells derived from another line having the features of the mesenchymal line). Though not wishing to be bound by any theory, the mesenchymal cells are used because the mesenchymal cells themselves are highly compatible with organs such as bones, while induced mesenchymal cells further have excellent ability to differentiate into cartilage or the like and may have an ability to differentiate into various tissue or organs. In addition, this is because it was found as a result thereof that the therapeutic results were desirable and the therapeutic speed was also improved.

In the preferred embodiment, it is advantageous that cells used in the three-dimensional synthetic tissue used in the present invention are cells derived from the subject to which the tissue is applied. In such a case, cells as used herein also are referred to as autologous cells. Immune rejection reactions can be prevented or reduced by using autologous cells. Alternatively, in another embodiment, cells used in the three-dimensional synthetic tissue used in the present invention may not be cells derived from a subject to which the tissue is applied. Even in such a case, since an induced mesenchymal stem cell is used, measures to prevent immune rejection reactions are generally not necessary. However, measures to prevent immune rejection reactions may be taken.

In a preferable embodiment, the three-dimensional synthetic tissue used in the present invention has sufficient tissue strength for clinical applications. The sufficient tissue strength for clinical applications varies depending on a site to which the synthetic tissue is intended to be applied. Such strength can be determined by those skilled in the art by referring to the disclosure of the specification and techniques well known in the art. The tensile strength of the three-dimensional synthetic tissue used in the present invention may be low. Such tensile strength increases when the matrix concentration is increased, and decreases when the cell ratio is increased in a cell/extracellular matrix ratio. The present invention is characterized in that the strength can be freely adjusted as needed. The present invention is also characterized in that the strength can set to be relatively high or low to approximate that of a tissue to be implanted. Therefore, it is understood that the goal can be set to comply with any site.

In another embodiment, it is preferable that strength of the three-dimensional synthetic tissue used in the present invention is sufficient for having a self-supporting ability. Conventional synthetic tissues do not have a self-supporting ability after production. Therefore, when synthetic tissues made from a technique that is not part of the present invention are transferred, at least a part of them are injured. However, when the technique of the present invention is used, a synthetic tissue having a self-supporting ability is provided. Preferable self-supporting ability is such that, when a tissue is picked up with tweezers having tips with a thickness of 0.5 to 3 mm (preferably, tips with a thickness of 1 to 2 mm, and more preferably, tips with a thickness of 1 mm), the tissue is not substantially destroyed. Herein, whether the tissue is substantially destroyed can be visually confirmed, or also by performing, for example, a water leakage test after the tissue is picked up in the above-described conditions and confirming that water does not leak. Alternatively, the self-supporting ability as described above can also be confirmed by not being destroyed when picked up by fingers instead of tweezers. In a particular embodiment of the present invention, portions to which clinical application is intended include, but are not limited to, a bone, a joint, a cartilage, a meniscus, a tendon, and a ligament. The origin of cells contained in the synthetic tissue of the present invention is characterized in not being affected by clinical applications. Further, when a site of defect is a cartilage portion, the attachment ability of the synthetic tissue can be tested by determining whether the synthetic tissue remains attached without an artificial fixation procedure when the synthetic tissue is implanted into a defect portion of the intra-articular tissue (e.g., 2, 3 minutes later).

The three-dimensional synthetic tissue used in the present invention is an implantable synthetic tissue. Attempts have been heretofore made to produce synthetic tissues by cell culture. However, there were no synthetic tissues suitable for implantation in terms of size, strength, physical injuries when it is detached from a culture container, or the like. The present invention utilizes a tissue culturing method which cultures cells in the presence of extracellular matrix synthesis promoting agent as described in detail in another section of the specification, resulting in no issues in terms of size or strength, in addition to imposing no particular difficulty in detaching the cells. The three-dimensional synthetic tissue used in the present invention is provided by utilizing a tissue culture method as described in detail in another section of the specification as an implantable synthetic tissue. The three-dimensional synthetic tissue used in the present invention provides a complex comprising a cell and a component derived from the cell. Herein, it is understood that, preferably, the complex is substantially made of cells and the components derived from the cells. Herein, the complex of the present invention is provided for reinforcing, repairing, or regenerating a part of an organism. As used herein, the term "complex" means that cells and other components are integrated into a complex by some kind of interaction. Therefore, the complex of the present invention often has an appearance like a synthetic tissue, and it is understood that the meaning of the term "complex" overlaps with what is referred to by a synthetic tissue. It should be noted that "complex" itself is a synthetic tissue, which is different from a "composite tissue". A "complex" can be one component of a "composite tissue" of the present invention.

In another embodiment, the three-dimensional synthetic tissue used in the present invention is preferably isolated. In this case, it should be noted that the term "isolate" means that the three-dimensional synthetic tissue is separated from a scaffold, a support, a culture solution and the like used in culture and separated from anything other than an artificial bone used in a composite tissue. The three-dimensional synthetic tissue used in the present invention is substantially free of materials such as a scaffold so that it is possible to suppress adverse reactions after implantation, such as immune rejection reactions or inflammatory reactions. The base area of the composite tissue of the present invention and thus the three-dimensional synthetic tissue included therein may be, for example, about 1 $cm^2$ to 20 $cm^2$. However, the area is not limited thereto and may be less than about 1 $cm^2$ or greater than about 20 $cm^2$. It is understood that the essential feature of the present invention is that a tissue of any size (area, volume) can be produced, and it is not limited in size.

In a preferable embodiment, the three-dimensional synthetic tissue used in the present invention is thick. The term "thick" typically means that the three-dimensional synthetic tissue has a thickness which provides sufficient strength to cover a site to which the synthetic tissue is implanted. Such a thickness is, for example, at least about 50 $\mu$m or greater, more preferably at least about 100 $\mu$m or greater, at least about 200 $\mu$m or greater, at least about 300 $\mu$m or greater, even more preferably at least about 400 $\mu$m or greater, still more preferably at least about 500 $\mu$m or about 1 mm. It is understood that, in some cases, a tissue having a thickness of about 3 mm or greater and a tissue having a thickness of about 5 mm or greater can also be produced. Alternatively, such a thickness may be less than about 1 mm. It is understood that an essential feature of the present invention is that a tissue or a complex having any thickness can be produced, and the tissue or complex is not limited in size.

The three-dimensional synthetic tissue used in the present invention provides a scaffold-free synthetic tissue. By providing such a scaffold-free synthetic tissue, a therapeutic method and a therapeutic agent for providing an excellent condition after implantation can be obtained. The three-dimensional synthetic tissue used in the present invention solves all long outstanding problems with biological formulations, which are attributed to contamination of the scaffold itself by utilizing a scaffold-free synthetic tissue. Despite the absence of a scaffold, the therapeutic effect is comparable with or more satisfactory than conventional techniques. In addition, when a scaffold is used, the alignment of implanted cells in the scaffold, the cell-to-cell adhesion, *in vivo* alteration of the scaffold itself (eliciting inflammation), acceptance of the scaffold by recipient tissue, and the like become problematic. However, these problems can be solved by the present invention. In particular, in the present invention, usefulness of use of a synthetic tissue in cartilage regeneration is directly passed on to use of a proven composite tissue having an artificial bone with usefulness and safety that are already proven as bone regenerating implant in terms of preferably using an actual bone component and being free of biological formulation and synthetic polymer. This is recognized as an advantageous point in comparison to conventional synthetic tissue, composite tissue and the like. The three-dimensional synthetic tissue used in the present invention is also different from methods using conventional cell therapy in that the three-dimensional synthetic tissue is self-organized and biologically integrated inside. It is easy to form a three-dimensional structure with the three-dimensional synthetic tissue used in the present invention, and thus it is easy to design the tissue into a desired form. Thus, the versatility thereof should be noted. Further, treatment of sites that could not be considered for implantation treatment with conventional synthetic products is made possible. The synthetic tissue of the present invention biologically integrates within tissues and with the environment and actually works in implantation therapies. The composite tissue of the present invention has a capability to biologically integrate with surrounding tissues, cells, and the like after implantation (preferably by extracellular matrix). Therefore, the post-operational acceptance is satisfactory. Thus, the composite tissue of the present invention enables medical treatment which provides a therapeutic effect by filling, replacing, and/or covering an affected portion.

The three-dimensional synthetic tissue used in the present invention biologically integrates with the environment after implantation, such as surrounding tissues and cells. Therefore, excellent results are achieved such as satisfactory post-operational acceptance and cells being reliably supplied. An effect of the present invention is that such satisfactory biological integration allows the formation of a composite tissue with another synthetic tissue or the like, thus enabling a more complex therapy. Another effect of the three-dimensional synthetic tissue used in the present invention is that differentiation can be induced after a tissue is provided as a three-dimensional synthetic tissue. Alternatively, differentiation is induced so that such a three-dimensional synthetic tissue can be formed before providing a three-dimensional synthetic tissue. Another effect of the three-dimensional synthetic tissue used in the present invention is that the cell implantation achieves an effect such as satisfactory replacement ability and a comprehensive supply of cells by covering, compared to conventional cell-only implantation and sheet implantation. The composite tissue of the present invention provides an implantable synthetic tissue that has biological integration capability by a three-dimensional synthetic tissue used in the present invention. The above-described characteristics and effects of such a tissue make it possible to treat a site which could not be considered as an implantation site for conventional synthetic products. The three-dimensional synthetic tissue used in the present invention has biological integration in tissues and between other tissues and actually works in implantation therapies. The synthetic tissue is not provided by conventional techniques and is first provided by the present invention. The three-dimensional synthetic tissue used in the present invention has an ability to biologically integrate with surrounding tissues, cells, and the like after implantation, thus having excellent post-operation results. A synthetic tissue having such biological integration capability does not exist outside of the method used in the present invention. Thus, the present invention achieves a therapeutic effect that could not be accomplished with a synthetic tissue by a method other than this method. The composite tissue of the present invention enables medical treatment which provides a therapeutic effect by filling, replacing, and/or covering an affected portion.

In a preferred embodiment, a three-dimensional synthetic tissue used in the present invention is biologically integrated in three dimensional directions. The three-dimensional synthetic tissue is in an adhering state upon integration with an artificial bone, which is essentially recognized as a close adhesion. In this regard, biological integration is explained in other portions of the present specification. For example, biological integration includes, but is not limited to, physical integration by an extracellular matrix and electric integration. It is particularly important that an extracellular matrix within a tissue is biologically integrated. A synthetic tissue in such a biologically integrated state is not provided by methods other than the method used by the present invention. Furthermore, in a preferred embodiment of having a capability to biologically integrate with the surrounding, a synthetic tissue is recognized as achieving a significant effect in terms of providing a composite tissue comprising a synthetic tissue capable of constituting a part of a living body even after implantation. The present invention can provide a composite tissue comprising a synthetic tissue which is first frozen to kill cells so that cells are practically not included. Such a tissue is still unique in that the tissue has a property to adhere to the surrounding even in such a case.

An extracellular matrix or a cell adhesion molecule, such as fibronectin or vitronectin, is distributed throughout the three-dimensional synthetic tissue used in the present invention. Meanwhile, in cell sheet engineering, a cell adhesion molecule is localized on a surface of culture cells which is attached to a Petri dish. The most prominent difference therebetween is that, in the sheet provided by the cell sheet engineering, cells are the major component of the sheet. The sheet is recognized as a mass of cells with glue of an adhesion molecule attached on the bottom surface. The synthetic tissue of the present inventors, however, is literally a "tissue" such that an extracellular matrix surrounds cells. Thus, the present invention is significantly different from conventional techniques. The present invention achieves improvement in acceptance of other synthetic tissues such as an artificial bone.

In one embodiment, the three-dimensional synthetic tissue used in the present invention can be deemed different from conventional synthetic tissues in that the former comprises a cell. Particularly, high density thereof should be noted in that cells can be included at a density of up to $5 \times 10^6/cm^2$. The present invention is noteworthy in that it is suitable for implanting cells rather than implanting a tissue.

In one embodiment, the mesenchymal stem cell used in the present invention or an equivalent cell thereof is made in low oxygen conditions. More preferably, the mesenchymal stem cell of the present invention or an equivalent cell thereof is obtained by culturing the pluripotent stem cells (e.g., ES cells or iPS cells) in suspension culture to form an embryoid body and culturing the embryoid body in 1% oxygen condition. An example of such a low oxygen condition includes the method described in Non Patent Literature 5. Alternatively, it is understood that any method illustrated in the Examples herein is used.

(Composite tissue of induced three-dimensional synthetic tissue and artificial bone)

In one aspect, the present invention provides a composite tissue for treating or preventing a disease, disorder, or condition associated with an osteochondral defect, comprising a three-dimensional synthetic tissue and an artificial bone. The composite tissue of the present invention has successfully healed a disease, disorder, or condition associated with an osteochondral defect at a level that was impossible with conventional techniques. Thus, the present invention achieves a significant effect in terms of drastic improvement in therapeutic results. It is understood that any embodiment described in (Induced three-dimensional synthetic tissue) may be used as an induced three-dimensional synthetic tissue used in the composite tissue of the present invention.

Recovery has been observed in rabbits one month from a surgical operation. Thus, the composite tissue of the present invention achieves an early and more complete healing, which was not possible with conventional therapeutic methods. That is, the characteristic effect of the present invention is the speed of "integration" and healing (data at 1 month) in comparison to conventional methods. In a rabbit model, natural healing is observed after about 6 months. However, there was a significant difference in the level and quality of healing such as integration at 6 months. Thus, the present invention is recognized as capable of achieving an early and more complete healing that could not be achieved by conventional therapeutic methods. The present invention is proven in rabbits, while the rabbits are established models for other animals such as humans. For humans , those skilled in the art can understand that a similar effect is achieved in "mammals" in general because the above results are proven examples with rabbits, which are established models in osteochondral defect treatment. The following references can be referred for such animal models, which constitute the common general knowledge in the art.

<Examples of references of conventional techniques in animal models>

*F. Berenbaum, The OARSI histopathology initiative - the tasks and limitations, Osteoarthritis and Cartilage 18 (2010) S1

*Trattnig S, Winalski CS, Marlovits S, Jurvelin JS, Welsch GH, Potter HG. Magnetic resonance imaging of cartilage repair: a review. Cartilage. 2011; 2:5-26.

*Mithoefer K, Saris DBF, Farr J, Kon E, Zaslav K, Cole B, Ranstam J, Yao J, Shive MS, Brittberg M. Guidelines for the design and conduct of clinical studies in knee articular cartilage repair: International Cartilage Repair Society recommendations based on current scientific evidence and standards of clinical care. Cartilage. 2011; 2: 100-121.

*Roos EM, Engelhart L, Ranstam J, Anderson AF, Irrgang JJ, Marx R, Tegner Y, Davis AM. Patient-reported outcome instruments for use in patients with articular cartilage injuries. Cartilage. 2011; 2:122-136.

*Hurtig M, Buschmann MD, Fortier L, Hoemann CD, Hunziker EB, Jurvelin JS, Mainil-Varlet P, McIlwraith W, Sah RL, Whiteside RA. Preclinical studies for cartilage repair: recommendations from the International Cartilage Repair Society. Cartilage. 2011; 2:137-153.

*Hoemann CD, Kandel R, Roberts S, Saris D, Creemers L, Manil-Varlet P, Methot S, Hollander A, Buschmann MD. Recommended guidelines for histological endpoints for cartilage repair studies in animal models and clinical trials. Cartilage. 2011; 2:154-173.

*C. Wayne McIlwraith and David D. Frisbie, Microfracture: Basic Science Studies in the Horse, Cartilage 2010 1: 87-95

*F. Berenbaum, The OARSI histopathology initiative - the tasks and limitations Osteoarthritis and Cartilage 18 (2010) S1

*T. Aigner, J.L. Cook, N. Gerwin, S.S. Glasson, S. Laverty, C.B. Little, W. McIlwraith, V.B. Kraus, Histopathology atlas of animal model systems e overview of guiding principles Osteoarthritis and Cartilage 18 (2010) S2-S6

*K.P.H. Pritzker, T. Aigner, Terminology of osteoarthritis cartilage and bone histopathology - a proposal for a consensus Osteoarthritis and Cartilage 18 (2010) S7-S9

*R. Poole, S. Blake, M. Buschmann, S. Goldring, S. Laverty S. Lockwood, J. Matyas, J. McDougall, K. Pritzker, K. Rudolphi, W. van den Berg, T. Yaksh, Recommendations for the use of preclinical models in the study and treatment of osteoarthritis, Osteoarthritis and Cartilage 18 (2010) S10-S16

*S.S. Glasson, M.G. Chambers, W.B. Van Den Berg, C.B. Little, The OARSI histopathology initiative e recommendations for histological assessments of osteoarthritis in the mouse, Osteoarthritis and Cartilage 18 (2010) S17-S23

*N. Gerwin, A.M. Bendele, S. Glasson, C.S. Carlson, The OARSI histopathology initiative - recommendations for histological assessments of osteoarthritis in the rat, Osteoarthritis and Cartilage 18 (2010) S24-S34

*V.B. Kraus, J.L. Huebner, J. DeGroot, A. Bendele, The OARSI histopathology initiative - recommendations for histological assessments of osteoarthritis in the guinea pig, Osteoarthritis and Cartilage 18 (2010) S35-S52

*S. Laverty, C.A. Girard, J.M. Williams, E.B. Hunziker, K.P.H. Pritzker, The OARSI histopathology initiative - recommendations for histological assessments of osteoarthritis in the rabbit, Osteoarthritis and Cartilage 18 (2010) S53-S65

*J.L. Cook, K. Kuroki, D. Visco, J.-P. Pelletier, L. Schulz, F.P.J.G Lafeber, The OARSI histopathology initiative - recommendations for histological assessments of osteoarthritis in the dog, Osteoarthritis and Cartilage 18 (2010) S66-S79

*C.B. Little, M.M. Smith, M.A. Cake, R.A. Read, M.J. Murphy, F.P. Barry, The OARSI histopathology initiative - recommendations for histological assessments of osteoarthritis in sheep and goats, Osteoarthritis and Cartilage 18 (2010) S80-S92

*C.W. McIlwraith, D.D. Frisbie, C.E. Kawcak, C.J. Fuller, M. Hurtig, A. Cruz, The OARSI histopathology initiative - recommendations for histological assessments of osteoarthritis in the horse, Osteoarthritis and Cartilage 18 (2010) S93-S105

*P.C Pastoureau, E.B Hunziker, J.-P. Pelletier, Cartilage, bone and synovial histomorphometry in animal models of osteoarthritis, Osteoarthritis and Cartilage 18 (2010) S106-S112

*N. Schmitz, S. Laverty, V.B. Kraus, T. Aigner, Basic methods in histopathology of joint tissues, Osteoarthritis and Cartilage 18 (2010) S113-S116

*G.L. Pearce, D.D. Frisbie, Statistical evaluation of biomedical studies, Osteoarthritis and Cartilage 18 (2010) S117-S122

In a preferred embodiment, an artificial bone that is included in a composite tissue of the present invention is preferably smaller in size than a depth of a defect of a bone section in the osteochondral defect. Though not wishing to be bound by any theory, since it was found that this size creates a margin for a cartilage to form in a defect portion so that a bone/cartilage undergoes smooth biological integration or biological union, it is believed that it may be preferable to specify the size relative to the depth of the defect for improvement in therapeutic results. As a representative example, when an osteochondral defect is about 6 mm in a small animal such as a rabbit, a cartilage portion would be about 300-400 $\mu$m. Thus, the conditions would be met if the depth of an artificial bone is about 4 mm and a three-dimensional synthetic tissue (TEC) is about 0.5-2 mm. The thickness of a cartilage varies by animals. In humans, it is understood to be about 1mm to about 5 mm, depending on the site. Thus, when a cartilage portion is about 3 mm, a composite tissue can be produced by adding a three-dimensional synthetic tissue (TEC) deeper than about 3 mm, e.g., about 4 mm, from the depth of an osteochondral defect (mm) to an artificial bone with a length that is about 4mm less than the depth of a defect. Alternatively, as another embodiment, it is possible to implant at a constant depth irrespective of the thickness of a cartilage. In one embodiment, the artificial bone is smaller in size than a depth of a defect of a bone section in the osteochondral defect by about 1 mm or greater. In another embodiment, the artificial bone is smaller in size than a depth of a defect of a bone section in the osteochondral defect by twice the thickness of a cartilage or less. In yet another embodiment, the artificial bone is smaller in size than a depth of a defect of a bone section in the osteochondral defect by about 1 mm or greater and twice the thickness of a cartilage or less. Thus , for example, a boundary surface of a TEC/artificial bone complex is preferably at a position that has an addition about 1 mm to about 6 mm in depth from a native bone/cartilage boundary in humans. Preferably, the artificial bone is advantageously smaller than a depth of a defect of a bone section in the osteochondral defect by about 2 mm to about 4 mm. When the depth is shallow as in about 2 mm, a subchondral bone is repaired quickly, but a cartilage is poorly repaired. When the depth is deep as in about 4 mm, a cartilage is repaired satisfactorily, but the repair of subchondral bone is prolonged. Thus, although it depends of the case, in one embodiment, the depth is preferably about 3 mm from the surface layer of a cartilage. For example, a cartilage portion is about 1-5 mm in humans, and typically about 3 mm. Thus, in a typical case, an artificial bone is preferably about 1 mm as the minimum in terms of depth from the bone/cartilage boundary. Hence, it is preferable to use a depth of about 1 mm as the lower limit and about 6 mm, which is twice the cartilage portion, as the upper limit. In this case, if a defect of a bone portion is presumed to be about 10 mm, a portion of an artificial bone that is used would be about 9 mm to about 4 mm. Thus, since the thickness of an adjacent cartilage is approximately constant by the animal in this embodiment, a composite tissue can be suitably sized from the depth of a bone portion depending on the target animal. For example, it is known that the thickness of a cartilage in humans is about 1 mm for a joint cartilage to about 5mm for the largest patella cartilage. In addition, the thickness is known to vary by site. Thus, the thickness of a cartilage can be determined depending on the site. The cartilage thickness is known in the art for animals other than humans and rabbits. Thus, it is possible to determine the cartilage thickness by referring to references described in <Examples of references of conventional techniques in animal models> and other well-known references. A composite tissue of the present invention can be structured in accordance with such thickness.

In a more preferred embodiment, it is preferred that the total of depths of the artificial bone and the three-dimensional synthetic tissue is nearly the same as a depth of the osteochondral defect. A measurement error may be acceptable for the total depth (length). For example, similar depths with a measurement error of about 1 mm can be deemed approximately the same. However, it is preferable that the total depth is not longer than the depth of an osteochondral defect (e.g. , the total of depths of an artificial bone and a three-dimensional synthetic tissue is the same or about 1 mm shorter than the depth of the osteochondral defect). Though not wishing to be bound by any theory, since it was found that this size creates a margin for a cartilage to form in a defective portion so that a bone/cartilage undergoes smooth biological integration or biological union, it is believed that it may be preferable to specify the size relative to the depth of the defect for improvement in therapeutic results. More preferably, the artificial bone is smaller in size than a depth of a defect of a bone section in the osteochondral defect, and the total of depths of the artificial bone and the three-dimensional synthetic tissue is nearly the same as the depth of the osteochondral defect. This is because both of the advantages are utilized by combining both of these features.

In a preferred embodiment, a three-dimensional synthetic tissue and an artificial bone are diphasic in a composite tissue of the present invention. That is, in a preferred embodiment, these two components are preferably unmixed and are present as substantially separate constituent elements in a composite tissue of the present invention. Thus, as used herein, the term "biphasic" refers to a state of two or more components being unmixed and present as substantially separate constituent elements. Though not wishing to be bound by any theory, it is demonstrated that subchondral bone formation is promoted thereby in a three-dimensional synthetic tissue.

In one embodiment, the three dimensional synthetic tissue and the artificial bone are attached to each other. The three-dimensional synthetic tissue used in the present invention can adhere or closely adhere to another synthetic tissue such as an artificial bone (including, for example, artificial bones made of calcium phosphate, hydroxy apatite or the like). In addition, the three-dimensional synthetic tissue can be provided in an integrated form as a composite tissue. In this manner, it is understood that the composite tissue of the present invention has a feature that is helpful in improving therapeutic results after implantation.

It is understood that a composite tissue of the present invention can be used in any animal having an osteochondral defect in an osteochondral tissue. In one embodiment, an example of such an animal is a mammal. In particular, it was difficult for an osteochondral defect of primates including humans to completely heal. Thus, it should be noted that the present invention can achieve a significant effect in terms of achieving a complete recovery or a condition close thereto.

Anything that is known as a bone substitute can be used as an artificial bone used in the present invention. For example, it is possible to use a material that has excellent affinity to a bone in a body, such as hydroxyapatite, β-tricalcium phosphate, silicon, bioceramics such as carbon, alumina, or zirconia, metals such as titanium or tungsten, and materials made of materials such as coral materials. A representative examples thereof include, but are not limited to, porous β-tricalcium phosphate (β-TCP) (Olympus and the like), hydroxyapatite synthetic bone substitute material NEOBONE® (MMT Co., Ltd.), Apacerum, Superpore, Cellyard, Biopex-R, Bonetite, and Bonefill (each from Hoya/Pentax).

In one embodiment, an artificial bone that can be used in a composite tissue of the present invention may be made of a material selected from the group consisting of hydroxyapatite and β-tricalcium phosphate.

It is understood that the present invention may target any disease, disorder or condition associated with osteochondral disorders for treatment or prevention. Examples of such a disease, disorder and condition especially include, but are not limited to, any disease involving osteochondral degeneration, necrosis or injury, including osteoarthritis, osteochondral injury, osteonecrosis, osteochondral injury, intractable fracture, bone tumor and other similar diseases (bone cyst and the like), osteochondral lesion, bone necrosis, rheumatoid arthritis, cartilage injury (cartilage full thickness injury, cartilage partial injury, osteochondral injury and the like), meniscus injury, ligament injury, conditions requiring ligament repair (chronic injury, degenerative tear, biological augmentation for reconstructive surgery, etc.), tendon injury, conditions requiring tendon (including Achilles' heel) repair (chronic injury, degenerative tear, biological augmentation for reconstructive surgery, etc.), cartilage degeneration, meniscus degeneration, intervertebral disk denaturation, ligament degeneration or tendon degeneration, delayed union; nonunion; skeletal muscle repair/regeneration and any other diseases with injury to tissue or faulty union of a bone and body inserts such as artificial joint.

As used herein, the term "prophylaxis" or "prevention" in relation to a certain disease or disorder refers to a treatment which keeps such a condition from happening before the condition is induced, or causes the condition to occur at a reduced level or to be delayed.

As used herein, the term "therapy" in relation to a certain disease or disorder means that when such a condition occurs, such a disease or disorder is prevented from deteriorating, preferably is maintained in the current condition, more preferably is diminished, and even more preferably extinguished. As used herein, the term "radical therapy" refers to a therapy which eradicates the root or cause of a pathological process. Therefore, when a radical therapy is performed for a disease, there is no recurrence of the disease in principle.

As used herein, the term "prognosis" is also referred to as prognostic treatment. The term "prognosis" in relation to a certain disease or disorder refers to a diagnosis or treatment of such a condition after a therapy.

The composite tissue of the present invention may be provided as a drug. Alternatively, the composite tissue of the present invention may be prepared by a physician in a clinical setting, or a physician may first prepare the cells, and then a third party may culture the cells for preparation as a three-dimensional synthetic tissue to attach the tissue to an artificial bone for use in a surgery. In such a case, cells are not necessarily cultured by a physician. The cells can be cultured instead by those skilled in the art of cell culture. Thus, those skilled in the art can determine culturing conditions in accordance with the type of cells and intended implantation site after reading the disclosure herein.

From another viewpoint, the present invention provides a composite tissue comprising a scaffold-free three dimensional synthetic tissue. By providing such a composite tissue comprising a scaffold-free synthetic tissue, a therapeutic method and a therapeutic agent for providing an excellent condition after implantation can be obtained.

From another viewpoint, a composite tissue comprising a scaffold-free synthetic tissue solves a long outstanding problem with biological formulations, which is attributed to contamination of the scaffold itself. Despite the absence of a scaffold, the therapeutic effect is comparable with, or more satisfactory than conventional techniques. When a scaffold is used, the alignment of implanted cells in the scaffold, the cell-to-cell adhesion, in *vivo* alteration of the scaffold itself (eliciting inflammation), the integration of the scaffold to recipient tissue, and the like become problematic. However, these problems can be solved by the present invention. The three-dimensional synthetic tissue used in the present invention is also self-organized and biologically integrates inside. Thus, the present invention is distinguished from conventional cell therapies in terms of these points. It is easy to form a three-dimensional structure with the three-dimensional synthetic tissue used in the present invention. In addition, it is easy to design the tissue into a desired form. Thus, the versatility of the three-dimensional synthetic tissue used in the present invention is noteworthy. The three-dimensional synthetic tissue used in the present invention biologically integrates with the post-implantation environment, such as surrounding tissues and cells. Therefore, excellent effects are achieved, e.g. , the post-operational stability is satisfactory and cells are reliably supplied. The satisfactory biological integration capability from use of the present invention results in very satisfactory adhesion upon the formation of a composite tissue with another synthetic tissue such as an artificial bone, thus enabling in a complicated the rapy by using acomposite tissue of the present invention. Another effect of the composite tissue of the present invention is that differentiation can be induced after providing a tissue as a composite tissue of the present invention. Alternatively, differentiation can be induced before providing a tissue as a composite tissue of the present invention to form such a composite tissue of the present invention. In terms of cell implantation, the present invention provides effects such as satisfactory replacement ability and a comprehensive supply of cells by covering, compared to conventional cell-only implantation, sheet implantation, and the like.

The three-dimensional synthetic tissue used in the present invention is free of injury caused by a protein degradation enzyme, such as, representatively, dispase or trypsin, during culture. Therefore, the three-dimensional synthetic tissue can be recovered as a cell mass with strength for holding proteins between cells, between cells and extracellular matrix, and between extracellular matrices. The three-dimensional synthetic tissue also retains functions intact as a cell-extracellular matrix complex as a three-dimensional structure. When typical protein degradation enzymes such as trypsin are used, substantially no cell-to-cell link or cell-to-extracellular matrix link are retained, so that cells are individually separated and detached. Among these protein degradation enzymes, dispase destroys most of basement membrane-like proteins between cells and base materials. In this case, the resultant synthetic tissue has weak strength.

The composite tissue of the present invention achieves a level of histological score related to cartilage repair and histological score related to subchondral bones (e.g., "O'Driscoll score related to bone layer reconstruction", "surface", "matrix", "exposure of subchondral bone", "alignment of subchondral bone", "biological union of bone" (also referred to as "integration"), "bone infiltration into defect region", "hardening of cartilage" and "cell form" (cell distribution, survival rate of cell population)) that could not be achieved conventionally (see O'Driscoll SW, Keeley FW, Salter Rub. J Bone Joint Surg Am 1988; 70:595-606; Mrosek EH, Schagemann JC, Chung HW, Fitzsimmons JS, Yaszemski MJ, Mardones RM, et al. , J Orthop Res 2010; 28:141-148; Olivos-Meza A, Fitzsimmons JS, Casper ME, Chen Q, An KN, Ruesink TJ, et al. , Osteoarthritis Cartilage 2010; 18:1183-1191, Examples and the like). Items that can be examined maybe as follows. These items can be examined whether improvement is observed in comparison to prior art.

O'Driscoll score related to cartilage layer: Cell form; Matrix; Dye affinity of tissue; Continuity of surface layer; Continuity of tissue; Thickness of repaired tissue; Union with host tissue; Cell density, Survival rate; Cartilage cell clustering ratio; Host tissue metamorphism.

O'Driscoll score related to bone layer reconstruction: Surface; Matrix; Exposure of subchondral bone; Alignment of subchondral bone; Biological union (integration) of bone; Bone infiltration into defect region; Cartilage calcification (tidemark formation); Cell form; Cell distribution; Survival rate of cell population; Exposure of subchondral bone.

<Manufacturing method of composite tissue for therapy application>

In one aspect, the present invention provides a method for producing a composite tissue of the present invention, comprising positioning the three-dimensional synthetic tissue and the artificial bone so that the three-dimensional synthetic tissue and the artificial bone are in contact. The method of the present invention is advantageous in that a synthetic tissue, when positioned on an artificial bone, would immediately adhere thereto and become inseparable, whereby an inconvenient step as in a conventional composite tissue is not required. It is understood that any form as described in (Induced three-dimensional synthetic tissue), (Composite tissue of induced three-dimensional synthetic tissue and artificial bone) and (Production of three-dimensional synthetic tissue) and (Manufacturing kit of a composite tissue for therapeutic applications) and the like described below can be used herein as a three-dimensional synthetic tissue and artificial bone. It is also understood that any form as described in (Composite tissue of induced three-dimensional synthetic tissue and artificial bone) can be used in the treatment or prevention of a disease, disorder, or condition associated with an osteochondral defect.

(Production of three-dimensional synthetic tissue)

In one aspect, the present invention is a method for producing the induced three-dimensional synthetic tissue of the present invention, the method comprising A) providing a cell selected from the group consisting of a myoblast, mesenchymal stem cell, adipocyte, synovial cell, and bone marrow cell, preferably a mesenchymal stem cell induced from a pluripotent stem cell or an equivalent cell thereof ; B) positioning the cell in a container containing a cell culture solution including a promoting agent selected from ascorbic acid, ascorbic acid 2-phosphate, or a derivative or salt thereof, wherein the container has a base with an area sufficient to accommodate a three-dimensional synthetic tissue having the desired size; C) culturing the cell in the container with the cell culture solution containing the agent for a period of time sufficient for forming the synthetic tissue having the desired size to convert the cell into a synthetic tissue; D) detaching the synthetic tissue from the container to elicit self-contraction by the synthetic tissue, wherein the self-contraction is performed in a condition where (a) at least one component selected from the group consisting of sugar, vitamins and amino acids and/or (b) a basic fibroblast growth factor (bFGF) is enriched for $\alpha$MEM; and E) adjusting a thickness of the synthetic tissue by a physical stimulus or a chemical stimulus to obtain a desired thickness. Herein, the cell culture solution used in the production method used in the present invention may be any medium which allows a cell of interest to grow. Examples of such a solution include DMEM, MEM, F12, DME, RPMI1640, MCDB104, 199, MCDB153, L15, SkBM, Basal medium and the like which are suitably supplemented with glucose, FBS (fetal bovine serum) or human serum, antibiotics (penicillin, streptomycin, etc.). However, step D) is preferably performed in a condition where bFGFs are added to αMEM or in a condition of DMEM, but not limited to such conditions. It is understood that any form described in (Induced three-dimensional synthetic tissue) herein can be employed as the embodiment that could be employed in this method. Though not wishing to be bound by any theory, as exemplified in the Examples, the difference in the composition of DMEM and αMEM is such that the former contains 4.5 times the amount of glucose, about 4 times the amount of vitamins, and about 2 times the amount of amino acids relative to the latter. Meanwhile, the latter contains nucleic acids that are not contained in the former. Synthesis of extracellular matrix proteins by cells is extremely important for the creation of a TEC. An ES-TEC created with nutritionally superior DMEM exhibited early chondrogenesis after a biological implantation. In contrast, with αMEM, which is a growth medium optimized for ES-MSCs, a TEC formed in vitro did not exhibit chondrogenesis *in vivo.* It is demonstrated that a TEC exhibiting very strong chondrogenic ability *in vivo* can be created if a bFGF is added to αMEM. In view of the above, it is understood that the capability to differentiate into cartilage can be enhanced by enhancing a specific component.

When manufacturing a three-dimensional synthetic tissue used in the present invention, the period of time required for culture may be determined depending on the purpose of use of the composite tissue of the present invention. In order to detach and recover a cultured three-dimensional synthetic tissue from a support material, the cultured cell sheet or three-dimensional synthetic tissue can be detached directly or with a macromolecular membrane attached thereto. The three-dimensional synthetic tissue may be detached in culture solution in which cells have been cultured, or in other isotonic solutions. Such solutions may be selected depending on the purpose. When a monolayer cell sheet is prepared, examples of the macromolecular membrane, which is used when optionally attached to the cell sheet or three-dimensional synthetic tissue, include hydrophilized polyvinylidene difluoride (PVDF), polypropylene, polyethylene, cellulose and derivatives thereof, chitin, chitosan, collagen, paper (e.g. , Japan paper), urethane, and net-like or stockinette-like macromolecular materials (e.g., spandex). When a net-like or stockinette-like macromolecular material is employed, the composite tissue of the present invention has a higher degree of freedom, so that the contraction/relaxation function thereof can be further increased. A method for producing the three-dimensional structure of the present invention is not particularly limited. For example, the three-dimensional structure of the present invention can be manufactured by utilizing the above-described cultured cell sheet attached to a macromolecular membrane. A synthetic tissue that is substantially made of a cell and extracellular matrix derived from the cell cannot be manufactured by another method. Thus, the present invention is recognized as providing a composite tissue with a significant feature with respect to this point.

In a preferred embodiment, it is understood that placement of an extracellular matrix used in a three-dimensional synthetic tissue used in the present invention in a three-dimensional synthetic tissue used in the present invention can be readily achieved by a specific production method utilized in the present invention. However, it is understood that the production method is not limited thereto.

In order to detach and recover the three-dimensional synthetic tissue with a high yield from the cell culture support when manufacturing the three-dimensional synthetic tissue used in the present invention, the cell culture support is lightly tapped or shaken, or the medium is stirred with a pipette. These procedures may be performed alone or in combination. In addition, the three-dimensional synthetic tissue may be detached and recovered by deforming the base of the culture container or rinsing the container with isotonic solution or the like as needed. By stretching the three-dimensional synthetic tissue in a specific direction after being detached from the base material, the three-dimensional synthetic tissue is provided with alignment. Stretching may be performed by using a tensile device (e.g., Tensilon), or simply forceps, but the stretching method is not particularly limited. By providing alignment, it is possible to confer directionality to the motion of the three-dimensional synthetic tissue itself. This, for example, allows the three-dimensional synthetic tissue to be overlaid in accordance with the motion of a specific organ. The three-dimensional synthetic tissue can thus be efficiently applied to organs.

The methods disclosed in Japanese Patent NO. 4522999 can be appropriately referred with regard to the methods for manufacturing a three-dimensional synthetic tissue used in the present invention. Although described in detail below, the present invention can utilize techniques besides those described below.

A (three-dimensional) synthetic tissue used in the present invention can be produced as follows. In summary, the producing method comprises the steps of: A) providing a cell; B) positioning the cell in a container containing a cell culture solution including an extracellular matrix synthesis promoting agent, wherein the container has a base with an area sufficient to accommodate a desired size of the synthetic tissue; and C) culturing the cell in the container for a period of time sufficient to form the synthetic tissue having the desired size.

Cells used herein are mesenchymal stem cells. Examples of mesenchymal stem cells used herein include adipose tissue-derived stem cells, bone marrow-derived stem cells, cells differentiated from ES cells, and cells differentiated from iPS.

The method for producing a three-dimensional synthetic tissue used in the present invention employs a cell culture solution containing an extracellular matrix synthesis promoting agent. Examples of such an extracellular matrix synthesis promoting agent include, but are not limited to, ascorbic acid or a derivative thereof, ascorbic acid 2-phosphate, L-ascorbic acid, and the like.

The container used in production method used in the present invention may be any container typically used in the art which has a base with an area sufficient to accommodate a desired size of a synthetic tissue. Examples of such a container include petri dishes, flasks, and mold containers, and preferably containers having a large area of the base (e.g., at least 1 cm$^2$). The material of the container may be any material including, but are not limited to, glass, plastic (e.g. , polystyrene and polycarbonate, etc.), and silicone.

In a preferable embodiment, the production method used in the present invention may comprise separating a produced (three-dimensional) synthetic tissue. As used herein, the term "separate" indicates that after a synthetic tissue of the present invention is formed in a container, the synthetic tissue is separated from the container. The separation can be achieved by, for example, physical means (e.g., pipetting of medium) and chemical means (addition of a substance). In the present invention, a synthetic tissue can be separated by providing a stimulus around the synthetic tissue by physical means or chemical means, but not by aggressive means (e.g., treatment with a protein degradation enzyme) to the synthetic tissue. Thus, it should be noted that the present invention achieves an effect of providing ease of handling, which cannot be conventionally achieved, and the resulting synthetic tissue is substantially intact, resulting in a high-performance implant.

In a preferable embodiment, the production method used in the present invention further comprises separating cells which construct a synthetic tissue. In a more preferable embodiment, the separating step can apply a stimulus for contracting a synthetic tissue, including a physical stimulus (e.g., pipetting). Such a physical stimulus is not directly applied to the produced synthetic tissue. This is a preferred feature of the present invention. This is because it is possible to suppress damage to the synthetic tissue by not directly applying a physical stimulus to a synthetic tissue. Alternatively, the separating step includes chemical means , such as adding an actin regulatory agent. Such an actin regulatory agent includes a chemical substance selected from the group consisting of actin depolymerizing agents and actin polymerizing agents. Examples of actin depolymerizing agents include Slingshot, cofilin, CAP (cyclase associated protein), AIP1 (actin-interacting-protein 1), ADF (actin depolymerizing factor), destrin, depactin, actophorin, cytochalasin, and NGF (nerve growth factor). Examples of actin polymerizing agents include RhoA, mDi, profilin, Rac1, IRSp53, WAVE2, ROCK, LIMkinase, cofilin, cdc42, N-WASP, Arp2/3, Drf3, Mena, LPA (lysophosphatidic acid), insulin, PDGFa, PDGFb, chemokine, and TGF-β. Though not wishing to be bound by any theory, these actin regulatory agents cause actomyocin-based cytoskeleton to contract or extend. It is believed that regulation of contraction and extension of a cell itself results in promoting or delaying a three-dimensional synthetic tissue itself from being separated from the base of a container.

In another embodiment, the production method utilized in of the present invention is characterized in the production from cells which are cultured in monolayer culture. Synthetic tissues with various thicknesses can be constructed as a result despite the cells being cultured in monolayer culture. This is deemed a significant effect with respect to conventional methods. For example, a thick tissue could not be constructed without using a multilayer structure when a temperature responsive sheet or the like is used. No other method can achieve a method for constructing a three-dimensional structure, which does not require feeder cells and can construct multilayer cells including ten or more layers. A typical cell implantation method which does not employ a scaffold is a cell sheet engineering technique utilizing a temperature sensitive culture dish in Non Patent Literature 27. The technique has won international recognition as an original technique. However, when using this cell sheet technique, a single sheet is often weak and requires modification such as layering sheets for obtaining the strength resistant to a surgical operation such as implantation.

A three-dimensional synthetic tissue used in a composite tissue of the present invention is a cell/matrix complex that does not require a temperature sensitive culture dish unlike the cell sheet technique. The cell/matrix complex is characterized in that cells and extracellular matrix are readily constructed into a multilayer tissue. There is no other technique that can produce a multilayer complex having 10 or more layers without using so-called feeder cells, such as rodent stroma cells, in approximately three weeks. By adjusting conditions for matrix production of material cells such as synovial cells, it is possible to create a complex having strength which allows surgical manipulation, such as holding or transferring the complex, without a special instrument. Therefore, the present invention is an original, ground-breaking technique for reliably and safely performing cell implantation.

In a preferable embodiment, the extracellular matrix synthesis promoting agents used in the production method utilized in the present invention include ascorbic acid 2-phosphate (see Hata R. , Senoo H. , J. Cell Physiol., 1989, 138(1): 8-16). In the present invention, addition of a certain amount or more of ascorbic acid 2-phosphate promotes production of an extracellular matrix, so that the resultant three-dimensional synthetic tissue is hardened for easy detachment. Thereafter, self-contraction is elicited by applying a stimulus for detachment. Hata et al. do not report that a tissue becomes strong and obtains a property of being readily detachable after adding such an ascorbic acid and culturing. Though not bound by any theory, one of the significant differences is that Hata et al. used a significantly different cell density. Hata et al. do not suggest an effect of making a tissue hard. Such an effect of the tissue being hardened, an effect of contraction, and an effect of the tissue becoming readily detachable are found in no other method. The synthetic tissue used in the composite tissue of the present invention can be recognized to be completely different from the synthetic tissue which has been manufactured conventionally at least on the point that it is produced through the process of hardening, contraction, detachment and the like. Contraction when the culture is detached and promotion in constructing a three-dimensional structure, a multilayer tissue, and the like are surprising effects. Enhancement in a capability to differentiate into cartilage by adjusting a condition upon contraction is found to be achieved in the present invention.

In a preferable embodiment, ascorbic acid 2-phosphate used in the production method utilized in the present invention typically has a concentration of at least about 0.01 mM, preferably at least about 0.05 mM, more preferably at least about 0.1 mM, even more preferably at least about 0.2 mM, still more preferably about 0.5 mM, and still even more preferably about 1.0 mM. Herein, any concentration of about 0.1 mM or higher may be employed. However, there may be a situation where a concentration of about 10 mM or lower is desired. In a certain preferable embodiment, the extracellular matrix synthesis promoting agent used in the production method utilized in the present invention includes ascorbic acid 2-phosphate or a salt thereof, and L-ascorbic acid or a salt thereof.

In a preferable embodiment, after the culturing step, the production method of the present invention further comprises D) detaching the synthetic tissue to elicit self-contraction. The detachment can be accelerated by applying a physical stimulus (e.g., application of physical stimulus (shear stress, pipetting, deformation of the container, etc.) to a corner of a container with a stick or the like). Self-contraction naturally occurs when a physical stimulus is applied after the detachment. When a chemical stimulus is applied, self-contraction and detachment occur simultaneously. By self-contraction, biological integration is accelerated, particularly in the three dimensional directions (direction perpendicular to the two-dimensional directions in the case of tissue on a sheet). Therefore, a synthetic tissue of the present invention may take a form of a three-dimensional structure due to being manufactured in such a manner. In a production method utilized in the present invention, sufficient period of time preferably means at least 3 days, though it varies depending on the application of a synthetic tissue of interest. An exemplary period of time is 3 to 7 days.

In another embodiment, the production method utilized in the present invention may further comprise causing a synthetic tissue to differentiate. This is because a synthetic tissue can have a form closer to that of a desired tissue by causing differentiation. Examples of such differentiation include, but are not limited to, differentiation into a bone and differentiation into cartilage. In a preferable embodiment, differentiation into a bone may be performed in a medium containing dexamethasone, $\beta$-glycerophosphate, and ascorbic acid 2-phosphate. More preferably, bone morphogenetic proteins (BMPs) are added. This is because the BMP-2, BMP-4, and BMP-7 promote osteogenesis.

In another embodiment, the production method utilized in the present invention is a step of causing differentiation a synthetic tissue. Examples of a form of differentiation include differentiation into cartilage. In the preferable embodiment, differentiation into cartilage may take place in a medium including pyruvic acid, dexamethasone, ascorbic acid 2-phosphate, insulin, transferrin, and selenite. More preferably, bone morphogenetic proteins (BMP-2, BMP-4, BMP-7, TGF-$\beta$1, or TGF-$\beta$3) are added. This is because such BMPs promote further differentiation into cartilage.

An important point in the production method utilized in the present invention is that it is possible to manufacture a tissue having pluripotency into various differentiated cells such as a bone and cartilage. Conventionally, differentiation into a cartilage tissue was difficult in other scaffold-free synthetic tissues. If a certain size is required, in any other method, it was necessary to coculture a tissue with a scaffold, construct a three-dimensional structure, and add a cartilage differentiation medium. Conventionally, scaffold-free differentiation into cartilage was difficult. The present invention enables differentiation into a cartilage in a synthetic tissue. This is a characteristic effect of the present invention which has not been demonstrated by a method other than the methods utilizing the present invention. In a cell therapy which aims to regenerate a tissue, a method for performing a treatment efficiently and safely by using a tissue with a sufficient size without a scaffold was difficult. The present invention is considered to achieve a significant effect with respect to this point. Particularly, the present invention is significant in that it freely allows manipulation of differentiated cells such as cartilage, which had been impossible conventionally. In methods other than the methods of the present invention, for example, cells can be aggregated in a pellet form while causing the cells to differentiate to obtain a tissue of about 2 mm$^3$. However, use of a scaffold was required for obtaining a larger tissue.

The differentiation step in the production method utilized in the present invention may be performed before or after providing the cells.

Primary culture cells can be used as cells used in the production method utilized in the present invention. However, the present invention is not limited thereto. Subcultured cells (e.g., three or more passages) can also be used. Preferably, it is advantageous, when subculture cells are used, that the cells have undergone four passages or more, more preferably 5 passages or more, and even more preferably 6 passages or more. It is believed that since the upper limit of cell density increases with an increase in the number of passages beyond a certain level, a denser synthetic tissue can be produced. However, the present invention is not limited thereto. It appears that a certain range of passages (e.g., 3 to 8 passages) are appropriate.

In the production method utilized in the present invention, cells are preferably provided at a cell density of 5.0 $\times$ 10$^4$/cm$^2$ or more. However, the present invention is not limited thereto. This is because a synthetic tissue with greater strength can be provided by sufficiently raising the cell density. However, it is understood that the lower limit of the cell density may be lower than the above-described density. It is also understood that those skilled in the art can define the lower limit based on the descriptions herein.

Cells that are used in the production method utilized in the present invention are mesenchymal stem cells induced from a pluripotent stem cell. Preferably, an induced mesenchymal stem cell derived from an ES cell or an iPS cell is advantageously used. These cells can be applied to, for example, a bone, a cartilage, a tendon, a ligament, a joint, or a meniscus., or the like

Another aspect of the production of a three-dimensional synthetic tissue utilized in the present invention can utilize a method for producing a three-dimensional synthetic tissue having a desired thickness. This method comprises: A) providing cells; B) positioning the cell in a container containing a cell culture solution including an extracellular matrix synthesis promoting agent (e.g., ascorbic acids, TGF-$\beta$1, or TGF-$\beta$3), wherein the container has a base with an area sufficient to accommodate a desired size of the three-dimensional synthetic tissue; C) culturing the cell in the container with the cell culture solution containing the agent for a period of time sufficient for forming the synthetic tissue having the desired size to convert the cell into a synthetic tissue; and D) adjusting a thickness of the three-dimensional synthetic tissue to obtain a desired thickness by a physical stimulus or a chemical stimulus, wherein the stimulus is applied in a condition where (a) at least one component selected from the group consisting of sugar, vitamins and amino acids and/or (b) a basic fibroblast growth factor (bFGF) is enriched for $\alpha$MEM. In this regard, the steps of providing a cell, positioning the cell, stimulating and converting the cell into a synthetic tissue or complex are explained in detail in the (Composite tissue of induced three-dimensional synthetic tissue and artificial bone) or the current section and the like herein, and it is understood that any embodiment can be used.

Next, examples of a physical or chemical stimulus to be used may include, but are not limited to, pipetting and use of actin interacting substance. Pipetting may be preferable because a pipette is readily operated and no harmful substance is produced. Alternatively, examples of the chemical stimulus to be used include actin depolymerizing factors and actin polymerizing factors. Examples of such an actin depolymerizing factor include ADF (actin depolymerizing factor), destrin, depactin, actophorin, cytochalasin, and NGF (nerve growth factor). Examples of the actin polymerizing factor include LPA (lysophosphatidic acid), insulin, PDGFa, PDGFb, chemokine, and TGF$\beta$. The polymerization or depolymerization of actin can be observed by checking the action on actin. It is possible to test any substance whether it has such an action. It is understood that a substance which is tested and identified in this manner can be used to achieve the desired thickness upon production of the synthetic tissue of the present invention. For example, in the present invention, adjustment of the desired thickness is achieved by adjusting the ratio of actin depolymerizing factors to actin polymerizing factors.

(Manufacturing kit of composite tissue for therapeutic applications)

In one aspect, the present invention provides a kit for treating or preventing a disease, disorder, or condition associated with an osteochondral defect, comprising a three-dimensional synthetic tissue and an artificial bone of the present invention. It is understood that any form as described in (Induced three-dimensional synthetic tissue), (Composite tissue of induced three-dimensional synthetic tissue and artificial bone) or (Production of three-dimensional synthetic tissue) can be used as the induced three-dimensional synthetic tissue and artificial bone. It is understood that any form as described in (Composite tissue of induced three-dimensional synthetic tissue and art if icial bone) can be used in the treatment or prevention of a disease, disorder or condition associated with an osteochondral defect.

In another aspect, a kit of the present invention can comprise a cell culture composition for producing an induced three-dimensional synthetic tissue from a pluripotent stem cell instead of the three-dimensional synthetic tissue itself, i.e. , the present invention is a kit for treating or preventing a disease, disorder, or condition associated with an osteochondral defect, comprising a cell culture composition for producing an induced three-dimensional synthetic tissue and an artificial bone. This is because it is possible to produce a three-dimensional synthetic tissue by using a cell culture composition to attach an artificial bone to the tissue with a kit of the present invention. The cell culture composition contains a component (e.g., commercially available medium) for maint aining or growing a cell, and an extracellular matrix synthesis promoting agent. Such an extracellular matrix synthesis promoting agent has been described in detail in the above description for a production method. Therefore, the extracellular matrix synthesis promoting agent includes ascorbic acid or a derivative thereof (e.g., TGF-$\beta$1, TGF-$\beta$3, ascorbic acid 1-phosphate or a salt thereof, ascorbic acid 2-phosphate or a salt thereof, or L-ascorbic acid or a salt thereof). The culture composition of the present invention contains ascorbic acid 2-phosphate or a salt thereof at a concentration of at least 0.1 mM. Alternatively, in the case of a condensed culture composition, the condensed culture composition contains ascorbic acid 2-phosphate or a salt thereof such that the concentration would be at least 0.1 mM at preparation. It appears that the effect of ascorbic acids barely changes at 0.1 mM or greater. Thus, 0.1 mM can be considered to be sufficient. For TGF-$\beta$1 and TGF-$\beta$3, 1 ng/ml or more, or representatively 10 ng/ml, may be sufficient. The present invention may provide a composition for producing an induced three-dimensional synthetic tissue, comprising such an extracellular matrix synthesis promoting agent.

An extracellular matrix synthesis promoting agent used in a cell culture composition used in the present invention includes ascorbic acid 2-phosphate (seeHataR. , SenooH., J. Cell Physiol . , 1989, 138(1):8-16). In the present invention, by adding at least a predetermined amount of ascorbic acid 2-phosphate, the production of an extracellular matrix is promoted. As a result, the resultant synthetic tissue or complex is hardened, and therefore, becomes readily detachable. Thereafter, the tissue undergoes self-contraction in response to a stimulus for detachment. Hata et al. do not report that the culture supplemented with such ascorbic acid causes the tissue to harden and thus confers to the tissue a property of being readily detachable. Though not bound by any theory, a significant difference between the present invention and Hata et al. is in cell density used. Also, Hata et al. do not suggest the effect of hardening. In terms of such an effect of hardening and contraction and being readily detachable, the synthetic tissue produced by a kit of the present invention can be considered completely different from conventionally-manufactured synthetic tissues at least in that the synthetic tissue produced by a kit of the present invention is produced via the procedures of hardening, contraction and detachment.

In a preferable embodiment, ascorbic acid 2-phosphate used in a kit of the present invention is typically present at a concentration of at least 0.01 mM, preferably at least 0.05 mM, more preferably at least 0.1 mM, even more preferably at least 0.2 mM, and still more preferably at least 0.5 mM. Still even more preferably, the minimum concentration may be 1.0 mM.

In one embodiment of the present invention, the cell density is, but is not particularly limited to, $5 \times 10^4$ to $5 \times 10^6$ cells per 1 $cm^2$. These conditions may be applied, for example, to myoblasts. In this case, the extracellular matrix synthesis promoting agent is preferably provided as ascorbic acids at a concentration of at least 0.1 mM. This is because a thick synthetic tissue can be created. In this case, if the concentration is increased, a synthetic tissue having a dense extracellular matrix is produced. If the concentration is low, the amount of an extracellular matrix is decreased but the self-supporting ability is maintained.

(Cartilaginous/osteochondral regeneration application)

In another aspect, the present invention provides a composite tissue for regenerating a cartilage, comprising an induced three-dimensional synthetic tissue and an artificial bone. It is understood that any form as described in (Induced three-dimensional synthetic tissue), (Composite tissue of induced three-dimensional synthetic tissue and artificial bone) or (Production of three-dimensional synthetic tissue) can be used as the induced three-dimensional synthetic tissue and artificial bone. It is also understood that any form as described in (Composite tissue of induced three-dimensional synthetic tissue and artificial bone) can be used for cartilage regeneration as needed.

Cartilage regeneration is recognized as a significant effect that could not be achieved by convention therapeutic methods in that a synthetic tissue alone can provide effective treatment.

In another aspect, the present invention provides a composite tissue for regenerating an osteochondral system, comprising an induced three-dimensional synthetic tissue and an artificial bone. It is understood that any form as described in (Induced three-dimensional synthetic tissue), (Composite tissue of induced three-dimensional synthetic tissue and artificial bone) or (Production of three-dimensional synthetic tissue) can be used as the induced three-dimensional synthetic tissue and artificial bone. It is also understood that any form described in (Composite tissue of induced three-dimensional synthetic tissue and artificial bone) can be used for osteochondral system regeneration as needed.

An example of a significant point of osteochondral system regeneration is that implantation of a complex is better in comparison to that of a synthetic tissue alone. Further, it is preferable that an artificial bone remains a little below the osteochondral boundary surface. Though not wishing to be bound by any theory, this is because it was found that regeneration of a subchondral bone is promoted in the space between the osteochondral boundary and the surface of an artificial bone to yield significant therapeutic results in biological integration of cartilaginous portions.

In another aspect, the present invention provides a composite tissue for regenerating a subchondral bone, comprising an induced three-dimensional synthetic tissue and an artificial bone. It is understood that any form as described in (Composite tissue of induced three-dimensional synthetic tissue and artificial bone) or (Production of three-dimensional synthetic tissue) can be used as the induced three-dimensional synthetic tissue and artificial bone. It is also understood that any form described in (Composite tissue of induced three-dimensional synthetic tissue and artificial bone) can be used for subchondral bone regeneration as needed.

Preferably, one of the features of the regeneration of a cartilaginous or osteochondral system of the present invention is the cartilage integrating with an existing cartilage after regeneration. In a conventional therapy with only an artificial bone, integration with a cartilage after regeneration does not occur, while regeneration occurs in a form of a population of substantially different tissue fragments (bone fragments or cartilage fragments) . Thus, the present invention is significant in that the regeneration for substantial recovery to a condition prior to a defect is possible. In addition, the ability to suppress ossification and early regeneration are also significant effects.

With regard to subchondral bone regeneration, it should be noted that a subchondral bone is efficiently formed directly above an artificial bone by implantation of the artificial bone and differentiation of undifferentiated cells into a cartilage in a synthetic tissue is subsequently promoted. Though not wishing to be bound by any theory, this is one of the significant points because it is known that formation of a cartilage bone has a significantly correlation with the extent of cartilage tissue formation within a synthetic tissue.

(Therapy using composite tissue)

In another aspect, the present invention provides a method of treating or preventing a disease, disorder, or condition associated with an osteochondral defect. The method comprises the steps of: A) positioning a composite tissue of the present invention to replace or cover the defect; and B) holding the synthetic tissue or complex for a time sufficient for biological integration of the portion and the synthetic tissue of complex. It is understood that any form as described in (Composite tissue of induced three-dimensional synthetic tissue and artificial bone), (Production of three-dimensional synthetic tissue) or the like herein can be used as a "composite tissue" used in the present invention. It is also understood that any form as described in (Composite tissue of induced three-dimensional synthetic tissue and artificial bone) or the like herein can be used for a disease, disorder, or condition associated with an osteochondral defect. Here, to position a portion for replacement typically means to perform debridement or curetage of an affected portion as needed and then position a composite tissue of the present invention on the affected portion, and to allow it to stand so as to promote replacement. An objective of such replacement is to fill the replaced portion with cells. Techniques known in the art can be combined and used. The step of positioning a synthetic tissue to cover a portion can be carried out using a technique well known in the art. The sufficient time varies depending on the combination of the portion and synthetic tissue, but can be readily determined as appropriate by those skilled in the art depending on the combination. Examples of such a time include, but are not limited to, 1 week, 2 weeks, 1 month, 2 months, 3 months, 6 months, 1 year, and the like after an operation. In the present invention, a synthetic tissue preferably comprises substantially only cell (s) and material(s) derived from the cell. Therefore, there is no particular material which needs to be extracted after an operation. The lower limit of the sufficient time is not particularly important for this reason. Thus, it can be said that a longer time is preferable in this case. If the time is extremely long, it can be said that reinforcement is substantially completed. Therefore, there is no particularly need for a limit. The synthetic tissue of the present invention is also characterized in that it is easily handled, is not destroyed during an actual treatment, and facilitates a surgery due to its self-supporting ability.

Alternatively, the above-described portion may include a bone or cartilage. Examples of such portions include, but are not limited to, meniscus, ligament, tendon, and the like. The method of the present invention may be utilized for treating, preventing or reinforcing a disease, disorder, or condition of a bone, cartilage, ligament, tendon, meniscus, or the like.

In another preferred embodiment, the reinforcement method of the present invention includes biological integration (e.g. , interconnection of extracellular matrices, electrical integration, and intracellular signal transduction) that is mentioned with respect to a composite tissue of the present invention. The biological integration is preferably provided in all three dimensional directions.

In another preferred embodiment, the method of the present invention further comprises culturing a cell in the presence of an extracellular matrix synthesis promoting agent to form a composite tissue of the present invention. Such an implantation/regeneration technique which comprises the step of culturing a cell in the presence of an extracellular matrix synthesis promoting agent had not been provided by conventional methods. The method enables a therapy for diseases (e.g., cartilage injury or intractable bone fracture), which was considered impossible by conventional therapies.

In a preferred embodiment, in the method of the present invention, a cell used in the composite tissue of the present invention is derived from an animal to which the composite tissue is to be implanted (i. e. , an autologous cell). Since an autologous cell can be sufficiently used, adverse side effects such as immune rejection reactions can be avoided especially in the induced three-dimensional synthetic tissue of the present invention.

Examples of targets of the therapeutic methods of the present invention include: cartilage full thickness injury, cartilage partial injury; osteochondral injury; osteonecrosis; osteoarthritis; meniscus injury; ligament injury (chronic injury, degenerative tear, biological augmentation for reconstruction surgery, etc.); tendon (including Achilles heel) repair (chronic injury, degenerative tear, biological augmentation for reconstruction surgery, etc.); rotator cuff repair (particularly, chronic injury, degenerative tear, etc.); delayed union; nonunion; faulty union of a bone and body inserts such as artificial joint, and skeletal muscle repair/regeneration, and the like.

For some organs, it is said that it is difficult to radically treat a specific disease, disorder, or condition thereof for diseases caused by an osteochondral defect or the like. However, the present invention provides the above-described effect, thereby making it possible a treatment which was considered impossible by conventional techniques . It has been revealed that the present invention can be applied to radical therapy. Therefore, the present invention has usefulness that could not be achieved by conventional pharmaceutical products.

Improvement in the condition by the method of the present invention can be determined in accordance with the function of the portion to be treated. In the case of a bone, for example, an improvement in the condition can be determined by measuring its strength or by evaluating bone marrow and/or a bone quality with MRI. If a cartilage or meniscus should be treated, a surface of a joint can be observed by an arthroscopy. Further, it is possible to determine an improvement in the condition by performing a biomechanical inspection under arthroscopy. It is also possible to determine an improvement in the condition by examining the status of repair by using MRI. For ligaments, improvement in the condition can be determined by examining the presence of lability by a joint stability inspection. Further, an improvement of the condition can be determined by examining a continuousness of a tissue by an MRI. In the case of any tissue, it is possible to determine whether the condition is improved by performing a biopsy of the tissue and making a histological evaluation.

In a preferred embodiment, the treatment treats, prevents, or enhances a disease, disorder, or condition of a bone, cartilage, ligament, tendon, or meniscus. Preferably, the composite tissue has a self-supporting ability. For such composite tissues, those skilled in the art can use a composite tissue of any form described above herein or a variant thereof.

(Combined therapy)

In another aspect, the present invention provides a regeneration therapy which uses a cytokine, such as BMP (e.g. , BMP-2, BMP-4, or BMP-7 and the like), TGF-β1, TGF-β3, HGF, FGF, IGF, or the like in combination with a composite tissue of the present invention. It is understood that any form as described in the section of (Composite tissue of induced three-dimensional synthetic tissue and artificial bone) or the like herein can be used as a composite tissue or the like to be used.

Some cytokines used in the present invention are already commercially available (e.g. , BMP (Astellas Pharma Inc.), bFGF2 (Kaken Pharmaceutical), TGF-β1 (R&D for research), IGF(Astellas Pharma Inc.), and HGF-101 (Toyo Boseki)). However, cytokines prepared by various methods can be used if they are purified to an extent which allows them to be used as a medicament. Certain cytokines can be obtained as follows: primary cultured cells or an established cell line capable of producing the cytokine is cultured; and the cytokine is separated from the culture supernatant or the like, followed by purification. Alternatively, a gene encoding the cytokine is incorporated into an appropriate vector by a genetic engineering technique; the vector is inserted into an appropriate host to transform the host; a recombinant cytokine of interest is obtained from the supernatant of the transformed host culture (see, Nature, 342, 440(1989); Japanese Laid-Open Publication No. 5-111383; Biochem-Biophys. Res. Commun., 163, 967 (1989), etc.). The above-described host cell is not particularly limited and examples thereof include various host cells conventionally used in genetic engineering techniques, such as, *Escherichia coli,* yeast, and animal cells. The cytokine obtained in such a manner may have one or more amino acid substitutions, deletions and/or additions in the amino acid sequence as long as it has substantially the same action as that of naturally-occurring cytokine. Examples of a method for introducing the cytokine into patients in the present invention include a Sendai virus (HVJ) liposome method with high safety and efficiency (Molecular Medicine, 30, 1440-1448(1993); Jikken Igaku (Experimental Medicine), 12, 1822-1826 (1994)), an electrical gene introduction method, a shotgun gene introduction method, and an ultrasonic gene introduction method. In another preferable embodiment, the above-described cytokines can be administered in a form of proteins.

Hereinafter, the present invention will be described byway of examples. Examples described below are provided only for illustrative purposes. Accordingly, the scope of the present invention is not limited except as by the appended claims.

[Examples]

In the present Examples, all procedures in this study were carried out in accordance with the Declaration of Helsinki. When applicable, experiments were conducted while handling animals in compliance with the rules set forth by Osaka University.

(Production Example 1: Production of three-dimensional synthetic tissue using synovial cells)

In this example disclosed below, a three-dimensional synthetic tissue was produced by using various synovial cells used as a comparative example.

<Preparation of cells>

Synovial cells were collected from a knee joint of a pig (LWD ternary hybrid, 2-3 months old upon removal of cells), followed by treatment with collagenase. The cells were cultured and subcultured in a 10% fetal bovine serum+High Glucose-DMEM medium (fetal bovine serum available from HyClone, DMEM was obtained from GIBCO). It has been reported that 10th passage synovial cells still have pluripotency. Although cells of 10 or less passages were used in this production example, it is understood that cells of more than 10 passages may be used depending on the application. Autotransplantation was performed for actual human implantation, but it was necessary to secure a sufficient number of cells and to culture the cells for a short period of time so as to reduce the risk of infection or the like.

Considering the above points , cells of various passages were used. Actually, primary culture cells, first passage cells, second passage cells, third passage cells, fourth passage cells, fifth passage cells, sixth passage cells, eighth passage cells, and tenth passage cells were used in experiments. These cells were used for use of synthetic tissues.

<Preparation of synthetic tissue>

Synovial cells ($4.0 \times 10^5$) were cultured in 0.1-0.2 ml/cm$^2$ of 10% FBS-DMEM medium in a 35-mm dish, 60-mm dish, 100-mm dish, 150-mm dish, 500-m dish, 6-well culture dish, 12-well culture dish, or 2 4-well culture dish (BD Biosciences, cell culture dish and multiwell cell culture plate). In this case, ascorbic acid was added. The dishes, the ascorbic acid and cell concentrations are described below: *Dishes: BD Biosciences, cell culture dishes and multiwell cell culture plates; *Ascorbic acid 2-phosphate: 0 mM, 0.1 mM, 0.5 mM, 1 mM, 2 mM, and 5 mM; *The number of cells: $5 \times 104$ cells/cm$^2$, $1 \times 10^5$ cells/cm$^2$, $2.5 \times 10^5$ cells/cm$^2$, $4.0 \times 10^5$ cells/cm$^2$, $5 \times 10^5$ cells/cm$^2$, $7.5 \times 10^5$ cells/cm$^2$, $1 \times 10^6$ cells/cm$^2$, $5 \times 10^6$ cells/cm$^2$, and $1 \times 10^7$ cells/cm$^2$

Medium was exchanged twice a week until the end of a predetermined culture period. At the end of the culture period, a cell sheet was detached from the dish by pipetting circumferentially around the dish using a 100-$\mu$l pipette. After detachment, the cell sheet was made as flat as possible by lightly shaking the dish. Thereafter, 1 ml of medium was added to completely suspend the cell sheet. The cell sheet was allowed to stand for two hours, resulting in the contraction of the cell sheet into a three-dimensional form. In this manner, a synthetic tissue was obtained.

<Hematoxylin-Eosin (HE) Staining>

The acceptance or vanishment of support in cells was observed by HE staining. The procedure is described below. A sample was optionally deparaffinized (e.g., with pure ethanol) and washed with water. The sample was immersed in Omni' s hematoxylin for 10 min. Thereafter, the sample was washed with running water and then the sample was subjected to color development with ammonia water for 30 sec. Thereafter, the sample was washed with running water for 5 min and stained with eosin hydrochloride solution (10 x diluent) for 2 min, followed by dehydration, clearing, and mounting.

(Various extracellular matrix)

1. Make 5 $\mu$m thick sections from frozen stock.
2. Fix sections in acetone at -20°C for 5-10 mins (paraffin blocks should be deparaffinized and rehydrated).
3. Block endogenous peroxide activity in 0.3% $H_2O_2$ in methanol for 20 mins at RT (1 ml 30% $H_2O_2$ + 99 ml methanol).
4. Wash with PBS ($3 \times 5$ mins).
5. Incubate with a primary monoclonal antibody (a mouse or rabbit antibody against various extracellular matrices) in a moist chamber at 4°C overnight (1 $\mu$l antibody + 200 $\mu$l PBS per slide).
6. Next day, wash with PBS ($3 \times 5$ mins).
7. Apply anti mouse and anti-rabbit no. 1 Biotynalated link for 30 mins to 1 hr at RT (apply 3 drops directly on slide).
8. Wash with PBS ($3 \times 5$ mins).
9. Apply Streptavidin HRP no. 2 for LSAB and soak for 10-15 mins.
10. Wash with PBS ($3 \times 5$ mins).
11. Apply DAB (5 ml DAB+5 $\mu$l $H_2O_2$).
12. Observe under microscope for brownish color.
13. Dip in water for 5 mins.
14. Apply HE for 30 sec-1 min.
15. Wash several times.
16. Wash once with ion exchange water.
17. Wash for 1 min with 80% ethanol.
18. Wash for 1 min with 90% ethanol.
19. Wash for 1 min with 100% ethanol (3 times).
20. Wash for 1 min with Xylene (3 times) and apply coverslip.
21. Examine color development.

As a result, when ascorbic acid 2-phosphate was added as an extracellular matrix synthesis promoting agent, only a small amount of multilayer structure of the cells was observed. On the other hand, the cells were observed to form a multilayer structure and promoted to form a three-dimensional structure by detaching the sheet-like cells from the base of the culture dish and allowing the cells to self-contract. A large tissue without a hole was also produced when synovial cells were used. This tissue was thick and rich in extracellular matrix. When synthetic tissues with ascorbic acid 2 -phosphate concentrations of 0 mM, 0.1 mM, 1 mM and 5 mM were observed, it could be seen that the formation of an extracellular matrix was promoted when ascorbic acid 2-phosphate was added at a concentration of 0.1 mM or more. If synthetic tissues on Day 3, 7, 14, and 21 of culture were observed, it could be seen that the tissue was already so rigid that it can be detached after 3 days of culture. As the number of culture days is increased, the density of the extracellular matrix fluctuates and increases.

These were synthetic tissue detached from the base of a culture dish and self-contracted. The synthetic tissue was prepared in a sheet form. When the sheet was detached from the dish and was allowed to stand, the sheet self contracted into a three-dimensional structure. It could be seen from the tissue that a number of layers of cells were present in the tissue.

Next, various markers including extracellular matrices were stained.

When the result of staining extracellular matrices was studied, it could be seen that various extracellular matrices (collagen I, II, III, IV, fibronectin vitronectin, etc.) were present. When immunostaining was conducted, collagen I and III were strongly stained, while collagen II staining was limited to a portion. When strongly magnified, collagen was stained at a site slightly away from the nuclei, and collagen could be confirmed as an extracellular matrix. On the other hand, when fibronectin and vitronectin, which are deemed important cell adhesion molecules, were strongly magnified, it could be seen that unlike collagen, fibronectin and vitronectin were stained at a region adjacent to the nuclei while fibronectin and vitronectin were also present around the cells.

It appears that cells of at least 3 to 8 passages are preferable for production of a synthetic tissue, but cells with any number of passages can be used.

For comparison, an example is shown in which a normal tissue and a collagen sponge (CMI, Amgen, USA) are stained. When the normal tissue (normal synovial membrane tissue, tendon tissue, cartilage tissue, skin, and meniscus tissue) was compared to a commercially-available stained collagen sponge used as the comparative example, the conventional synthetic tissue was not stained with fibronectin or vitronectin. Therefore, the synthetic tissue is different from collagen sponge synthetic tissue. Existing collagen scaffolds do not contain adhesion agents fibronectin and vitronectin. The originality of the tissues of the present invention is clearly understood In view of this. Stains are not found in any extracellular matrix. When the synthetic tissue of this production example was compared with normal tissue, it is confirmed that manner of integration of the synthetic tissue is close to the natural integration.

Further, when the synthetic tissue was contacted with a filter paper to remove moisture, the filter adhered to the synthetic tissue where it was difficult to manually detach the synthetic tissue.

In order to determine the collagen concentration, the collagen content was measured. As a result, the amount of hydroxyproline clearly indicated that the product ion of collagen was significantly promoted when 0.1 mM of ascorbic acid 2-phosphate or more was added. The amount of produced collagen was substantially proportional to the time period of culture.

(Production Example 2: Production of three-dimensional synthetic tissue using cells from adipose-derived tissue)

Next, cells derived from adipose tissue were used to produce a synthetic tissue.

A) Cells were collected as follows.

1) A specimen was removed from the fat-pad of a knee joint.
2) The specimen was washed with PBS.
3) The specimen was cut into as many pieces as possible using scissors.
4) 10 ml of collagenase (0.1%) was added to the specimen, followed by shaking for one hour in a water bath at 37°C.
5) An equal amount of DMEM (supplement with 10% FBS) was added, followed by filtration using a 70 $\mu$l filter (available from Millipore or the like).
6) Cells which passed through the filter and residues which remained on the filter were placed and cultured in a 25 cm$^2$ flask (available from Falcon or the like) containing 5 ml of DMEM supplemented with 10% FBS.
7) Cells attached to the bottom of the flask (including mesenchymal stem cells) were removed and subjected to the production of a synthetic tissue as follows.

B) Production of synthetic tissue

Next, the adipose-derived cells were used to produce a synthetic tissue. The concentrations of ascorbic acid 2-phosphate were 0 mM (absent), 0.1 mM, 0.5 mM, 1.0 mM, and 5.0 mM. The synthetic tissue was produced in accordance with the above-described method of producing synovial cells (described in Production Example 1). Cells were inoculated at an initial concentration of $5\times10^4$ cells/cm$^2$. The cells were cultured for 14 days. A synthetic tissue was also produced from an adipose tissue-derived cell and such a tissue had fibronectin and vitronectin as much as the synovial cell-derived synthetic tissue. Collagen I and III were similarly expressed in abundance.

Ascorbic acid 2-phosphate 0 mM: tangent tensile modulus (Young's modulus) 0.28
Ascorbic acid 2-phosphate 1.0 mM: tangent tensile modulus (Young's modulus) 1.33

C) Implantation experiment

Next, the above-described synthetic tissue can be used to produce a composite tissue described in the following Example. It is demonstrated as a result that adipose-derived synthetic tissue has repairing capability similar to that of a composite tissue made of a three-dimensional synthetic tissue from synovial cells.

D) Differentiation induction of adipose-derived synthetic tissue into bone/cartilage

The synthetic tissue of a bone or cartilage made in this example can be induced to differentiate into a cartilage or a bone. The synthetic tissue was confirmed to have a positive reaction to Alizarin Red in an osteogenesis induction medium to verify osteogenesis. In a chondrogenesis induction experiment, a synthetic tissue was confirmed to differentiate by a stimulus due to chondrogenesis induction medium+BMP-2 into a cartilage-like tissue which was Alcian blue positive. Thus, it is confirmed that the adipose-derived synthetic tissue also has the ability to differentiate into a bone or a cartilage as with a synovial cell-derived synthetic tissue (see Japanese Patent No. 4522994).

(Production Example 3: Production example with human synovial cells)

Next, a synovial cell is collected from a patient having an injured meniscus to determine whether the synovial cell can be used to produce a synthetic tissue.

(Collection of synovial cell)

A human patient, who is diagnosed by an imaging technique as having a cartilage injury or meniscus injury, is subjected to arthroscopy under lumber anesthesia or general anesthesia. In this case, several tens of milligrams of synovial membrane are collected. The collected synovial membrane is transferred to a 50-ml centrifuge tube (manufactured by Falcon) and washed with phosphate buffered saline (PBS). Thereafter, the sample is transferred to a 10-cm diameter culture dish (Falcon) and is cut into small pieces using a sterilized blade. Thereafter, 10 ml of 0.1% collagenase (Sigma) is added to the cut pieces. The dish is shaken in a constant temperature bath at 37°C for 1 hour and 30 minutes. To the solution, 10 ml of medium (DMEM, Gibco) containing previously collected self-serum or bovine serum (FBS) is added to inactivate the collagenase, followed by centrifugation at 1500 rpm for 5 minutes to pellet the cells. Thereafter, 5 ml of the serum-containing medium is added again. The medium is passed through a 70-$\mu$l filter (Falcon). The collected cells are transferred to a 25 cm$^2$ flask (Falcon), followed by culture in a CO$_2$ incubator maintained at 37°C.

(Subculture of synovial cell)

During primary culture, a medium is exchanged twice a week. When cells reach confluence, the cells are subcultured. For initial subculture, the medium is suctioned and thereafter the cells are washed with PBS. Trypsin-EDTA (0.25%Trypsin-EDTA (1x) , catalog number: 25200-056 100mL (one bottle) or 25200-114 20x100 mL, Gibco) is added to the cells which are in turn allowed to stand for 5 minutes. Thereafter, a serum-containing medium is added and the resultant mixture is transferred to a 50-ml centrifuge tube (Falcon), followed by centrifugation at 1500 rpm for 5 minutes. Thereafter, 15 ml of the serum-containing medium is added to the pellet. The cells are placed in a 150-cm$^2$ culture dish (Falcon). Subsequent subculture is performed so that the cell ratio is 1:3. The same procedure is repeated up to 4 to 5 passages.

(Production of synthetic tissue)

The synovial cell of 4 to 5 passages is treated with trypsin-EDTA. The synovial cells ($4.0\times10^6$) are dispersed in 2 ml of medium containing 0.2 mM ascorbic acid 2-phosphate on a 35-ml culture dish (Falcon), where the cells are cultured in a CO$_2$ incubator maintained at 37°C for 7 days. As a result, a culture cell-extracellular matrix complex is formed. The complex is mechanically detached from the culture dish by pipetting the periphery thereof two or more hours before an implantation operation. After detachment, the culture cell-extracellular matrix complex contracts into a three-dimensional tissue having a diameter of about 15 mm and a thickness of about 0.1 mm.

(Production Example 4: Production of synthetic tissue from human adipocyte)

A collection-intended site (e.g., around a knee joint) from a patient under local anesthesia is resected. Several tens of milligrams of adipocytes are collected. For example, the collected adipocytes are treated in a manner similar to that of the synovial cells as in the above-described production example. As a result, a three-dimensional synthetic tissue can be produced.

(Production Example 5: Production of three-dimensional synthetic tissue (TEC) with synovial MSCs)

(Collection of synovial tissue and isolation of cells)

All animal experimentations were approved by Osaka University Faculty of Medicine animal experiment facility. A synovial membrane of rabbits was aseptically collected from a knee joint of a rabbit with a mature (24 weeks of age) skeleton within 12 hours post mortem. The protocol for cell isolation was substantially the same as that reported previously with regard to isolation of MSCs derived from human synovial membrane [Ando W, Tateishi K, Katakai D, Hart DA, Higuchi C, Nakata K, et al., Tissue Eng Part A 2008; 14:2041-2049]. Briefly stated, synovial membrane samples were rinsed with phosphate buffered saline (PBS), cut into small pieces, and treated with 0.4% collagenase XI (Sigma-Aldrich, St. Louis, MO, USA) for 2 hours at 37° C. After the collagenase was neutralized in a growth medium containing a high-glucose Dulbecco's modified eagle's medium (HG-DMEM; Wako, Osaka, Japan) added with 10% fetal bovine serum (FBS; HyClone, Logan, UT, USA) and 1% penicillin/streptomycin (Gibco BRL, Life Technologies Inc., Carlsbad, CA, USA), cells were collected by centrifugation, washed with PBS, resuspended in a growth medium, and plated on a culture dish. The characteristics of rabbit mesenchymal stem cells were similar to human synovial membrane-derived MSCs in terms of form, growth characteristics, and pluripotency (into bone system, cartilage system and fat system) [Ando W, Tateishi K, Katakai D, Hart DA, Higuchi C, Nakata K, et al. , Tissue Eng Part A 2008; 14:2041-2049; Tateishi K, Higuchi C, Ando W, Nakata K, Hashimoto J, Hart DA, et al. , Osteoarthritis Cartilage 2007; 15: 709-718]. For cell growth, these cells were grown in a growth medium at 37°C in a humidified atmosphere of 5% $CO_2$. The medium was changed once a week. When cells reached confluence 7-10 days after the primary culture, the cells were washed twice with PBS, recovered from treatment with trypsin-EDTA (0.25% trypsin and 1mM EDTA: Gibco) BRL, Life Technologies Inc., Carlsbad, CA, USA), and diluted so that the cell concentration would be 1:3. The cells were again plated. When culture cells substantially reached confluence, the cells were similarly diluted to a 1: 3 ratio to continue passage of cells. The present application used cells of 3-7 passages.

(Production of somatic three-dimensional tissue (TEC))

Synovial membrane MSCs were plated on a 6-well plate (9.6 cm$^2$) at a density of $4.0 \times 10^5$ cells/cm$^2$ in a growth medium containing 0.2 mM of ascorbate-2-phosphate (Asc-2P), which is the optimal concentration from previous studies [AndoW, Tateishi K, Katakai D, Hart DA, Higuchi C, Nakata K, et al., Tissue Eng Part A 2008; 14:2041-2049; Ando W, Tateishi K, Hart DA, Katakai D, Tanaka Y, Nakata K, et al. , Biomaterials 2007; 28:5462-5470; Shimomura K, Ando W, Tateishi K, Nansai R, Fujie H, Hart DA, et al., Biomaterials 2010; 31:8004-8011]. The cells reached confluence within a day. Furthermore, after continuous culturing for 7-14 days, a complex of culture cells and ECM synthesized by said cells was detached from the lower layer by applying shearing stress by using a light pipette. The detached monolayer complex was maintained in a suspension to allow formation of a three-dimensional structure by self-tissue contraction. This tissue was called a somatic (also called natural) three-dimensional TEC that is not dependent on a scaffold (also called scaffold-free).

(Production Example 6: Production of composite tissue (hybrid graft) consisting of somatic three-dimensional synthetic tissue and artificial bone)

Synthetic HA (diameter 5 mm, height 4 mm (NEOBONE®; MMT Co. Ltd., Osaka, Japan)) with an interconnected, porous structure was used as an artificial bone. A TEC was detached from a culture dish immediately prior to an implant surgery and integrated with the artificial bone without using an adhesive to produce a bone/cartilage hybrid (composite tissue) . Once a TEC integrates with an artificial bone, the integration is so strong that separation would be difficult.

(Implantation Example: Implantation of hybrid graft (composite tissue) into osteochondral defect)

Implantation was performed in the following manner.

New Zealand white rabbits with a mature skeleton (24 weeks of age or older) were maintained in a cage while freely providing food and water. The rabbits were administered with anesthesia by an intravenous injection of 1 ml of pentobarbital [50 mg/ml (Nembutal®, Dainippon Pharmaceutical Co. Ltd., Osaka, Japan)] and 1ml of xylazine hydrochloride (25 mg/ml (Seractal®; Bayer, Germany)). The rabbits were shaved and disinfected. After covering the rabbits with a sterilized fabric, a straight 3 cm medial parapatellar incision was made on the right knee. The patella was moved outward to expose the femoral fossa. A joint osteochondral defect through the full thickness of 5 mm diameter and 6 mm depth was mechanically made in the femoral fossa of the right distal femur. A hybrid of a TEC and artificial bone was formed immediately prior to implantation. Then, a diphasic graft was implanted within the defect of the right knee of the rabbits (TEC group). In a control group, only an artificial bone was implanted in a defect of the right knee of the rabbits. The right limbs of all the animals were stabilized in a cast for 7 days. The rabbits were then euthanized under anesthesia at one month, 2 months, and 6 months after the surgical operation or after other suitable periods. The implantation site was secured for use in the subsequent paraffin section production and histological analysis. Other implantation sites were subjected to biodynamic tests. The present inventors prepared untreated left knees of rabbits as untreated normal controls for the biodynamic tests.

(Histological evaluation of repaired tissue)

For histological evaluation, tissues were fixed with 10% neutral buffer formalin, decalcified with K-CX (Falma, Tokyo, Japan) and embedded in paraffin to produce a 4 mm section. The section was stained with hematoxylin-eosin (H&E) and toluidine blue.

At one, two, and six months or other periods, histology of repaired tissue was evaluated with improved "O'Driscoll score" for cartilage and subchondral bones [O' Driscoll SW, Keeley FW, Salter RB., J Bone Joint Surg Am 1988; 70:595-606; Mrosek EH, Schagemann JC, Chung HW, Fitzsimmons JS, Yaszemski MJ, Mardones RM, et al., J Orthop Res 2010; 28:141-148; Olivos-Meza A, Fitzsimmons JS, Casper ME, Chen Q, An KN, Ruesink TJ, et al. , Osteoarthritis Cartilage 2010;18:1183-1191]. In the improved version, standard classification "toluidine blue stain" was changed to "Safranin O stain". For subchondral bone repair, new standard classifications "Cell form" and "Exposure of subchondral bone" were established. For "Cell form", normal subchondral repair has a score of 2, a tissue repaired with cartilage-like tissue mixed therein has a score of 1, and a tissue repaired with fibrous tissue mixed therein has a score of 0. For "Exposure of subchondral bone" , no exposure of a subchondral bone has a score of 2, exposure of subchondral bone in one side of the boundary between a repaired tissue and an adjacent cartilage has a score of 1, and exposure of a subchondral bone on both sides has a score of 0. The repaired tissue was divided into three sections for each 2mm width to form a central region and two boundary regions. Each of the regions was evaluated by the improved O' Driscoll score. The average of the three scores was the "overall evaluation". Furthermore, the score of the central region was evaluated as the "central region" and the average score of the two boundary regions was evaluated as the "boundary region". The standard classifications "Integration with adjacent cartilage", "Free of degeneration of adjacent cartilage", and "Exposure of subchondral bone" were only evaluated by "overall evaluation".

In order to quantify the degree of repair of a subchondral bone and cartilage, bone and cartilage formation ratios of the repaired tissue were calculated at one, two, and six months. For bone formation ratios, the length of repaired bone tissue was divided by the length of an artificial bone. The results are shown in percentages. The cartilage formation ratios were calculated by the same method. Furthermore, the correlation of the bone formation ratio and cartilage formation ratio was calculated.

(Biochemical test)

A cylindrical sample with a 4 mm diameter and 5 mm depth was extracted from implantation sites of the TEC group and the control group. Similarly, a cylindrical sample was extracted from the interchondylar center of femurs of an untreated normal knee. AFM (Nanoscope IIIa, Veeco Instruments, Santa Barbara, CA, USA) and silicone probe (spring constant: 0.06 N/m, DNP-S, Veeco Instruments, Santa Barbara, CA, USA) were used. Each sample was mounted on an AFM sample stage and dipped in a saline solution at room temperature. Microcompression tests were conducted on these samples. Furthermore, a surface image of a sample was obtained in a contact mode in a 30 $\mu$m $\times$ 30 $\mu$m scan region, and microcompression tests were then conducted with a scan rate of 0.3 Hz on the samples at a 5.12 $\mu$m pushing rate.

(Statistical analysis)

For statistical analysis, analysis of variance (ANOVA) was carried out. Comparative tests were run ex post facto for post-operation changes in overall histological score and bioengineering tests. Comparisons of other experimental parameters between the reference group and the TEC group were analyzed by the Mann-Whitney U-test. The correlation of bone and cartilage formation was calculated with Spearman's rank correlation coefficient. The results are represented by mean $\pm$ SD (standard deviation). The data was analyzed with JMP9 (SAS Institute, Cary, NC, USA). Statistical significance was set at $p<0.05$ for judgment.

(Results)

(Formation of composite tissue (hybrid) of somatic TEC with artificial bone)

A somatic TEC immediately integrated on an artificial bone block to form a complex with sufficient strength for surgical implantation.

(Example 1: Induction from embryonic stem cells (ES cells) to ES cell derived MSCs)

An ES cell colony was recovered with a CTK colony-dissociation solution (0.25% trypsin, 0.1% collagenase IV, 20% KSR, 1 mM $CaCl_2$, and phosphate buffered saline (PBS)) and washed with an ES cell culture medium (e.g., the following rabbit ESC medium). The colony was cultured in suspended culture for four days at a ratio of 100 colonies/ml with 10% FBS DMEM (Invitrogen). The formed embryoid body (EB) was then allowed to attach to a gelatin-coated culture dish. A basal medium (for the basal medium, 5g of powdered $\alpha$MEM (Invitrogen, GIBCO Cat#11900-024, lot#755272) and 1.1g of sodium bicarbonate are dissolved in 500 ml of distilled water, and each of 1% Antibiotic-Antimycotic, 100x (Invitrogen, 15240) and 10% FBS (lot #FTM33793, Hyclone, Thermo Scientific) is then added thereto) was cultured for 6 days under extremely low oxygen partial pressure (1% $O_2$, 5% $CO_2$) conditions. Nitrogen was used for adjustment and the atmospheric pressure was used as a culture condition. The embryoid body was cultured in a multi-gas incubator (MCO-5M, SANYO Electric Co. Ltd. , Osaka, Japan). The adhering cells (EB derived cells) were treated and washed with trypsin (Invitrogen). The cells were resuspended in the basal medium and cultured for three more days under extremely low oxygen partial pressure (1% $O_2$, 5% $CO_2$). After the grown cells were again treated with trypsin and subjected to limiting dilution, the cells were cultured for about 10 days under the same conditions. A single colony was recovered with a glass pipette as an ES-MSC clone.
The details thereof are described below.

Use and care of animals

Animals were used in accordance with the code of ethics set forth by the Osaka University (or another university). Each rabbits were maintained in individual cages while freely providing food and water. The animals were exposed to an artificially managed 14 hour: 10 hour bright and dark cycle. The temperatures were maintained at 20-25° C in a ventilated room. All treatments were conducted under anesthesia induced by an intravenous injection of 45 mg/kg pentobarbital sodium and a topical injection of 2% xylocaine with epinephrine.

Differentiation and culture of rabbit embryonic stem cells

A female Dutch belted rabbit (KITAYAMA LABES CO., LTD, Nagano) was intraperitoneally injected with pregnant mare serum gonadotropin (PMSG; Sumitomo Dainippon Pharma, Osaka) and human chorionic gonadotropin (hCG; Sumitomo Dainippon Pharma, Osaka) to induce superovulation, and allowed to mate with a male Dutch belted rabbit. Two days after mating, fertilized embryos in 4-8 cell stages were recovered from the fallopian tube, and then cultured in *vitro* to the expanded blastocyst stage.
For ESC differentiation, a blastocyst was transferred onto a mouse embryonic fibroblast (MEF) feeder cell treated with mitomycin C (90 minutes at 10 $\mu$g/ml in medium; Invitrogen Corporation, Carlsbad CA, USA) and cultured for 10 days in a rabbit ESC medium (rESM) comprising 20% knockout serum replacement (KSR; Invitrogen), Dulbecco's Modified Eagle's Medium (DMEM)/F12 (Invitrogen), 2mM L- glutamine, Wako, Tokyo), 1% nonessential amino acid (Invitrogen), 0.1 mM $\beta$-mercaptoethanol (Invitrogen) and 8 ng/ml human recombinant basic fibroblast growth factor (bFGF; Wako). These rESCs were subcultured with a CTK colony dissociation solution (0.25% trypsin, 0.1% collagenase IV, 20% KSR and 1 mM $CaCl_2$ in PBS) as previously reported (Suemori, H. et al., Biochem.Biophys.Res.Commun., 2006, 345:926-932). The multi-differentiating capability of these cells was studied in *vitro* and in *vivo.* For *in vitro* differentiation, an rESC colony was recovered with a CTK solution and washed once in a new rESM, after which the colony was transferred to and suspension cultured in DMEM (Invitrogen supplemented with 10% fetal bovine serum (FCS). Embryoid bodies (EB) formed from rESCs were recovered 6 days after the start of suspension culture. The embryoid bodies were again plated in a gelatin coated culture dish. EB derived cells were evaluated by immunofluorescent staining using a specific antibody after 6 days of culture.
The rESC differentiation capability was evaluated *in vivo* by a teratoma formation assay. A cell suspension

comprising $5 \times 10^6$ rESCs was subcutaneously injected into the femur of a severe combined immunodeficient (SCID) mouse. After 8 weeks from the cellular injection, teratoma was recovered and stained with hematoxylin-eosin to conduct a histological observation.

In order to observe the effect of an inhibitor against the pluripotency of rESCs, ESCs were subcultured on a matrigel (BD Falcon, Bedford, MA, USA) and cultured in an MEF-acclimated rESM. After 24 hours from the start of matrigel culture, a leukemia inhibitory factor (LIF; Millipore Billerica, MA, USA), 1 $\mu$M Janus kinase (JAK) inhibitor I (Merck, Darmstadt, Germany), 1 $\mu$M specific mitogen-activated protein kinase (MEK)/extracellular signal-regulated kinase (Erk) inhibitor PD0325901 (Stemgent, Cambridge, MA, USA) or 0.5 $\mu$M specific activin-like receptor 4/5/7 (ALK 4/5/7) inhibitor A83-01 (Stemgent) was added to the culture medium. The culture was recovered after 48 hours to analyze the pluripotency marker gene Nanog and POU5f 1 (pituitary gland-specific octamer transcription factor [OCT] Unc-86 domain family class 5 homeobox 1) by using quantitative RT-PCR (qRT-PCR).

In order to further demonstrate that these cells exhibited pluripotency from receiving stimulus, the present inventors treated rESCs with 0.25% trypsin (Invitrogen) and 0.04% EDTA (Sigma-Aldrich, St. Louis, MO, USA) (trypsin-EDTA) and seeded the dissociated rESCs on a gelatin-coated tissue culture dish, and cultured the rESCs for 24 hours in an rESM with or without 10 $\mu$M Rho-associated, coiled-coil containing protein kinase (ROCK) inhibitorY-2763 (Wako) toconductasingle-celldigestion assay. Cell death was investigated with western blot analysis on caspase-3, which is an important protein mediating apoptosis, by flow cytometry (fluorescence activated cell sorting; FACS) using necrotic cell marker propidium iodide (PI; BD Pharmingen, SanDiego, CA, USA). Three independent tests were run with qRT-PCR and FACS.

Establishment and culture of rabbit bone marrow mesenchymal stem cells (rBMMSC)

Control MSC lines were established from a rabbit bone marrow tissue. Stem cell lines were established in accordance with the previously disclosed protocol (Sekiya, I. et al., Stem Cells 2002, 20:530-541; Wang, G.et al., Proc.Natl.Acad.Sci.USA,2005, 102:186-191) with minor improvements. Briefly stated, the bone marrow cells were isolated from a male Japanese white (JW) rabbit (3.0 kg in weight) by washing the femur medullary cavity and tibia medullary cavity with phosphate buffered saline and plating the substance washed off on a 10 cm dish in a 10% FCS-$\alpha$ modified minimum essential medium ($\alpha$MEM). The non-adhering cells were washed off in PBS to further grow adhering cells three days after plating. Upon reaching confluence, the cells were dissociated with trypsin-EDTA, washed, diluted to 200 cells/35 mm plate, and cultured. The cells were determined to be positive for MSC markers Vimentin, CD29, and CD105 by western blot analysis prior to conducting further tests. The differentiation capability of the established rBMMSCs, when established by inductive differentiation into adipocytes, osteocytes, and chondrocytes, was comparable with that of standard MSCs (data not shown).

Low oxygen treatment of undifferentiated rESCs

Rabbit ESCs were cultured for 24 hours under normal oxygen pressure (20% $O_2$ + 5% $CO_2$) culture conditions on a matrigel (BD Falcon) in an MEF-acclimated rESM medium. The culture was then left standing for 24 hours after being transferred to one of 20% $O_2$ + 5% $CO_2$, 5% $O_2$ + 5% $CO_2$, or 1% $O_2$ + 5% $CO_2$. Balance of low oxygen atmosphere was maintained with nitrogen. All conditions were controlled by a multi-gas incubatorMCO-5M (SANYO Electric Co. Ltd., Osaka, Japan). The actual $O_2$ concentration was monitored throughout the entire culture period with a paperless recorder DX Advanced DX1000 (Yokogawa Electric Corporation) (data not shown).

Quantitative RT-PCR (qRT-PCR) analysis

A TRIzol reagent (Invitrogen) was used to extract total RNA. High Capacity cDNA Reverse Transcript ion Kit (Applied Biosystems, Foster City, CA, USA) was used for reverse transcription. QRT-PCR using total cDNA was conducted by using a rabbit specific primer (Table 1) and Perfect real-time SYBR green II (Takara Bio Inc.) in 40 cycles of 10 seconds at 95° C,followed by 5 seconds at 95° C and 30 seconds at 60° C in Thermal Cycler Dice® Real Time System (Takara Bio Inc. , Shiga) . Primers for estimate determining region Y-box2 (Sox2), Kruppel-like factor 4 (Klf4) andplatelet-derived growth factor receptor $\alpha$ (PDGRF$\alpha$) were designed in the sequence obtained first by TA-cloning and BigDye terminator sequencing (no data shown). Briefly stated, partial sequences of rabbit estimate Sox2, Klf4, and pDGFR$\alpha$ were amplified with Platinum Taq PCRx DNA polymerase (Invitrogen) and a universal primer designed for a sequence conserved in total cDNA of a mouse or a human derived from rabbit bone marrow tissues or rESCs. Amplicons were then ligated into a pGEM-T easy vector (Promega Corporation, Madison, WI, USA), transformed to E. coli JM109, purified with a QIAprep Spin Miniprep Kit (QIAGEN, Valencia, CA, USA), and sequenced by using Big Dye Terminator v3. 1 cycle sequencing ready reaction kit (Applied Biosystems) and an ABI3730 capillary sequencer (Applied Biosystems). To quantify the relative expression of each gene, the Ct (threshold value cycle) value was standardized with respect to

an internal standard ($\Delta$Ct = Ct$_{target}$ - Ct$_{internal}$ standard), and "$\Delta\Delta$Ct method ($\Delta\Delta$Ct = $\Delta$Ct$_{sample}$ - $\Delta$Ct$_{standard}$ substance) " (Dussault, A.A. et al., Biol.Proced.2006, Online8:1-10) was used for comparison with a standard substance (control). With regard to the internal standard gene, the present inventors used 28s rRNA for evaluation of pluripotency or mesenchymal gene expression in low oxygen experiments. In addition, glyceraldehyde-3-phosphate dehydrogenase(GAPDH)was used for observing the expression of chondrocyte specific genes in *vitro* and in *vivo*.
[Table 1]

### Table 1. Sequences of primers used in qRT-PCR

| Primer name | Primer sequence (5'→3') |
|---|---|
| Pou5f1 FOR<br>Pou5f1 REV | GATGACTCTGGGCTACACTC (SEQ ID NO: 9)<br>AGATGGTGGTTTGGCTGAAG (SEQ ID NO: 10) |
| Manog FOR<br>Nanog REV | TAGTGAAAACTCCCGACTCTG (SEQ ID NO: 11)<br>AGCTGGGTCTGGGAGAATAC (SEQ ID NO: 12) |
| Sox2 FOR<br>Sox2 REV | GAGTGGAAACCCTTGTGG (SEQ ID NO: 13)<br>ATTTATAACCCTGTGCTCCTCC (SEQ ID NO:14) |
| Klf4 FOR<br>Klf4 REV | AGGAGCCCAAGCCAAAGAG (SEQ ID NO: 15)<br>AATCGCAAGTGTGGGTG (SEQ ID NO: 16) |
| PDGFR α FOR<br>PDGFR α REV | AGTGAGCTGGCAGTACCCGG (SEQ ID NO: 17)<br>AGCCAGTGTTGTTCTCCTC (SEQ ID NO:18) |
| Vimentin FOR<br>Vimentin REV | TTGACAATGCTTCTTTGGC (SEQ ID NO: 19)<br>TTCCTCATCGTGCAGTTTC (SEQ ID NO: 20) |
| Aggrecan FOR<br>Aggrecan REV | TTGAGGACAGCGAGGCTAC (SEQ ID NO: 21)<br>GCCCGATAGTGGAACACA (SEQ ID NO:22) |
| Col2A1 FOR<br>Col2A1 REV | TCGTGGCCGAGATGGAGAG (SEQ ID NO: 23)<br>TCAAATCCTCCAGCCATTTG (SEQ ID NO: 24) |
| 28s rRNA FOR<br>28s rRNA REV | AGCAGAATTCACCAAGCGTTG (SEQ ID NO: 25)<br>TAACCTGTCTCAGGACGGTC (SEQ ID NO: 26) |
| Gapdh FOR<br>Gapdh REV | GTGAAGGTCGGAGTGAACG (SEQ ID NO: 27)<br>TAAAAGCAGCCCTGGTGAC (SEQ ID NO: 28) |

Immunofluorescence of cultured cells

Culture was fixed with Mildform 10N (Wako) for one hour at room temperature. The fixed cells were washed with PBS. Block Ace (Sumitomo Dainippon Pharma, Osaka) was used for one hour blocking. The cells were washed twice or more and incubated over night at 4° C with each primary antibody. The sample was washed twice and incubated with secondary antibodies (polyclonal antibody of rabbit, goat, donkey, or cow labeled with fluorescein isothiocyanate [FITC] or Texas Red; all were purchased from Santa Cruz Biotechnology) (Table 2). The samples were then stained in pairs with DAPI (1 μg/ml in PBS) and directly observed thereafter. Culture without a primary antibody was prepared as a negative control (no data shown).
[Table 2]

### Table 2. Primary antibodies used in immunofluorescence analysis and/or western blot analysis

| Antibody | Distributor | Dilution | Specific Band(kDa) |
|---|---|---|---|
| SSEA-4 (sc-21704) | Santacruz biotechnology | 1:100 in 10% Block-Ace containing PBS | - |
| POU5fl (sc-8628) | Santacruz biotechnology | 1/5,000 in 10% Block-Ace containing 0.2% Tween-TBS | 45 |
| CD140a (sc-338) | Santacruz biotechnology | 1/3,000 in10% Block-Ace containing 0.2% Tween-TBS | 170 |

(continued)

| Antibody | Distributor | Dilution | Specific Band(kDa) |
|---|---|---|---|
| P53 (sc-6243) | Santacruz biotechnology | 1/1,000 in Immuno-enhancer | 53 |
| Vimentin (sc-7558) | Santacruz biotechnology | 1/3.000 in 10% Block-Ace containing 0.2%Tween-TBS | 57 |
| **Neuron-specific class III β-tubulin** (TU-20) | Millipore | 1/2,000 in 10% Block-Ace containing 0.2% Tween-TBS | - |
| **Desmin** (E-2571) | Spring Bioscience | 1/3,000 in 10% Block-Ace containing 0.2% Tween-TBS | - |
| **Albumin** (A90-134A) | BETHYL | 1/1,000 in 10% Block-Ace containing 0.2% Tween-TBS | - |
| Cdx2 (sc-19478) | Santacruz biotechnology | 1/3,000 in 10% Block-Ace containing _ 0.2% Tween-TBS | - |

Western blot analysis

Cells were scraped off and recovered, and then homogenized in a sodium dodecyl sulfate (SDS) buffer (in 30% glycerol, 4% SDS, 125 mM tris-glycine, 10% 2-mercaptoethanol, 2% bromophenol blue). The cells were then subjected to polyacrylamide gel electrophoresis (PAGE) in the presence of SDS (SDS-PAGE) and subsequently electrotransferred onto a polyvinylidene fluoride (PVDF) membrane (Hybond-P; Amersham Pharmacia Biotech, Buckinghamshire, UK). For the blotted membrane, Block Ace (Sumitomo Dainippon Pharma, Osaka) was used for blocking overnight and treatment was applied overnight at 4° C with each primary antibody (Tables 2 and 3). Detection was realized with chemical fluorescence enhanced by horseradish peroxidase (HRP) labeled secondary antibodies corresponding to each primary antibody (all purchased from Santa Cruz Biotechnology, Santa Cruz, CA, USA) and ECL plus western blotting detection system (Amersham Pharmacia Biotech, Buckinghamshire, UK). The photolabeled membrane was analyzed with a CCD based chemiluminescence analyzer LAS 4000 (Fuji Film, Tokyo).

**Table 3. Primary antibodies used in western blot analysis**

| Antibody | Distributor | Dilution | Specific band (kDa) |
|---|---|---|---|
| **Actin** (sc-1616) | Santacruz biotechnology | 1/5,000 in 10% Block-Ace/0.2% Tween-TBS | 43 |
| **Caspase 3** (#9662) | Cell Signaling Technology | 1/1,000 in Immuno-enhancer | 19, 35 |
| **E Cadherin** (sc-7870) | Santacruz biotechnology | 1/1,000 in Immuno-enhancer | 120 |
| CD105 (sc-19793) | Santacruz biotechnology | 1/3.000 in 10% Block-Ace containing 0.2% Tween-TBS | 84 |
| CD29 (sc-6622) | Santacruz biotechnology | 1/3,000 in 10% Block-Ace containing 0.2% Tween-TBS | 138 |
| Mdm2(sc-813) | Santacruz biotechnology | 1/3,000 in 10% Block-Ace containing 0.2% Tween-TBS | 90 |
| POU5fl (sc-8628) | Santacruz biotechnology | 1/5,000 in10% Block-Ace containing 0.2% Tween-TBS | 45 |
| CD140a (sc-338) | Santacruz biotechnology | 1/3,000 in 10% Block-Ace containing 0.2% Tween-TBS | 170 |
| **Phospho pRb** (Ser780, #9307) | Cell Signaling Technology | 1 /1,000 in Immuno-enhancer | 110 |
| P53 (sc-6243) | Santacruz biotechnology | 1/1,000 in Immuno-enhancer | 53 |
| CD90 (sc-9163) | Santacruz biotechnology | 1/3,000 in 10% Block-Ace containing 0.2% Tween-TBS | 37 |

(continued)

| Antibody | Distributor | Dilution | Specific band (kDa) |
|---|---|---|---|
| Vimentin (sc-7558) | Santacruz biotechnology | 1/3.000 in 10% Block-Ace containing 0.2% Tween-TBS | 57 |

Cell cycle analysis

To observe the effects of low oxygen culture on rESC cell cycles, rESC-1 cell lines were cultured on matrigel in an MEF acclimated rESM for 48 hours under conditions of 20% $O_2$ + 5% $CO_2$, 5% $O_2$ + 5% $CO_2$, or 1% $O_2$ + 5% $CO_2$. Trypsin-EDTA was then used to dissociate cells into single cells and washed with PBS containing 2% FCS. 99.5% cold ethanol (final concentration 70%) was used to fix the cells overnight at -20° C. After fixation, the cells were washed once with PBS and resuspended in PBS containing 2% FCS. In addition, the cells were incubated for 30 minutes at 37° C with 50U DNase-free RNase A (Calbiochem, San Diego, CA, USA). After incubation, the cells were stained with PI for 15 minutes at room temperature. FACS Calibur (BD Biosciences, San Diego, CA, USA) was used for flow cytometry analysis. Further, CELLQUEST software was used to analyze cell cycles. RESCs cultured for 6 hours with 1 $\mu$g/ml colcemid (Invitrogen) or 1 $\mu$g/ml nocodazole (Sigma) was used as a control sample.

Embryoid body (EB) formation, adhesion, and low oxygen treatment of differentiated cells

Suspension culture of embryoid bodies was started by resuspending 100 colonies per 1 ml in 10% FCS-DMEM. EBs were allowed to precipitate in a 15 ml centrifuge tube and to absorb the old medium and then resuspended in a new medium to maintain the EBs in a suspension for 4 days while exchanging a medium every 2 days. EBs were then transferred onto a gelatin-coated dish and cultured for 6 days in 10% FCS-$\alpha$MEM. During the adhesion culture, the oxygen concentration was changed to one of 20% $O_2$, 5% $CO_2$, or 1% $O_2$ (Step 1). After 6 days of oxygen controlled culture, EB derived cells were treated with trypsin-EDTA, washed once, and resuspended in 10% FCS-$\alpha$MEM. In addition, the cells were cultured for additional three days at each $O_2$ concentration (Step 2). Cells were dissociated with trypsin and the treated cells were diluted to 200 cells/ 35 mm dish. EB derivatives were then cultured in Step 1 or Step 2 over 14 days to assay colony formation. To prevent cell death in relation to single cell digestion of human ESCs, 10 $\mu$M of Y-27632 was supplemented to the suspension medium. After 14 days, the culture was fixed with ice-cold ethanol, washed twice with PBS, and used to analyze alkaline phosphatase (ALP) activity or to count colonies after crystal violet staining. Reproducibility was confirmed by three independent experiments.

Cloning and regrowth of induced MSCs

Fibroblast colonies were scraped off from the substrate thereof with a glass capillary tube. The colonies were transferred into and dissociated in TrypLE Express (Invitrogen) in a 96 holemultiwell plate, which were plated on a gelatin-coated plate in 10% FCS-$\alpha$MEM supplemented with 10 ng/ml bFGF. After cell adhesion, the medium was exchanged every 2 days. The cells were subcultured every 3-4 days with trypsin-EDTA (Invitrogen) and maintained in 10% FCS-$\alpha$MEM supplemented with 10 ng/ml bFGF for further experimentations.

*In vitro* differentiation assay of rESC-derived MSCs

Cells were plated at a density of either $2 \times 10^5$ cells/ 35 mm plate or $1 \times 10^4$ cells/ 96 hole multiwell plate and cultured until reaching confluence. To promote lipogenesis, a medium was switched to an adipocyte differentiation medium made of 10% FCS-$\alpha$MEM supplemented with 0.5 mM isobutylmethylxanthine (Sigma) and 100 $\mu$M indomethacin (Sigma). After 10 days, culture producing lipids was fixed with 10% neutral formalin and stained with an oil red O solution (Wako). To promote differentiation of osteoblasts, the medium was switched for 14 days to an osteoblast differentiation medium made of 10% FCS-$\alpha$MEM supplemented with $10^{-8}$M dexamethasone (Sigma), 10 mM 2-glycerophosphate (Sigma) and 50 $\mu$g/ml ascorbic acid (Sigma). After differentiation, the dish was fixed with 10% neutral formalin and stained with a 0.5% Alizarin Red S (Sigma) solution. To promote cartilage formation, $2.5 \times 10^5$ cells were cultured as a pellet in 15 ml polypropylene tube (BD Falcon) or $1 \times 10^5$ cells were plated on a 96 well plate and cultured for 21 days in a chondrocyte differentiation medium (Invitrogen). After differentiation of chondrocytes, the cells were treated with a TRIzol solution (Invitrogen) and RNA was purified for use in further studies.

Preparation of rESMSCs expressing green fluorescent protein (GFP)

RESCs expressing GFP were created by introducing a pCAG-GFP-IRES-Puro vector into the rESCs that were also used above. To make a pCAG-GFP-IRES-Puro vector, a GFP sequence obtained from a pCAG-GFP vector (Addgene, #11150, apportioned from Dr. Connie Cepko) was cloned to the NotI/EcoRI site of a pCAG-IRES-Puro plasmid (obtained from Dr. Hirofumi Suemori). The pCAG-GFP-IRES-Puro vector was formed into a straight chain with SspI. Gene Pulser II (BioRad laboratories, Hercules, CA, USA) used at 250V and 950 $\mu$F to electroporate $3 \times 10^6$ rESCs. RESC culture was selected for 4 days in rESM supplemented with 10 $\mu$M Y-27632 and 7 ng/ml puromycin. Cells that have undergone puromycin selection were treated with trypsin and passed through a cell strainer (BD Biosciences, San Diego, CA, USA), washed once with rESM, and resuspended in rESM supplemented with 10 $\mu$M Y-27632. Cells expressing GFP were further purified with FACSVantage (BD Biosciences) . The sorted cells were seeded on a mitomycin-C treated MEF feeder cell and cultured for 24 hours in rESM supplemented with Y-27632. Media were exchanged with a new rESM every two days until colonies were observed. MSCs were induced by the procedure described herein. Induced MSCs derived from rESC line expressing GFP (rgESMSC-1) were used for studying implantation after cloning, determining the differentiation into osteoblasts and adipocytes, and confirming the expression of MSC markers.

Implant assay using rabbit articular cartilage defect

Cells were implanted as a sheet (Kaneshiro, N. et al., Eur.Cell.Mater., 2007, 13:87-92). Since a cell sheet adheres stably and repeatably to a defective site in a very short period of time, implantation as a cell sheet is quick and simple. Cell line rESMSC-1 was directly seeded on a temperature responsive cell culture dish (diameter of 35 mm, CellSeed Inc, Tokyo) with at a density of $2 \times 10^6$ cells per sheet. The cells were maintained in 10% FCS-$\alpha$MEM supplemented with 10 ng/ml bFGF until the cells reached confluence to form a cell sheet. On the day of implantation, the temperature of the culture was decreased to room temperature (about 24°C) for 30 minutes in accordance with the previously discussed protocol to recover a sheet of viable cells. Male JW rabbits with a mature skeleton and an average weight of 3.0 kg were used for the cell implantation. The rabbits were anesthetized. The right knee joint was approached from a medial parapetellar incision to laterally move the patella. As previously reported (Koga, H. et al., StemCells, 2007, 25:689-696), an osteochondral defect of full thickness (diameter: 5 mmm, depth: 3 mm) was created in a trochlear nerve groove of the femur, and the defect was then filled with the cell sheet. After the surgery, all rabbits were returned to the cage, where they were allowed to move freely. After 2 and 4 weeks from the surgery, excessive amount of pentobarbital sodium was used to euthanize the animals to study articular tissues by histological staining, immunofluorescence method or FACS. Three animals for histological analysis and three animals for FACS analysis were prepared (total of 6 animals) for the implantation experiment.

Histology and fluorescence microscope inspection

The knee joint including a regeneration site was fixed in Mildform 10 N (4% paraformaldehyde, (4% paraformaldehyde/ phosphate buffer for tissue fixation) Wako Pure Chemical Industries, or 4% paraformaldehyde/phosphate buffer for tissue fixation (163-20145, Wako Pure Chemical Industries, Ltd)). 85% formic acid and 20% sodium citrate solution was used to remove calcareous portions. The sample was dehydrated and embedded in paraffin for histological observation. Staining was applied with Safranin O or double staining was applied with Alcian blue and Alizarin Red to a section. To observe the fluorescence of GFP expressing cells in regenerated cartilage, the section was deparaffinized. The rehydrated paraffin section was subjected to blocking with Block Ace (1% bovine serum albumin (BSA); Sumitomo Dainippon Pharma, Osaka) for one hour, washed twice with PBS, and incubated overnight at 4° C with anti-GFP rabbit polyclonal antibodies (Santa Cruz biotechnology, sc-8334) (1/200 dilution). The specimen was then washed twice with PBS containing 10% Block Ace and incubated with FITC labeled anti-rabbit IgG bovine secondary antibody (1/1000 dilution). After washing twice, the FITC labeled specimen was stained with DAPI (1/1000 dilution). The specimen was observed using a fluorescence microscope (BZ-9000, Keyence Corporation, Osaka).

FACS sorting of GFP positive implant cells from cartilage tissue of recipient

A regenerated site was recovered with a scalpel, washed twice with PBS, and dissociated with an enzyme for three hours in 300 U/mg collagenase (Wako Pure Chemical Industries) in DMEM/F12 supplemented with 0.3% bovine serum albumin (Sigma). Dissociated cells were recovered and passed through a 40 $\mu$m cell strainer (BD Falcon), washed twice with PBS, and sorted with FACSVantage. To avoid contamination with false positive cells, the present inventors examined samples by using two band pass filters (530 nm for FITC/GFP, 585 nm for phycoerythrin [PE]). In addition, data was displayed as a density plot of FL-1 (GFP) to FL-2 (PE) as previously suggested (Lengner, C. J. et al., Cell Cycle, 2008, 7:725-728). FL-1 cells were selected to further analyze implantation cells expressing GFP.

Statistical analysis of data

A significant difference was detected by Tukey-Kramer HSD test or Student's t-test. A p-value of less than 0.05 was deemed significant.

Figure 1 shows a comparison of MSCs obtained from the synovial membrane of a rabbit manufactured in the above-described Production Example (left) and MSCs induced from ES cells in the present Example (ESC-MSC; right). ESC-MSCs had a self-replication capability, and cell surface antigens had MSC characteristics (e.g. , was PDFGRα+, CD105+ and CD271+) as well as osteogenecity, chondrogenicity, and adipogenicity. Although data is not shown, the same result is described in Non Patent Literature 5 (particularly Figures 3, 5, 6, and 7). The results can be referred as needed.

(Example 2: Creation of embryonic stem cell derived TEC from ES-MSCs (ES-TEC))

Each clone of ES-MSCs was grown in plate culture, which does not use a feeder, to secure a certain number of cells. The aforementioned basal medium to which 10 ng/ml basic fibroblast growth factor (bFGF) is added was used as the growth medium. 100 $\mu$g of Wako: recombinant human bFGF : Cat.No.060-04543: was used for the bFGF, which was dissolved in PBS added with 0.1% CHAPS (SIGMA) and 0.5% BSA (bovine serum albumin, from bovine serum, A2153-100G, SIGMA). The solution was dispensed and stored at -20° C.

A TEC was created by the same method as that for somatic (synovial) stem cells as described in Production Example 5. Cells were inoculated at $4 \times 10^5$ cells/cm$^2$. After 5-14 days of plate culture, shear stress was applied on the boarder between the cells and culture dish by pipetting to form a suspension culture of a cell-matrix complex to create a TEC. The culture solutions used were 1) conventional culture solution for creating TEC [DMEM (043-30085, lot#TLG7036, Wako), 10% FBS (172012-500ML, SIGMA), 1% Antibiotic-Antimycotic, 100x (Invitrogen, 15240)], 2) previously mentioned basal medium, and 3) previously mentioned growth medium. 0.2 mM of L-ascorbic acid 2-phosphate (SIGMA, Cat#49752-10G, lot#BCBC4071V) was added to all culture solutions (1-3).

Since 2) can be used in culture for 14 days, the culture period was set to 14 days. For 1) and 3), tissues are naturally folded due to contraction of the cytoskeleton prior to 14 days. Since continuation for 14 days is difficult, the culture period was set to 5-12 days.

The results are partially shown in Figure 2. Figure 2 shows a comparison of a cell sheet (left) to a TEC induced from ES cells, which is an example of the present invention. As shown, the induced TEC of the present invention was demonstrated to be highly capable of three-dimensional formation. The tissue prior to being detached from the bottom surface by applying shear stress in the TEC formation procedure in the Example described above was used as the cell sheet. Thus, a cell sheet made by plate culture for 5-12 days in a TEC formation medium was used.

Further, as shown in Figure 3, a general view of tissues and tissue fragments with or without ascorbic acid 2-phosphate were observed to evaluate the difference in the volume (left column) and weight (right column) and the amount of hydroxyproline with or without ascorbic acid 2-phosphate. The protocol thereof is disclosed below.

The difference was evaluated with a hydroxyproline measurement kit (e.g., available from BioVision). Inflammation was evaluated with a plethysmometer or the like.

ES-MSCs were inoculated at a TEC creation concentration ($1.52 \times 10^5$ cells/well) in each well of a 12 well plate to create a TEC. A medium was created by separating conventional 10% FBS-containing DMEM for TEC creation into a 0. 2 mM ascorbic acid added group (n=6) and non-added group (n=6) . Three of each group were used for weight/volume measurements and the rest of the three were used in hydroxyproline measurements. For hydroxyproline, the following kit was used (Hydroxyproline Assay Kit Cat#K555-100; Lot#60855, BioVision), where the measurements were taken in accordance with the protocol on the package insert.

For the TEC made from $0.125 \times 10^6$, $0.25 \times 10^6$, $0.5 \times 10^6$, $1 \times 10^6$, $2 \times 10^6$, $4 \times 10^6$ , $8 \times 10^6$, or $16 \times 10^6$ cells/well in a 6 well plate which was cultured over 12 days, detached, and allowed to undergo self-contraction, the volume was measured with a plethysmometer (model TK-101CMP; UNICOM, Chiba, Japan), and the weight was measured with a scale (Ando W, Tateishi K, Katakai D, Hart DA, Higuchi C , NakataK, et al., Tissue Eng Part A 2008; 14 : 2041-2049). 4 samples were evaluated at each cell density. In short, a plethysmometer is a minute volume controlled measuring device that is specially designed for accurate measurement. A plethysmometer consists of Perspex cells filled with water. A transducer records a small difference in the water level caused by a change in volume. A plethysmometer shows an accurate volume by digitally reading out such a difference. The weight was measured with a common scale (METTLER TOLEDO AG204, Mettler-Toledo International Inc., Max210gd=0.1mg, METTLER TOLEDO AG204 (Mettler-Toledo International Inc.)) in a laboratory (Lot# 60855). Measurements were taken in accordance with the protocol on the package insert.

Figure 3 shows increase in the production of extracellular matrices (collagen) by an addition of ascorbic acid 2-phosphate. The top left section shows the difference in a tissue fragment with or without ascorbic acid 2-phosphate. The top right section shows the difference in the general view of the tissue with or without ascorbic acid 2-phosphate (top row: without addition of ascorbic acid 2-phosphate, bottom row: with addition of ascorbic acid 2-phosphate). The bottom left section shows the difference in the volume (left column) and weight (right column) with or without ascorbic acid 2-phosphate. Two bars on the left side of the graph are without ascorbic acid 2-phosphate, and the two bars on the right side are with addition of ascorbic acid 2-phosphate. The y-axis indicates volume (left, $\mu$l) or weight (right, mg). The bottom right section is a comparison of the amount of hydroxyproline with or without ascorbic acid 2-phosphate. In this manner, the volume, weight, hydroxyproline generation amount were demonstrated to be significantly increased by an addition of ascorbic acid. Formation of abundant extracellular matrix is recognized due to an addition of ascorbic acid. The effect of being able to form a tissue as in somatic TECs is demonstrated.

Further as shown in Figure 4, a comparison of a monolayer culture produced with ES cells to a three-dimensional synthetic tissue (TEC) which is a representative example of the present invention (prior to differentiation into cartilage) was evaluated. FITC immunofluorescence analysis was performed for H&E stain, collagen 1, collagen 2, and fibronectin. H&E staining was performed in accordance with conventional methods. Iron hematoxylin is made of I solution: 1g of hematoxylin and 100 ml of 95% ethanol and II solution: 2g of ferric chloride, 95 ml of distilled water, and 1 ml of concentrated hydrochloric acid. The I solution and the II solution are mixed at the time of use for staining.

The method used is the same as that for H&E staining and immunostaining in somatic TECs. A TEC was similarly created with a 12 well plate. The medium used is conventional 10% FBS-containing MEM containing 0.2 mM ascorbic acid. A tissue like sample was fixed for 24 hours with 4% paraformaldehyde (PFA) and washed with running water (tap water) for 4 hours, and then soaked in 70% ethanol (12 hours), 100% ethanol (three days), and 70% ethanol (12 hours) in this order as a dilipidization step, including the experiments discussed below (regardless of animal samples or TEC experiment in *vitro*). After washing with running water (4hours), the samples were soaked for 2-4 weeks in EDTA (high grade disodium ethylenediaminetetraacetate (dihydrate) (reagent), 000-29135, Kishida Chemical Co., Ltd.; or tetrasodium ethylenediaminetetraacetate (dihydrate), 000-29295, Kishida Chemical Co., Ltd., 276 g is dissolved in 3L of D. W. and autoclaved) . If the sample is soft after cutting with a scalpel, decalcification was deemed complete (thus the period therefor has a range) . The method was completed by placing the sample in an embedding device (LEICA ASP200S, Leica Microsystems) to create a block to make a 3-4 $\mu$m section.

Immunostaining was performed as described below. ProtinaseK Ready-to-use (Dako, S3020) was added to a deparaffinized rehydrated paraffin section. In addition, the section was left standing for 5 minutes at room temperature to allow antigens to be activated. The section was washed three times with PBS. Blocking was then applied to the specimen for one hour with 1% BSA (prepared by dissolving Albumin, from bovine serum, A2153-100G, SIGMA Life Science, in PBS such that the concentration is 1%). The specimen was washed three times with PBS and incubated overnight at 4° C with MouseMonoclonal [COLI] to Collagen (1/400 dilution) (ab90395, Abcam plc, England), anti-human collagen type II antibody (1/100 dilution) (F-57, Clone No. 1, II- 4C11, Daiichi Fine chemical Co., LTD, Osaka), or Fibronectin antibodies (1/ dilution). After washing the specimen three times, AlexaFlour® 488 labeled anti mouse IgG (H+L) goat antibody (1/1000 dilution) (Alexa Flour® 488 goat anti-mouse IgG (H+L), A21202, Molecular Probes). After washing three times, the specimen was stained with DAPI (1/1000 dilution) (SlowFade® Gold antifade reagent with DAPI, S36939, Molecular Probes®) and observed with a fluorescence microscope (BZ-9000, Keyence Corporation, Osaka).

Figure 4 shows a comparison of a monolayer culture produced withES cells to a three-dimensional synthetic tissue (TEC) which is a representative example of the present invention (prior to differentiation into cartilage). The top row shows the result for the monolayer culture and the bottom row shows the result for the TEC. The results of FITC immunofluorescence analysis are shown for H&E stain, collagen 1, collagen 2, and fibronectin in this order from the left. The figure shows the state of an ES-TEC when differentiation is not induced. It is understood that it is mainly fibrous collagen such as type 1 collagen while type 2 collagen of hyaline cartilage is not expressed, as in the conventional technique. Further, adhesive proteins such as fibronectin are also expressed, which are related to implantation without suture being possible. Furthermore, it can be seen that the monolayer, organized in three-dimension, experiences a significant increase in thickness and tissue, which undergoes self-contraction from merely being detached from the bottom surface, is organized in three-dimension.

Further, to demonstrate that differentiation into cartilage results in a hyaline cartilage-like tissue as shown in Figure 5, Alcian blue staining and Safranin O staining were performed. Such staining was performed based on a conventional method based on the method partially described in the above-described Histology and fluorescence microscope inspection. An ES-TEC made on a 12 well plate was cultured in a conventional cartilage differentiation medium for 21 days and a tissue sample was created and stained. For Alcian blue stain , pH 1. 0 Alcian blue was prepared as follows: 1. distilled water is added to 8.4 ml of 0.1 N hydrochloric acid solution (HCl) to make a 1000 ml solution; 2. 1 g of pH 1.0 Alcian blue stain solution: Alcian blue 8GX (or 8GS) is dissolved into 0.1 N hydrochloric acid solution, and the mixture is stirred with a stirrer and filtered for use; 3. a Kernechtrot staining solution is prepared in the following manner with 0.1 g Kernechtrot (Nuclear Fast Red; $C_{14}H_8NO_4SNa$), aluminum sulfate ($Al_2(SO_4)$ 5g), and 100ml of distilled water: 5g of aluminum sulfate is dissolved in 100 ml of distilled water and 0.1 g of Kernechtrot is added. The solution is boiled for 5 minutes, cooled at room temperature, and then filtered to prepare a solution to be used.

Staining was performed as follows (pH 1.0 Alcian blue stain) .

1. A sample was deparaffinized and washed with running water for 2-3 minutes, and washed with distilled water for 5-10 seconds.

2. The sample was soaked for 3-5 minutes in 0.1 N hydrochloric acid water. 3. The sample was soaked for 30-120 minutes in pH 1.0 Alcian blue staining solution. 4. The sample was soaked for 2-3 minutes in each 0.1 N hydrochloric acid water (2 tanks).

5. The sample was placed directly into alcohol for dehydration without washing. 6. The sample was cleared and mounted.

Safranin O staining was performed in the following manner. A sample is washed 4 times for 5 minutes each with xylene for deparaffinization. The same was washed with 100% alcohol 1-2. The sample was washed with 100% alcohol 1-2 for five minutes each. The sample was washed with each of 95%, 80%, and 70% alcohol for 2 minutes each until acclimated. The sample was washed for 5 minutes with running water. The sample was then stained with iron hematoxylin for 5 minutes for nuclear staining. In addition, running water staining was performed for 5-10 minutes for color development, and staining was performed for 5 minutes with 0.02% FastGreen. For sorting, the sample was washed for several seconds with 1% acetic acid and stained for 7 minutes with 0.1% Safranin O. The sample was then washed with 95% alcohol (2 tanks). The sample was dehydrated first for 1 minute with 100% alcohol and for 5 minutes for the second time. Finally, the sample was cleared with xylene three times for 5 minutes each.

Figure 5 shows that differentiation of a three-dimensional synthetic tissue (TEC) produced with MSCs induced from ES cells, which is a representative example of the present invention, into cartilage results in a hyaline cartilage-like tissue. The top row shows Alcian blue staining and the bottom row shows Safranin O staining. The entire tissue is uniformly stained in bright red with Safranin O, indicating extremely strong cartilage differentiation. Staining in somatic TEC was at a level that could not be confirmed.

Broadly speaking, hyaline cartilage has chondrocytes accompanied by lacunas recognized in hyaline-like (not fibrous) extracellular matrix as its form. Furthermore, the extracellular matrix is mainly glycosaminoglycan (red with Safranin 0, blue with Alcian blue or toluidine blue) and collagen type 2. In therapy with a conventional somatic TEC and MSCs themselves, collagen type 2 is elevated, but collagen type 1 also remains strongly expressed. Collagen type 1 is almost eliminated in ESs. This is evidence that the tissue made in the present invention has a collagen composition very close to that of hyaline cartilage. Conventional (somatic) TECs appear hyaline in 6 months in some parts. However, this was not the case after 1 month. Thus, the present invention can be considered to exhibit a significant effect.

As shown in Figure 6, glycosaminoglycan (GAG) production was quantified. The protocol thereof is shown below. Glycosaminoglycan was quantified with a sulfated glycosaminoglycan quantification kit (Seikagaku Biobusiness K.K.). A three-dimensional synthetic tissue TEC was treated for 2 hours at 55° C by using a protease included in the kit and then boiled for 10 minutes and returned to room temperature. 50 $\mu$L of each sample was added to each well of a microplate (included in the kit). In addition 50 $\mu$L of reaction buffer II (included in the kit) was added to each well. Next, 150 $\mu$L of DMMB pigment solution (included in the kit) was added to each well, which was left standing for 5 minutes at room temperature while being kept away from light. The 530 nm wavelength of each well was then measured with immuno mini NT-2300 (COSMO BIO co., ltd.). The sulfated GAG standard solutions included in the kit (80, 40, 20, 10, 5, 2.5, 0 $\mu$g/mL) were processed similar to the sample. Standard curves were drawn to calculate the sulfated glycosaminoglycan concentration of the sample.

Figure 6 shows that a three-dimensional synthetic tissue (TEC) produced with MSCs induced from ES cells, which is a representative example of the present invention, produces significantly more glycosaminoglycan (GAG) than conventional somatic three-dimensional synthetic tissues upon differentiation into cartilage. The left side shows a three-dimensional synthetic tissue derived from a synovial membrane, and the right side shows the three-dimensional synthetic tissue (TEC) produced with MSCs induced from ES cells, which is a representative example of the present invention. For each side, the left side is without induction of differentiation into cartilage and the right side is with induction of differentiation into cartilage.

As shown in Figure 7, FITC immunofluorescence analysis and Alcian blue staining, on the left side, were performed on collagen 1a1 and collagen 2a1 in accordance with the aforementioned methods.

Figure 7 shows FITC immunofluorescence analysis demonstrating that a three-dimensional synthetic tissue (TEC) produced with MSCs induced from ES cells, which is a representative example of the present invention, has a hyaline cartilage-like phenotype when differentiated into cartilage. Alcian-blue staining is shown in the left, collagen 1a1 is shown second from the left, collagen 2a1 is shown second from the right, and a negative control is shown at the rightmost side. As a result, a three-dimensional synthetic tissue (TEC) produced with MSCs induced from ES cells is demonstrated to have the capability of hyaline cartilage-like differentiation.

Further, as shown in Figure 8, patterns of gene expression before and after inducing differentiation of a three-dimensional synthetic tissue (TEC) produced with MSCs induced from ES cells, which is a representative example of the present invention, into cartilage were studied. Quantitative RT-PCR was performed to determine the expression of a cartilage specific gene (collagen II and aggrecan), cartilage dedifferentiation marker (collagen I) and housekeeping gene (GAPDH). Pellets were homogenized for 60 seconds in buffer RLT for RNA extraction. The solution was then centrifuged to retrieve the supernatant. Total RNA was extracted with a TRIzol reagent (Invitrogen). After measuring the RNA concentration for each sample, complementary DNA (cDNA) was obtained at RT by using a random primer with a reverse transcriptase (Promega, San Luis Obispo, CA, USA). SYBR Premix Ex Taq (TaKaRa, JP) and a custom designed pig primer were used on the sample for qRT-PCR. QRT-PCR was then performed with ABI PRISM 7900HT (Applied Biosystems, Foster City, CA 94404, USA). The pellets were cultured in HGDMEM supplemented with 10% FBS (without chondrogenesis assisting ingredient) for use as a reference (for calibration) to compare mRNA levels of differentiated pellets (0, 2, or 10% fetal bovine serum FBS). Total RNA was extracted from cells with RNeasy fibrous tissue mini kit (QUIAGEN). After the extraction of the total RNA in accordance with the protocol enclosed with the kit, the RNA concentration of each sample was measured. For complementary DNA (cDNA) in each sample, reverse transcription was performed with Superscript III first strand synthesis system (Invitrogen), and an Oligo d(T) primer included with the kit was used as the primer. SYBR Premix Ex Taq (TaKaRa), a custom designed rabbit primer, and ABI PRISM 7900HT (Applied Biosystems, Foster City, CA 94404, USA) were used to conduct qRT-PCR. Rabbit synovial membrane-derived mesenchymal stem cells cultured as a calibration sample was used in comparing the expression of rabbit ES cell derived mesenchymal stem cells. Expression of genes was normalized with Equation 1 with respect to the expression level of GAPDH, which is the internal standard gene of each sample. A relative value to the calibration sample was calculated with Equations 2 and 3 for the normalized gene expression (GAPDH level = control).

$$\text{Equation 1} \quad \Delta Ct = (\text{standard gene Ct value}) - (\text{GAPDHCt value})$$

$$\text{Equation 2} \quad \Delta\Delta Ct = (\text{standard sample } \Delta Ct \text{ value}) - (\text{calibration sample } \Delta Ct \text{ value})$$

$$\text{Equation 3} \quad \text{relative value } 2^{-\Delta\Delta Ct}$$

Primers were designed with primer 3 software (open-source software) based on the sequence of GenBank database for the sequences corresponding to specific gene accession number. The following sequences were used:

pig-GAPDH (forward): CTGCCCCTTCTGCTGATGC (SEQ ID NO: 1),
pig-GAPDH (reverse): CATCACGCCACAGTTTCCCA (SEQ ID NO: 2),
pig-aggrecan (forward): ATTGTAGGACCCAAAGGACCTC (SEQ ID NO: 3),
pig-aggrecan (reverse): GGTCCCAGGTTCTCCATCTC (SEQ ID NO: 4),
pig-collagen1a2 (forward): ATTGTAGGACCCAAAGGACCTC (SEQ ID NO: 5),
pig-collagen1a2 (reverse): GGTCCCAGGTTCTCCATCTC (SEQ ID NO: 6),
pig-collagen2al (forward): ATTGTAGGACCCAAAGGACCTC, (SEQ ID NO: 7), and
pig--collagen2a1 (reverse): GGTCCCAGGTTCTCCATCTC (SEQ ID NO: 8)

Figure 8 shows patterns of gene expression before and after inducing differentiation of a three-dimensional synthetic tissue (TEC) produced with MSCs induced from ES cells, which is a representative example of the present invention, into cartilage, and shows that expression of a cartilage associated gene, which is 10-20 fold of somatic TEC, is observed. Sy-TEC indicates a TEC derived from synovial MSCs. ES-TEC indicates a TEC derived from ES cell-derived MSCs. Hyaline Cartilage indicates hyaline cartilage-like tissue. No Introduction indicates no induction. Chondrogenesis indicates the state after stimulus for differentiation into cartilage. $p<0.05$ indicates statistical significance. The y-axis indicates the relative expression intensity. Further, the therapeutic effect of a composite tissue using the induced TEC of the present invention after a month was observed. In this regard, a composite tissue using an induced TEC was implanted instead of a composite tissue of a somatic TEC with an artificial bone in accordance with the description in the implantation examples. The results are shown in Figures 9 and 10.

Figure 9 shows a result of treating a bone defect using a composite tissue made from conjugating a three-dimensional synthetic tissue (TEC) produced with MSCs induced from ES cells, which is a representative example of the present invention, to βTCP. As shown, repair with a hyaline cartilage-like tissue is observed only after one month. The left most section on the top row shows a bone defect site. The second section from the left in the top row shows an example of a composite tissue made by conjugating a TEC produced with MSCs induced from ES cells, which is a representative example of the present invention, to βTCP. The second section from the right in the top row shows a schematic diagram of a composite tissue of TEC + βTCP. Figure 10 shows the result of hematoxylin-eosin staining comparing the result of treating a bone defect by using a composite tissue made by conjugating a three-dimensional synthetic tissue (TEC) produced with MSCs induced fromES cells, which is a representative example of the present invention, with βTCP to that using a somatic (synovial membrane-derived) TEC. The left side shows the result using the synovial membrane-derived TEC composite tissue, and the right side shows the result using the composite tissue made by conjugating the TEC produced with MSCs induced from ES cells with βTCP.

Further, as shown in Figure 11, toluidine blue staining test was performed for comparison with a somatic (synovial membrane-derived) TEC in terms of suppression of ossification signal and maintenance of differentiation into cartilage after two months. Implantation experimentations were conducted in accordance with the description of the implantation examples. Toluidine blue staining was conducted based on a convention method. Briefly, a sample was deparaffinized, washed with water, and stained for 10-30 minutes with a 0.05% toluidine blue staining solution, washed twice with pure ethanol, dehydrated, cleared, and mounted.

Figure 11 is a result (toluidine blue staining) comparing the result of treating a bone defect by using a composite tissue made by conjugating a three-dimensional synthetic tissue (TEC) produced with MSCs induced from ES cells, which is a representative example of the present invention, with βTCP to that using a somatic (synovial membrane-derived) TEC in terms of suppression of ossification signals and maintenance of differentiation into cartilage after two months. The left side shows the result using the synovial membrane-derived TEC composite tissue, and the right side shows the result using the composite tissue made by conjugating a TEC produced with MSCs induced from ES cells with βTCP. The top row is the picture of the whole, and the bottom row is an expanded view. As shown, the TEC made with induced MSCs of the present invention suppresses ossification signals and maintains differentiation into cartilage. Thus, it is understood that an effect that was not accomplished in conventional techniques is achieved.

(Example 3: Induction from pluripotent stem cells (P cells) to P cell-derived MSCs)

As a variation method of Example 1, pluripotent stem cells (P cells) were cultured in an ultra-low attachment culture dish (Corning, Corning, NY) to form embryoid bodies. The culture solution (1) was DMEM, 15% FBS, 1 mM NEAA, 0.1 mM 2-mercaptoethanol, 1 mM L-glutamine, and 50 U/ml P/S. The embryoid bodies were cultured in suspension culture for three days. After treating the embryoid bodies with 0.5 mM retinoic acid, the embryoid bodies were cultured for an additional two days.

The embryoid bodies were transferred to a 0.1% gelatin coated plate. 10 ng/mL TGFβ1 was added to the aforementioned culture solution (1) and the embryoid bodies were cultured for 2 days. Culture was further continued with a culture solution (2) added with ascorbic acid, DMEM, and 10% FBS. After the cells reached near confluence, cells were detached with trypsin and recovered, which were inoculated onto a 0.1% gelatin coated culture dish to culture the cells with the aforementioned culture solution (2). Cells from two passages or more were used (Reference document: Derivation of murine induced pluripotent stem cells (iPS) and assessment of their differentiation toward osteogenic lineage. LiF, Bronson S, Niyibizi C. J Cell Biochem. 2010 Mar 1; 109(4): 643-52.doi:10.1002/jcb.22440). MCSs produced with this method can be used to manufacture a composite tissue as a TEC as in Example 2 while appropriately referring to the Production Example.

(Example 4 : Alternative method of induction from pluripotent stem cells (P cells) to P cell-derived MSCs)

As still another variation method of Example 1, pluripotent stem cells (P cells) were cultured in a matrigel coated culture dish (made by dissolving Matrigel (BD Bioscience, San Diego) in a DMEM/-Ham's F12 medium at a concentration of 100 ug/m, which is placed in a culture dish and left standing for one hour at room temperature, and then removing the culture solution) by using a serum-free medium mTeSR1 (StemCell Technologies) . When the cells reached near confluence, the medium was replaced with a DMEM/-Ham's F12 medium comprising 20% KOSR (Invitrogen), 1 mM L-glutamine (Invitrogen), and 10mM nonessential amino acids (Invitrogen), and SB431542 (final concentration 10 $\mu$M) dissolved in DMSO was added. After the cells were cultured for 10 days while replacing the medium every day, the cells were detached with TrypleSelect (Invitrogen) and recovered. The cells were then cultured in a common culture dish by using an MSC culture solution (DMEM medium (added with high glucose, 10% FBS, and 2 mM L-glutamine). Cells from two passages or more were used (Reference document: Small molecule mesengenic induction of human induced pluripotent stem cells to generate mesenchymal stem/stromal cells. Chen YS, Pelekanos RA, Ellis RL, Horne R, Wolvetang EJ, Fisk NM. Stem Cells Transl Med. 2012 Feb; 1(2):83-95.).

MCSs produced with this method can be used to manufacture a composite tissue as a TEC as in Example 2 while appropriately referring to the Production Example.

(Example 5: Experiment in another example of mesenchymal stem cells induced from iPS cells)

In this Example, it was confirmed that it is possible to induce mesenchymal stem cells, which are also called mesenchyme-like stem cells, from iPS cells and make a three-dimensional synthetic tissue therewith, and then produce a composite tissue with the same method as Example 1 to conduct experiments on safe and efficient repair of osteochondral detects by the same method as the above-described Examples or Production Examples. The details thereof are described below.

(Method of establishing MESc derived from iPS cells)

IPS cells (253G1) were obtained from RIKEN. When the iPS cells were cultured at oxygen partial pressure of 1%, spindle shaped cells were observed around the colony. When such cells were sorted for CD44/CD73/CD105 positive cells with a flow cytometer, they would be iPS-MSCs differentiating into cartilage, bone, and fat. These experiments were conducted based on the procedure described in Example 1. The results thereof are shown in Figure 15. As shown in Figure 15, it is understood that it was possible to produce MSCs (iPS-MSCs) differentiating into cartilage, bone, and fat by using iPS cells as in the case of using ES cells.

(Method of producing iPS-TEC)

Next, a cell sheet was made by culturing iPS-MSCs in high density plate culture added with ascorbic acid to make a TEC as in the case of using somatic MSCs or ES-MSCs. The production method thereof was implemented based on the procedure described in Example 2. The results thereof are shown in Figure 16. As shown in Figure 15, it is understood that it was possible to produce a three-dimensional synthetic tissue (TEC) (iPS-TEC) differentiating into cartilage, bone, and fat by using iPS cells as in the case of using ES cells. Mesenchyme-like stem cells can be induced from iPS cells while referring to Jung et al, STEM CELLS, 2011; doi:10.1002/stem.727.

(Attribute of iPS-TEC)

An iPS-TEC made in this manner was used to verify the function thereof or the like as shown in Example 2.
An iPS-TEC, when used, has been confirmed to have the same function as ES-TECs made by using ES cells.
For example, as shown in Figure 17, the state (prior to differentiation into cartilage) of iPS-TECswas evaluated. Here, FITC immunofluorescence analysis was conducted for H&E staining, collagen 1, collagen 2, and fibronectin. H&E staining was conducted in accordance with a conventional method. Iron hematoxylin is made of I solution: 1g of hematoxylin and 100 ml of 95% ethanol and II solution: 2g of ferric chloride, 95 ml of distilled water, and 1 ml of concentrated hydrochloric acid. The I solution and the II solution were mixed at the time of use for staining. The results are shown in each picture of Figure 17. In the state of iPS-TEC when differentiation is not induced, it is understood that it is mainly fibrous collagen such as type 1 collagen while type 2 collagen of hyaline cartilage is not expressed, as in the conventional technique. Further, adhesive proteins such as fibronectin are also expressed. Thus, implantation without suture is possible. Furthermore, it can be seen that a tissue, when organized from a monolayer into three-dimension, experiences a significant increase in thickness and undergoes self-contraction from merely being detached from the bottom surface, is organized in three-dimension.
Further, it is confirmed that hyaline cartilage-like tissue is also made by differentiation of an iPS-TEC into cartilage as in an ES-TEC by Alcian blue staining and Safranin O staining. Differentiation into cartilage is confirmed to be significantly improved in comparison to somatic TECs. Further, it is confirmed that collagen type 2 expression is elevated and collagen type 1 is eliminated in iPS-TECs as in ES-TECs.
Further, if sulfated glycosaminoglycan concentrations are studied, it is confirmed that significantly more glycosaminoglycan (GAG) is produced in iPS-TECs as in ES-TECs than in conventional somatic three-dimensional synthetic tissues.
Next, when FITC immunofluorescence analysis and Alcian blue staining, on the left side, are performed on collagen 1a1 and collagen 2a1, iPS-TECs are confirmed to have hyaline cartilage-like differentiation capability as in ES-TECs.
Furthermore, when gene expression patterns are observed, expression of cartilage associated genes is observed to be enhanced in iPS-TECs as in ES-TECs. Further, when implantation examples are implemented, it is confirmed that the effect thereof is improved relative to somatic TECs.

(Example 6: Alternative method with ES cells)

In this example, it was confirmed that it is possible to induce mesenchymal stem cells, which are also called mesenchyme-like stem cells, from rabbit iPS cells and make a three-dimensional synthetic tissue therewith, and then produce a composite tissue with the same method as Example 1 to conduct experiments of safe and efficient repair of osteochondral detects by the same method as Example 1. Upon implementation, experimentation was conducted with an approval of Osaka University Faculty of Medicine animal experiment facility for a genetic engineering experiment.

ES cells can be induced into mesenchymal stem cells, which are also called mesenchyme-like stem cells, by referring to the methods discussed up to this point as well as de Peppo et al. , TISSUE ENGINEERING: Part A, 2010; 16; 3413-3426; Toh et al., Stem Cell Rev. and Rep. , 2011; 7:544-559; Vargaet al. , Biochem. Biophys. Res. Commun., 2011; doi:10.1016/j.bbrc.2011.09.089; Barbet et al., Stem Cells International, 2011, doi:10.4061/2011/368192; Sanchez et al., STEM CELLS, 2011; 29:251-262; Simpson et al., Biotechnol. Bioeng., 2011;doi:10.1002/bit.23301.

(Differentiation induction of mesenchymal stem cells (MSCs) induced from embryonic stem cells (ESCs))

An inner cell mass was collected and cultured in a feeder cell (MEF) to induce ESCs. Next, an embryoid body (EB) was created and induced to differentiate into MSCs (ES-MSCs) in plate culture under controlled oxygen partial pressure.

(Creation of TEC and TEC/artificial bone hybrid implant)

ES-MSCs inoculated at $4 \times 10^5$/cm$^2$ were cultured for two weeks in a culture solution (HG-DMEM) containing ascorbic acid 2-phosphate. The cells were detached from the bottom surface by shear stress to form a three-dimensional structure to produce TECs. Furthermore, the TECS were placed on an artificial bone with any shape for integration by the adhesion property thereof to produce a TEC/artificial bone hybrid implant.

(Cartilage repair of rabbit osteochondral defect)

An integrated implant of a TEC made with ES-MSCs and an artificial bone with $\phi$ 5mm $\times$ height 4 mm was implanted in a $\phi$ 5 mm $\times$ height 6 mm osteochondral defect in a rabbit knee joint. Apparent cartilage repair was observed at one month after an operation due to a TEC/artificial bone hybrid implant therapy in comparison to a knee with only a defect.

(Example 7: Expression of BMP receptor)

In this Example, expression of BMP receptors is demonstrated to be significantly higher in a three-dimensional synthetic tissue (TEC) produced with MSCS induced from ES cells, which is a representative example of the present invention, than in somatic (synovial membrane-derived) TECs and synovial MSCs.
In this Example, for synovial membrane-derived MSCs, a synovial membrane was collected from a rabbit knee joint, and MSCs were then collected by a conventional method (Shimomura et al. Biomaterials 31 2010). RNA samples of Syn-MSCs were collected during normal plate culture. After culturing for 10 days in a TEC creation medium [DMEM (043-30085, lot#TLG7036, Wako), 10% FBS (172012-500ML, SIGMA), 1% Antibiotic-Antimycotic, 100x (Invitrogen, 15240), 0.2 mM L-ascorbic acid 2-phosphate (SIGMA, Cat#49752-10G, lot#BCBC4071V)], a Syn-TEC and ES-TEC were created and RNA samples were collected. RNeasy Fibrous Tissue Kit (Qiagen, Valencia, CA, USA) was used for RNA collection, Reverse Transcription System (Promega, San Luis Obispo, CA, USA) was used for cDNA synthesis, and SYBR Premix Ex Taq (TaKaRa, JP) was used for quantitative PCR to measure gene expression of BMP2 receptors and the like (e.g., BMPR1A, BMPR2).
The results are shown in Figure 12. As shown in Figure 12, expression of BMP receptors is demonstrated to be significantly higher in a three-dimensional synthetic tissue (TEC) produced with MSCs induced from ES cells , which is a representative example of the present invention, than in somatic (synovial membrane-derived) TECs and synovial MSCs. Syn-MSCs indicate synovial membrane-derived MSCs themselves. Syn-TEC indicates synovial membrane-derived TECs. ES-MSC indicates a TEC produced with MSCs induced from ES cells , which is a representative example of the present invention. The left side shows BMPR1A, which is one of the BMP receptors, and the right side shows BMPR2, which is another BMP receptor. The y-axis indicates the ratio of increase in the expression of each marker relative to the value before the induction of differentiation. The TEC manufactured with induced MSCs of the present invention has expression of BMP receptors such as BMPR1A and BMPR2 that are not seen in conventional somatic TECs. This also shows that the capability to differentiate into cartilage is enhanced.

(Example 8 : Examination of difference between DMEM and αMEM)

The difference in the composition of DMEM (043-30085, lot#TLG7036, Wako) from that of αMEM (Invitrogen, GIBCO Cat#11900-024, lot#755272) is that the former contains about 4.5 times the amount of glucose, about 4 times the amount of vitamins, and about 2 times the amount of amino acids relative to the latter. Meanwhile, the latter contains nucleic acids that are not contained in the former. Synthesis of extracellular matrix proteins by cells is extremely important for the creation of TECs. ES-TECs created with nutritionally superior DMEM exhibited early chondrogenesis after a biological implant. In contrast, with $\alpha$MEM, which is a growth medium optimized for ES-MSCs, a TEC formed *in vitro* did not exhibit chondrogenesis *in vivo*. A TEC exhibiting very strong chondrogenic ability *in vivo* could be created if bFGF is added to $\alpha$MEM.

The ratio of elevation in GAG (glycosaminoglycan) after induction of differentiation *in vitro* was studied. In the experimentation, after cells were cultured with various culture solutions for 10 days to create Syn-TECs and various ES-TECs which were each cultured for 21 days in cartilage differentiation inducing medium, glycosaminoglycan (GAG) was quantified with a sulfated glycosaminoglycan quantification kit (Cat#280560, SEIKAGAKU BIOBUSINESS, JAPAN). The cartilage differentiation inducing medium was DMEM (043-30085, lot#TLG7036, Wako), 50mg/ml ITSPremix (BD Biosciences; 6.25 mg/ml insulin, 6.25 mg/ml transferrin, 6.25ng/mlselenite, 1.25 mg/ml BSA, and 5. 35 mg/ml linoleic acid), 0.2m MAsc-2p(SIGMA, Cat#49752-10G, lot#BCBC4071V), 200 ng/ml recombinant human BMP-2 (OSTEOPHARMA, Japan). The results are shown in Figure **13.**

As shown in Figure **13,** the significance of the present invention is demonstrated in terms of the ratio of increase in GAG (glycosaminoglycan) after inducing differentiation *in vitro.* The Figure shows, from the left side, a synovial membrane - derived three-dimensional synthetic tissue (Syn-TEC), MSCs prepared fromEScells organized into a three-dimensional synthetic tissue with $\alpha$MEM (ES-aMEM), MSCs prepared from ES cells organized into a three-dimensional synthetic tissue with DMEM (ES-DMEM), and MSCs prepared from ES cells organized into a three-dimensional synthetic tissue with $\alpha$MEM + bFGF (ES-aMEM+bFGF). The y-axis shows the ratio of increase in GAG relative to the value before induction of differentiation. A more significant tendency (elevation in GAG) to exhibit early chondrogenesis was observed in ES-DMEM and ES-aMEM-bFGF relative to ES-aMEM. This also indicates that the TEC manufactured with induced MSCs of the present invention has enhanced capability to differentiate into cartilage.

Further, an osteochondral defect with $\phi$ 5mm depth 6 mm was created in the femur of femoral patella facies articularis of a 32 week old rabbit. Each of the ES TEC and Syn-TEC created were integrated with a $\beta$TCP artificial bone with $\phi$ 5mm height 4 mm to create a hybrid TEC-artificial bone, which was implanted. The results of examining chondrogenesis at 4 weeks with Alcian blue are shown in Figure 14.

As shown in Figure **14,** the capability to form cartilage *in vivo* after 4 weeks from implantation is demonstrated. The Figure shows, from the left side, a synovial membrane-derived three-dimensional synthetic tissue (Syn-TEC), MSCs prepared from ES cells organized into a three-dimensional synthetic tissue with $\alpha$MEM (ES-aMEM), MSCs prepared from ES cells organized into a three-dimensional synthetic tissue with DMEM (ES-DMEM), and MSCs prepared from ES cells organized into a three-dimensional synthetic tissue with $\alpha$MEM + bFGF (ES-aMEM+bFGF). A more significant tendency of early chondrogenesis was observed in ES-DMEM and ES-aMEM-bFGF relative to ES-aMEM. A TEC manufactured with induced MSCs of the present invention was found to significantly enhance the ability to form cartilage by allowing a self-contraction reaction to take place in a more eutrophicated medium (DMEM, or $\alpha$MEM added with basic fibroblast growth factors (bFGF) or the like) instead of a conventional medium considered to be suitable for MSCs (e. g., $\alpha$MEM). Thus, the present invention is demonstrated as capable of providing an improved method of producing a synthetic tissue for manufacturing a three-dimensional synthetic tissue (TEC) more suited to osteochondral therapy compared to conventional methods.

Although certain preferable embodiments and examples have been described herein, it is not intended that such embodiments and examples are construed as limitations on the scope of the invention except as set forth in the appended claims. All patents , published patent applications and publications cited herein are incorporated by reference as if set forth fully herein.

[Industrial Applicability]

The present invention is useful in providing a radical therapeutic method, technique, pharmaceutical agent, and medical device for diseases which are conventionally difficult to treat. Particularly, the present invention provides an epoch-making therapy and prevention because it promotes recovery to a substantially native state. The present invention is also understood to be useful in providing a pharmaceutical agent, cell, tissue, composition, system, kit, and the like, which are used for such an epoch-making therapy and prevention.

There is a demand for repair and regeneration of joint tissues targeted by the present invention, mainly bones and cartilages. The number of bone fracture patients, who are targets of bone regeneration, reaches several hundreds of thousands per year. It is also said that there are 30 million potential patients having osteoarthritis, which is the main target of cartilage regenerative therapy. Thus, the potential market is enormous. The present invention is also highly useful for peripheral industries. Acute competition has been started in the regenerative medical research on joint tissues, mainly including bone and cartilage. The synthetic tissue of the present invention is a safe and original material made

## EP 2 949 747 B1

of cells collected from an organism, such as a patient, and is highly useful in view of the lack of side effects or the like.

[Sequence Listing Free Text]

SEQ ID NO 1: pig-GAPDH forward primer sequence; CTGCCCCTTCTGCTGATGC
SEQ ID NO 2: pig-GAPDH reverse primer sequence; CATCACGCCACAGTTTCCCA
SEQ ID NO 3: pig-aggrecan forward primer sequence; ATTGTAGGACCCAAAGGACCTC
SEQ ID NO 4: pig-aggrecan reverse primer sequence; GGTCCCAGGTTCTCCATCTC
SEQ ID NO 5: pig-collagen1a2 forward primer sequence; ATTGTAGGACCCAAAGGACCTC
SEQ ID NO 6: pig-collagenla2 reverse primer sequence; GGTCCCAGGTTCTCCATCTC
SEQ ID NO 7: pig-collagen2a1 forward primer sequence; ATTGTAGGACCCAAAGGACCTC
SEQ ID NO 8: pig-collagen2al reverse primer sequence; GGTCCCAGGTTCTCCATCTC
SEQ ID NO 9: rabbit Pou5f1 forward primer sequence; GATCACTCTGGGCTACACTC
SEQ ID NO 10: rabbit Pou5f1 reverse primer sequence; AGATGGTGGTTTGGCTGAAC
SEQ ID NO 11: rabbit Nanog forward primer sequence; TAGTGAAAACTCCCGACTCTG
SEQ ID NO 12: rabbit Nanog reverse primer sequence; AGCTGGGTCTGGGAGAATAC
SEQ ID NO 13: rabbit Sox2 forward primer sequence; GAGTGGAAACCCTTGTGG
SEQ ID NO 14: rabbit Sox2 reverse primer sequence; ATTTATAACCCTGTGCTCCTCC
SEQ ID NO 15: rabbit Klf4 forward primer sequence; AGGAGCCCAAGCCAAAGAG
SEQ ID NO 16: rabbit Klf4 reverse primer sequence; AATCGCAAGTGTGGGTG
SEQ ID NO 17: rabbit PDGFR$\alpha$ forward primer sequence; AGTGAGCTGGCAGTACCCGG
SEQ ID NO 18: rabbit PDGFR$\alpha$ reverse primer sequence; AGCCAGTGTTGTTCTCCTC
SEQ ID NO 19: rabbit Vimentin forward primer sequence; TTGACAATGCTTCTTTGGC
SEQ ID NO 20: rabbit Vimentin reverse primer sequence; TTCCTCATCGTGCAGTTTC
SEQ ID NO 21: rabbit Aggrecan forward primer sequence; TTGAGGACAGCGAGGCTAC
SEQ ID NO 22: rabbit Aggrecan reverse primer sequence; GCCCGATAGTGGAACACA
SEQ ID NO 23: rabbit Col2A1 forward primer sequence; TCGTGGCCGAGATGGAGAG
SEQ ID NO 24: rabbit Col2A1 reverse primer sequence; TCAAATCCTCCAGCCATTTG
SEQ ID NO 25: rabbit 28srRNA forward primer sequence; AGCAGAATTCACCAAGCGTTG
SEQ ID NO 26: rabbit 28srRNA reverse primer sequence; TAACCTGTCTCACGACGGTC
SEQ ID NO 27: rabbit Gapdh forward primer sequence; GTGAAGGTCGGAGTGAACG
SEQ ID NO 28: rabbit Gapdh reverse primer sequence; TAAAAGCAGCCCTGGTGAC

**Claims**

1. An implantable synthetic tissue substantially made of a mesenchymal stem cell induced from a pluripotent stem cell and an extracellular matrix derived from the cell.

2. The synthetic tissue of claim 1, wherein a cell in the synthetic tissue expresses at least one receptor selected from the group consisting of bone morphogenetic protein receptor 1A (BMPR1A) and bone morphogenetic protein receptor 2 (BMPR2) more than a somatic mesenchymal stem cell obtained from within the body.

3. The synthetic tissue of claim 1 or 2, wherein the synthetic tissue produces more glycosaminoglycans (GAG) in comparison to a somatic mesenchymal stem cell obtained from within the body.

4. The synthetic tissue of any one of claims 1 to 3, wherein the synthetic tissue has an ability to differentiate into hyaline cartilage-like cartilage.

5. The synthetic tissue of any one of claims 1 to 4, wherein the extracellular matrix contains collagen I and/or collagen III and there is more of the collagen I and/or collagen III than collagen II.

6. The synthetic tissue of any one of claims 1 to 5, wherein the extracellular matrix is diffusedly distributed in the tissue.

7. The synthetic tissue of any one of claims 1 to 6, wherein the mesenchymal stem cell is made in a low oxygen condition.

8. An implantable synthetic tissue substantially made of a mesenchymal stem cell induced from a pluripotent stem cell, and an extracellular matrix derived from the cell, wherein the synthetic tissue is produced by performing self-contraction in a condition where basic fibroblast growth factors (bFGFs) are added to $\alpha$MEM or in a condition of DMEM.

9. A composite tissue for treating or preventing a disease, disorder, or condition associated with an osteochondral defect, comprising the synthetic tissue according to any one of claims 1 to 8 and an artificial bone.

10. The composite tissue of claim 9, wherein the artificial bone is made of a material selected from the group consisting of hydroxyapatite and β-tricalcium phosphate.

11. The composite tissue of claim 9 or 10, wherein the disease, disorder, or condition is selected from the group consisting of osteoarthritis, osteochondral defect, osteochondral lesion, osteonecrosis, rheumatoid arthritis, bone tumor and diseases similar thereto.

12. A method for producing the synthetic tissue of any one of claims 1 to 7, the method comprising:

    A) providing mesenchymal stem cells induced from a pluripotent stem cell;
    B) positioning the cell in a container containing a cell culture solution including an agent selected from ascorbic acid, ascorbic acid 2-phosphate, or a salt thereof, wherein the container has a base with an area sufficient to accommodate a three-dimensional synthetic tissue having a desired size;
    C) culturing the cell in the container with the cell culture solution containing the agent for a period of time sufficient for forming the synthetic tissue having the desired size to form a synthetic tissue with the cell;

D) detaching the synthetic tissue from the container to elicit self-contraction by the synthetic tissue, wherein the self-contraction is performed in a condition where (a) at least one component selected from the group consisting of sugar, vitamins and amino acids and/or (b) a basic fibroblast growth factor (bFGF) is enriched for $\alpha$MEM; and

E) adjusting a thickness of the synthetic tissue by a physical stimulus or a chemical stimulus to obtain a desired thickness.

13. The method of claim 12, wherein the self-contraction is performed in a condition where bFGFs are added to $\alpha$MEM or in a condition of DMEM.

**Patentansprüche**

1. Implantierbares synthetisches Gewebe, das im Wesentlichen aus einer mesenchymalen Stammzelle, die ausgehend von einer pluripotenten Stammzelle induziert wurde, und einer von der Zelle abgeleiteten extrazellulären Matrix besteht.

2. Synthetisches Gewebe gemäß Anspruch 1, wobei eine Zelle in dem synthetischen Gewebe mindestens einen Rezeptor mehr exprimiert als eine somatische mesenchymale Stammzelle, die aus dem Körper erhalten wurde, wobei der Rezeptor ausgewählt ist aus der Gruppe bestehend aus knochenmorphogenetischem Protein-Rezeptor 1A (bone morphogenetic protein receptor 1A, BMPR1A) und knochenmorphogenetischem Protein-Rezeptor 2 (bone morphogenetic protein receptor 2, BMPR2).

3. Synthetisches Gewebe gemäß Anspruch 1 oder 2, wobei das synthetische Gewebe mehr Glykosaminoglykane (GAG) produziert als eine somatische mesenchymale Stammzelle, die aus dem Körper erhalten wurde.

4. Synthetisches Gewebe gemäß einem der Ansprüche 1 bis 3, wobei das synthetische Gewebe die Fähigkeit besitzt, in Hyalinknorpel-ähnlichen Knorpel zu differenzieren.

5. Synthetisches Gewebe gemäß einem der Ansprüche 1 bis 4, wobei die extrazelluläre Matrix Kollagen I und/oder Kollagen III umfasst, und wobei mehr Kollagen I und/oder Kollagen III vorhanden ist als Kollagen II.

6. Synthetisches Gewebe gemäß einem der Ansprüche 1 bis 5, wobei die extrazelluläre Matrix diffus im Gewebe verteilt vorliegt.

7. Synthetisches Gewebe gemäß einem der Ansprüche 1 bis 6, wobei die mesenchymale Stammzelle unter Bedingungen mit geringem Sauerstoff hergestellt wird.

8. Implantierbares synthetisches Gewebe, das im Wesentlichen aus einer mesenchymalen Stammzelle, die ausgehend von einer pluripotenten Stammzelle induziert wurde, und einer von der Zelle abgeleiteten extrazellulären Matrix besteht, wobei das synthetische Gewebe mittels Selbstkontraktion unter Bedingungen hergestellt wird, bei denen basische Fibroblasten-Wachstumsfaktoren (basic fibroblast growth factors, bFGFs) zu $\alpha$MEM gegeben werden oder unter Bedingungen mit DMEM.

9. Kompositgewebe zur Behandlung oder Vermeidung einer Erkrankung, einer Störung oder eines Zustands, die/der mit einem osteochondralen Defekt assoziiert ist, wobei das Kompositgewebe das synthetische Gewebe nach einem der Ansprüche 1 bis 8 und einen künstlichen Knochen enthält.

10. Kompositgewebe nach Anspruch 9, wobei der künstliche Knochen aus einem Material besteht, das ausgewählt ist aus der Gruppe bestehend aus Hydroxyapatit und $\beta$-Tricalciumphosphat.

11. Kompositgewebe nach Anspruch 9 oder 10, wobei die Erkrankung, die Störung oder der Zustand ausgewählt ist aus der Gruppe bestehend aus Osteoarthritis, osteochondraler Defekt, osteochondrale Läsion, Osteonekrose, rheumatische Arthritis, Knochentumor und dazu ähnliche Erkrankungen.

12. Verfahren zur Herstellung eines synthetischen Gewebes gemäß einem der Ansprüche 1 bis 7, bei dem man

A) mesenchymale Stammzellen bereitstellt, die ausgehend von einer pluripotenten Stammzelle induziert wurden;

B) die Zelle in einem Behälter positioniert, der eine Zellkulturlösung enthält, einschließlich eines Mittels, das ausgewählt ist aus Ascorbinsäure, Ascorbinsäure-2-phosphat oder einem Salz davon, wobei der Behälter einen Boden mit einer Fläche aufweist, die ausreicht, um ein dreidimensionales synthetisches Gewebe der gewünschten Größe aufzunehmen;

C) die Zelle in dem Behälter, der die Zellkulturlösung mit dem Mittel enthält, für einen Zeitraum kultiviert, der ausreicht, um das synthetische Gewebe mit der gewünschten Größe zu bilden, wobei ein synthetisches Gewebe in der Zelle gebildet wird;

D) das synthetische Gewebe von dem Behälter ablöst, um eine Selbstkontraktion des synthetischen Gewebes hervorzurufen, wobei die Selbstkontraktion unter Bedingungen erfolgt, bei denen (a) mindestens ein Bestandteil ausgewählt aus der Gruppe bestehend aus Zucker, Vitaminen und Aminosäuren und/oder (b) ein basischer Fibroblasten-Wachstumsfaktor (bFGF) in Bezug auf αMEM angereichert ist; und

E) die Dicke des synthetischen Gewebes durch einen physikalischen Stimulus oder einen chemischen Stimulus anpasst, um eine gewünschte Dicke zu erhalten.

13. Verfahren gemäß Anspruch 12, wobei die Selbstkontraktion unter Bedingungen durchgeführt wird, bei denen bFGFs zu αMEM gegeben werden, oder Bedingungen mit DMEM.


**Revendications**

1. Tissu synthétique implantable pratiquement constitué de cellules souches mésenchymateuses induites à partir de cellules souches pluripotentes et d'une matrice extracellulaire dérivée des cellules.

2. Tissu synthétique selon la revendication 1, dans lequel des cellules dans le tissu synthétique expriment au moins un récepteur choisi dans le groupe constitué par le récepteur de protéine morphogénétique osseuse 1A (BMPR1A) et le récepteur de protéine morphogénétique osseuse 2 (BMPR2) de plus que des cellules souches mésenchymateuses somatiques obtenues à partir de l'intérieur du corps.

3. Tissu synthétique selon la revendication 1 ou 2, dans lequel le tissu synthétique produit davantage de glycosaminoglycanes (GAG) que des cellules souches mésenchymateuses somatiques obtenues à partir de l'intérieur du corps.

4. Tissu synthétique selon l'une quelconque des revendications 1 à 3, lequel tissu synthétique est capable de se différencier en cartilage de type cartilage de hyaline.

5. Tissu synthétique selon l'une quelconque des revendications 1 à 4, dans lequel la matrice extracellulaire contient du collagène I et/ou du collagène III et il y a davantage de collagène I et/ou de collagène III que de collagène II.

6. Tissu synthétique selon l'une quelconque des revendications 1 à 5, dans lequel la matrice extracellulaire est distribuée de manière diffusée dans le tissu.

7. Tissu synthétique selon l'une quelconque des revendications 1 à 6, dans lequel les cellules souches mésenchymateuses sont produites dans des conditions pauvres en oxygène.

8. Tissu synthétique implantable pratiquement constitué de cellules souches mésenchymateuses induites à partir de cellules souches pluripotentes et d'une matrice extracellulaire dérivée des cellules, lequel tissu synthétique est produit par mise en oeuvre d'une auto-contraction dans des conditions où des facteurs de croissance des fibroblastes basiques (bFGF) sont ajoutés à de l'α-MEM ou dans des conditions avec du DMEM.

9. Tissu composite pour traiter ou prévenir une maladie, un trouble ou un état associé à un défaut ostéochondral, comprenant le tissu synthétique selon l'une quelconque des revendications 1 à 8 et un os artificiel.

10. Tissu composite selon la revendication 9, dans lequel l'os artificiel est constitué d'un matériau choisi dans le groupe constitué par l'hydroxyapatitite et le phosphate β-tricalcique.

11. Tissu composite selon la revendication 9 ou 10, dans lequel la maladie, le trouble ou l'état est choisi dans le groupe constitué par l'arthrose, un défaut ostéochondral, une lésion ostéochondrale, une ostéonécrose, la polyarthrite rhumatoïde, une tumeur osseuse et des maladies similaires à ceux-ci.

**12.** Procédé pour produire le tissu synthétique de l'une quelconque des revendications 1 à 7, le procédé comprenant les opérations consistant à :

A) disposer de cellules souches mésenchymateuses induites à partir de cellules souches pluripotentes ;

B) positionner les cellules dans un récipient contenant une solution de culture cellulaire comprenant un agent choisi parmi l'acide ascorbique, le 2-phosphate d'acide ascorbique et un sel d'un tel acide, lequel récipient a une base avec une superficie suffisante pour accueillir un tissu synthétique tridimensionnel ayant une taille souhaitée ;

C) cultiver les cellules dans le récipient avec la solution de culture cellulaire contenant l'agent pendant une période de temps suffisante pour la formation du tissu synthétique ayant la taille souhaitée, pour former un tissu synthétique avec les cellules ;

D) détacher le tissu synthétique du récipient pour déclencher une auto-contraction par le tissu synthétique, laquelle auto-contraction est effectuée dans des conditions où (a) au moins un composant choisi dans le groupe constitué par les sucres, les vitamines et les acides aminés et/ou (b) un facteur de croissance des fibroblastes basiques (bFGF) est enrichi en $\alpha$MEM ; et

E) ajuster l'épaisseur du tissu synthétique par un stimulus physique ou un stimulus chimique pour obtenir l'épaisseur souhaitée.

**13.** Procédé selon la revendication 12, dans lequel l'auto-contraction est effectuée dans des conditions où des bFGF sont ajoutés à l'$\alpha$-MEM ou des conditions avec du DMEM.

# Fig. 1

### Synovial MSCs

### ESC-MSCs

EP 2 949 747 B1

Fig. 2

ES-TEC

Cell sheet

Fig. 3

Fig. 4

FITC immunofluorescence analysis

H&E          Col1          Col2          FIbronectin

## Monolayer Culture

H&E          Col1          Col2          Fibronectin

## ES-TEC

EP 2 949 747 B1

## Fig.  5

Hyaline cartilage-like tissue in differentiation of ES-TEC into cartilage

**Alcian blue staining**

**Safranin O staining**

EP 2 949 747 B1

**Fig. 6**

## In differentiation into cartilage, ES-TEC has 4-5 times the glycosaminoglycan of conventional TEC

GAG Content

mean ±SD, n=3

P<0.05

P<0.05

GAG (ug/ml)

No induction   Induction      No induction   Induction

**Synovial TEC**              **ESC-TEC**

EP 2 949 747 B1

## Fig. 7

## <u>Collagen is Col1-, Col2+, and hyaline cartilage-like</u>

FITC immunofluorescence analysis

Alcian-blue      Col1a1      Col2a1      Negative Control

EP 2 949 747 B1

Fig.   8

## Expression of cartilage associated genes is 10-20 fold of conventional TEC

**Sy-TEC**           Synovial MSCs-derived  TEC

**ES-TEC**           ESC- MSCs-derived  TEC

**Hyaline Cartilage**   rabbits' rib cartilage

EP 2 949 747 B1

Fig. 9

Repair with hyaline cartilage-like tissue in only one month

Fig. 10

H&E

ESC-Derived TEC

Synovial TEC

Fig. 11

## Inhibition of ossification signal and maintenance of differentiation into cartilage at two months

EP 2 949 747 B1

Fig. 12

Fig. 13

**Fig. 14**

Cartilage formation capability after early (4 weeks) in vivo implantation

| Syn-TEC | ES-aMEM | ES-DMEM | ES-aMEM+bFGF |

Fig. 15

Fig. 16

Fig. 17

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 0051527 A
- WO 03024463 A
- JP 4522994 B
- JP 4522999 B
- JP 5111383 A
- JP 2013012630 A

## Non-patent literature cited in the description

- **HARRIS ED, JR.** *Arthritis Rheum,* 2001, vol. 44, 1969-1970
- **KUSHIDA A ; YAMATO M ; KONNO C ; KIKUCHI A ; SAKURAI Y ; OKANO T.** *J Biomed. Mater. Res.,* 1999, vol. 45, 355-362
- **OKANO T ; YAMADA N ; SAKAI H ; SAKURAI Y.** *J Biomed Mater Res.,* 1993, vol. 27, 1243-1251
- **SHIMIZU T ; YAMATO M ; AKUTSU T et al.** *Circ Res.,* 22 February 2002, vol. 90 (3), e40
- **TERAMURA, T. ; TAKEHARA, T. ; KAWATA, N. ; FUJINAMI, N. ; MITANI, T. ; TAKENOSHITA, M. ; MATSUMOTO, K. ; SAEKI, K. ; IRITANI, A. ; SAGAWA, N.** Primate embryonic stem cells proceed to early gametogenesis in vitro. *Cloning Stem Cells,* 2007, vol. 9, 144-156
- **TAKAHASHI K ; YAMANAKA S.** Induction of pluripotent stem cells from mouse embryonic and adult fibroblast cultures by defined factors. *Cell,* 2006, vol. 126, 663-676
- **OKITA K ; ICHISAKA T ; YAMANAKA S.** Generation of germline-competent induced pluripotent stem cells. *Nature,* 2007, vol. 448, 313-317
- **TAKAHASHIK ; TANABEK ; OHNUKIM ; NARITAM ; ICHISAKA T ; TOMODA K ; YAMANAKA S.** Induction of Pluripotent Stem Cells from Adult Human Fibroblasts by Defined Factors. *Cell,* 2007, vol. 131, 861-872
- **JUNG et al.** *STEM CELLS,* 2011
- **PEPPO et al.** *TISSUE ENGINEERING:Part A,* 2010, vol. 16, 3413-3426
- **TOH et al.** *Stem Cell Rev. and Rep.,* 2011, vol. 7, 544-559
- **VARGA et al.** *Biochem.Biophys.Res.Commun.,* 2011
- **BARBET et al.** *Stem Cells International,* 2011
- **SANCHEZ et al.** *STEM CELLS,* 2011, vol. 29, 251-262
- **SIMPSON et al.** *Biotechnol. Bioeng.,* 2011
- **TERAMURA et al.** *Cell transplant,* 2012
- Hito Ishoku Zoki Kyozetsu Hanno no Byori Soshiki Shindan Kijyun Kanbetsu Shindan to Seiken Hyohon no Toriatsukai (Zufu) Jinzo Ishoku, Kanzo Ishoku, Suizo Ishoku, Shinzo Ishoku Oyobi Hai Ishoku. Pathological Tissue Diagnosis Criterion for Human Implanted Organ Rejection Reaction Handling of Differential Diagnosis and Biopsy Specimen (Illustrated Book) Kidney Implantation, Liver Implantation, Pancreas Implantation, Heart Implantation and Lung Implantation. The Japan Society for Transplantation and The Japanese Society for Pathology, 1998
- Saibokokkaku/Undo ga wakaru. Saibokokkaku/Undo ga wakaru. Yodo-sha
- **TAKENAKA T et al.** *J.Cell Sci.,* 2001, vol. 114, 1801-1809
- Shin Gekagaku Taikei, Dai 12 Kan, Zoki Ishoku (Shinzo Ishoku · Hai Ishoku Gijutsuteki, Rinriteki Seibi kara Jisshi ni Mukete. New Whole Surgery, Vol. 12, Organ Implantation (Heart Implantation · Lung Implantation From Technical and Ethical Improvements to Practice). Nakayama Shoten, vol. 12
- **KULYK WM ; FRANKLIN JL ; HOFFMAN LM.** Sox9 expression during chondrogenesis in micromass cultures of embryonic limb mesenchyme. *Exp Cell Res.,* 15 March 2000, vol. 255 (2), 327-32
- **SWARTZ MF et al.** *J Am Coll Cardiol,* 30 October 2012
- **HAASE HR ; IVANOVSKI S ; WATERS MJ ; BARTOLD PM.** Growth hormone regulates osteogenic marker mRNA expression in human periodontal fibroblasts and alveolar bone-derived cells. *J Periodontal Res.,* August 2003, vol. 38 (4), 366-74
- **WOLFMAN NM ; HATTERSLEY G ; COX K ; CELESTE AJ ; NELSON R ; YAMAJI N ; DUBE JL ; DIBLASIO-SMITH E ; NOVE J ; SONG JJ.** Ectopic induction of tendon and ligament in rats by growth and differentiation factors 5, 6, and 7, members of the TGF-beta gene family. *J Clin Invest.,* 15 July 1997, vol. 100 (2), 321-30

- **DREYER SD ; NARUSE T ; MORELLO R ; ZABEL B ; WINTERPACHT A ; JOHNSON RL ; LEE B ; OBERG KC.** Lmx1b expression during joint and tendon formation: localization and evaluation of potential downstream targets. *Gene Expr Patterns,* July 2004, vol. 4 (4), 397-405
- **BRENT AE ; SCHWEITZER R.** *Cell,* 18 April 2003, vol. 113 (2), 235-48
- **HUNZIKER E.B. et al.** *J. Bone Joint Surg. Am.,* May 1996, vol. 78 (5), 721-33
- **F. BERENBAUM.** The OARSI histopathology initiative - the tasks and limitations. *Osteoarthritis and Cartilage,* 2010, vol. 18, S1
- **TRATTNIG S ; WINALSKI CS ; MARLOVITS S ; JURVELIN JS ; WELSCH GH ; POTTER HG.** Magnetic resonance imaging of cartilage repair: a review. *Cartilage,* 2011, vol. 2, 5-26
- **MITHOEFER K ; SARIS DBF ; FARR J ; KON E ; ZASLAV K ; COLE B ; RANSTAM J ; YAO J ; SHIVE MS ; BRITTBERG M.** Guidelines for the design and conduct of clinical studies in knee articular cartilage repair: International Cartilage Repair Society recommendations based on current scientific evidence and standards of clinical care. *Cartilage,* 2011, vol. 2, 100-121
- **ROOS EM ; ENGELHART L ; RANSTAM J ; ANDERSON AF ; IRRGANG JJ ; MARX R ; TEGNER Y ; DAVIS AM.** Patient-reported outcome instruments for use in patients with articular cartilage injuries. *Cartilage,* 2011, vol. 2, 122-136
- **HURTIG M ; BUSCHMANN MD ; FORTIER L ; HOEMANN CD ; HUNZIKER EB ; JURVELIN JS ; MAINIL-VARLET P ; MCILWRAITH W ; SAH RL ; WHITESIDE RA.** Preclinical studies for cartilage repair: recommendations from the International Cartilage Repair Society. *Cartilage,* 2011, vol. 2, 137-153
- **HOEMANN CD ; KANDEL R ; ROBERTS S ; SARIS D ; CREEMERS L ; MANIL-VARLET P ; METHOT S ; HOLLANDER A ; BUSCHMANN MD.** Recommended guidelines for histological endpoints for cartilage repair studies in animal models and clinical trials. *Cartilage,* 2011, vol. 2, 154-173
- **C. WAYNE MCILWRAITH ; DAVID D. FRISBIE.** Microfracture: Basic Science Studies in the Horse. *Cartilage,* 2010, vol. 1, 87-95
- **F. BERENBAUM.** The OARSI histopathology initiative. *the tasks and limitations Osteoarthritis and Cartilage,* 2010, vol. 18, S1
- **T. AIGNER ; J.L. COOK ; N. GERWIN ; S.S. GLASSON ; S. LAVERTY ; C.B. LITTLE ; W. MCILWRAITH ; V.B. KRAUS.** *Histopathology atlas of animal model systems e overview of guiding principles Osteoarthritis and Cartilage,* 2010, vol. 18, S2-S6
- **K.P.H. PRITZKER ; T. AIGNER.** Terminology of osteoarthritis cartilage and bone histopathology. *a proposal for a consensus Osteoarthritis and Cartilage,* 2010, vol. 18, S7-S9
- **R. POOLE ; S. BLAKE ; M. BUSCHMANN ; S. GOLDRING ; S. LAVERTY ; S. LOCKWOOD ; J. MATYAS ; J. MCDOUGALL ; K. PRITZKER ; K. RUDOLPHI.** Recommendations for the use of preclinical models in the study and treatment of osteoarthritis. *Osteoarthritis and Cartilage,* 2010, vol. 18, S10-S16
- **S.S. GLASSON ; M.G. CHAMBERS ; W.B. VAN DEN BERG ; C.B. LITTLE.** The OARSI histopathology initiative e recommendations for histological assessments of osteoarthritis in the mouse. *Osteoarthritis and Cartilage,* 2010, vol. 18, S17-S23
- **N. GERWIN ; A.M. BENDELE ; S. GLASSON ; C.S. CARLSON.** The OARSI histopathology initiative - recommendations for histological assessments of osteoarthritis in the rat. *Osteoarthritis and Cartilage,* 2010, vol. 18, S24-S34
- **V.B. KRAUS ; J.L. HUEBNER ; J. DEGROOT ; A. BENDELE.** The OARSI histopathology initiative - recommendations for histological assessments of osteoarthritis in the guinea pig. *Osteoarthritis and Cartilage,* 2010, vol. 18, S35-S52
- **S. LAVERTY ; C.A. GIRARD ; J.M. WILLIAMS ; E.B. HUNZIKER ; K.P.H. PRITZKER.** The OARSI histopathology initiative - recommendations for histological assessments of osteoarthritis in the rabbit. *Osteoarthritis and Cartilage,* 2010, vol. 18, S53-S65
- **J.L. COOK ; K. KUROKI ; D. VISCO ; J.-P. PELLETIER ; L. SCHULZ ; F.P.J.G LAFEBER.** The OARSI histopathology initiative - recommendations for histological assessments of osteoarthritis in the dog. *Osteoarthritis and Cartilage,* 2010, vol. 18, S66-S79
- **C.B. LITTLE ; M.M. SMITH ; M.A. CAKE ; R.A. READ ; M.J. MURPHY ; F.P. BARRY.** The OARSI histopathology initiative - recommendations for histological assessments of osteoarthritis in sheep and goats. *Osteoarthritis and Cartilage,* 2010, vol. 18, S80-S92
- **C.W. MCILWRAITH ; D.D. FRISBIE ; C.E. KAWCAK ; C.J. FULLER ; M. HURTIG ; A. CRUZ.** The OARSI histopathology initiative - recommendations for histological assessments of osteoarthritis in the horse. *Osteoarthritis and Cartilage,* 2010, vol. 18, S93-S105
- **P.C PASTOUREAU ; E.B HUNZIKER ; J.-P. PELLETIER.** Cartilage, bone and synovial histomorphometry in animal models of osteoarthritis. *Osteoarthritis and Cartilage,* 2010, vol. 18, S106-S112
- **N. SCHMITZ ; S. LAVERTY ; V.B. KRAUS ; T. AIGNER.** Basic methods in histopathology of joint tissues. *Osteoarthritis and Cartilage,* 2010, vol. 18, S113-S116

- **G.L. PEARCE ; D.D. FRISBIE.** Statistical evaluation of biomedical studies. *Osteoarthritis and Cartilage,* 2010, vol. 18, S117-S122
- **O'DRISCOLL SW ; KEELEY FW ; SALTER RUB.** *J Bone Joint Surg Am,* 1988, vol. 70, 595-606
- **MROSEK EH ; SCHAGEMANN JC ; CHUNG HW ; FITZSIMMONS JS ; YASZEMSKI MJ ; MAR- DONES RM et al.** *J Orthop Res,* 2010, vol. 28, 141-148
- **OLIVOS-MEZA A ; FITZSIMMONS JS ; CASPER ME ; CHEN Q ; AN KN ; RUESINK TJ et al.** *Osteoarthritis Cartilage,* 2010, vol. 18, 1183-1191
- **HATA R. ; SENOO H.** *J. Cell Physiol.,* 1989, vol. 138 (1), 8-16
- **HATAR. ; SENOOH.** *J. Cell Physiol,* 1989, vol. 138 (1), 8-16
- *Nature,* 1989, vol. 342, 440
- *Biochem-Biophys. Res. Commun.,* 1989, vol. 163, 967
- *Molecular Medicine,* 1993, vol. 30, 1440-1448
- **JIKKEN IGAKU.** *Experimental Medicine,* 1994, vol. 12, 1822-1826
- **ANDO W ; TATEISHI K ; KATAKAI D ; HART DA ; HIGUCHI C ; NAKATA K et al.** *Tissue Eng Part A,* 2008, vol. 14, 2041-2049
- **TATEISHI K ; HIGUCHI C ; ANDO W ; NAKATA K ; HASHIMOTO J ; HART DA et al.** *Osteoarthritis Cartilage,* 2007, vol. 15, 709-718
- **ANDOW ; TATEISHI K ; KATAKAI D ; HART DA ; HIGUCHI C ; NAKATA K et al.** *Tissue Eng Part A,* 2008, vol. 14, 2041-2049
- **ANDO W ; TATEISHI K ; HART DA ; KATAKAI D ; TANAKA Y ; NAKATA K et al.** *Biomaterials,* 2007, vol. 28, 5462-5470
- **SHIMOMURA K ; ANDO W ; TATEISHI K ; NANSAI R ; FUJIE H ; HART DA et al.** *Biomaterials,* 2010, vol. 31, 8004-8011
- **O' DRISCOLL SW ; KEELEY FW ; SALTER RB.** *J Bone Joint Surg Am,* 1988, vol. 70, 595-606
- **SUEMORI, H. et al.** *Biochem.Biophys.Res.Commun.,* 2006, vol. 345, 926-932
- **SEKIYA, I. et al.** *Stem Cells,* 2002, vol. 20, 530-541
- **WANG, G. et al.** *Proc.Natl.Acad.Sci.USA,* 2005, vol. 102, 186-191
- **DUSSAULT, A.A. et al.** *Biol.Proced.,* 2006, vol. 8, 1-10
- **KANESHIRO, N. et al.** *Eur.Cell.Mater.,* 2007, vol. 13, 87-92
- **KOGA, H. et al.** *StemCells,* 2007, vol. 25, 689-696
- **LENGNER, C. J. et al.** *Cell Cycle,* 2008, vol. 7, 725-728
- **ANDO W ; TATEISHI K ; KATAKAI D ; HART DA ; HIGUCHI C ; NAKATAK et al.** *Tissue Eng Part A,* 2008, vol. 14, 2041-2049
- **LIF, BRONSON S ; NIYIBIZI C.** *J Cell Biochem.,* 01 March 2010, vol. 109 (4), 643-52
- **CHEN YS ; PELEKANOS RA ; ELLIS RL ; HORNE R ; WOLVETANG EJ ; FISK NM.** *Stem Cells Transl Med.,* February 2012, vol. 1 (2), 83-95
- **PEPPO et al.** *TISSUE ENGINEERING: Part A,* 2010, vol. 16, 3413-3426
- **VARGAET.** *Biochem. Biophys. Res. Commun.,* 2011
- **SHIMOMURA et al.** *Biomaterials,* 2010, vol. 31